(19) Europäisches Patentamt
European Patent Office
Office européen des brevets

(11) Veröffentlichungsnummer: **0 326 974 A1**

# (12) EUROPÄISCHE PATENTANMELDUNG

(21) Anmeldenummer: 89101493.8

(51) Int. Cl.⁴: **C07K 5/06 , A61K 37/02**

(22) Anmeldetag: 28.01.89

Claims for the following Contracting States: ES + GR.

(30) Priorität: 04.02.88 DE 3803225

(43) Veröffentlichungstag der Anmeldung: 09.08.89 Patentblatt 89/32

(84) Benannte Vertragsstaaten: AT BE CH DE ES FR GB GR IT LI LU NL SE

(71) Anmelder: **HOECHST AKTIENGESELLSCHAFT**
**Postfach 80 03 20**
**D-6230 Frankfurt am Main 80(DE)**

(72) Erfinder: **Rüger, Wolfgang, Dr.**
**Parkstrasse 10**
**D-6233 Kelkheim (Taunus)(DE)**
Erfinder: **Urbach, Hansjörg, Dr.**
**Le Lavandoustrasse 41**
**D-6242 Kronberg/Taunus(DE)**
Erfinder: **Becker, Reinhard, Dr.**
**Humboldtstrasse 17**
**D-6200 Wiesbaden(DE)**
Erfinder: **Hock, Franz, Dr.**
**Altstadt 19**
**D-6110 Dieburg(DE)**

## (54) Aminosäureamide mit psychotroper Wirkung, Verfahren zu ihrer Herstellung, sie enthaltende Mittel und deren Verwendung.

(57) Die Erfindung betrifft neue Amide der Formel

$$R^3 - \underset{\underset{O}{\|}}{C} - \underset{\underset{R^4}{|}}{CH} - \underset{\underset{R^5}{|}}{N} - \underset{\underset{O}{\|}}{C} - \underset{\underset{R^1}{|}}{CH} - NH - \underset{\underset{COOR^2}{|}}{CH} - (CH)_n - R$$

in welcher $R^3$ für $NR^{13}R^{14}$ bedeutet und die übrigen Reste die in der Beschreibung angegebene Bedeutung haben, Verfahren zu deren Herstellung, Zwischenprodukte bei ihrer Herstellung, diese enthaltende Mittel und deren Vewendung als Arzneimittel mit psychotroper Wirkung. Die Erfindung betrifft ferner die Verwendung bekannter Amide als Arzneimittel mit psychotroper Wirkung.

EP 0 326 974 A1

# Aminosäureamide mit psychotroper Wirkung, Verfahren zu ihrer Herstellung, sie enthaltende Mittel und deren Verwendung

Die Erfindung betrifft die Verwindung von Aminosäureamiden oder deren physiologisch verträglichen Salzen als Arzneimittel mit psychotroper Wirkung sowie deren Verwendung bei der Herstellung entsprechender pharmazeutischer Zubereitungen.

Für diese neuartige Verwendung kommen beispielsweise Verbindungen der Formel I in Betracht,

$X^1 - X^2$ (I)

in welcher

$X^1$ 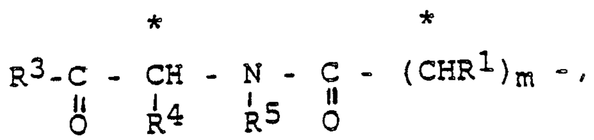

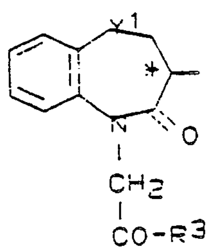 oder 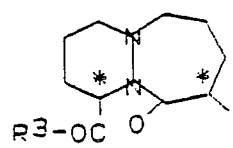 bedeutet,

$X^2$ $-CH_2SH$, 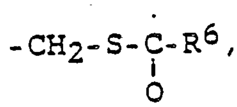, 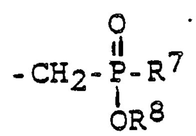 oder

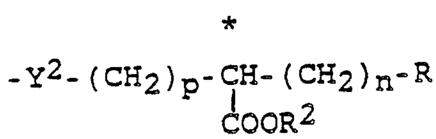 bedeutet,

$Y^1$ für -S- oder $-CH_2-$ steht,

$Y^2$ für $-NR^9-$ oder $-CH_2-$ steht,

m = 0 oder 1 ist,

n = 0, 1 oder 2 ist,

p = 0 oder 1 ist,

R = Wasserstoff,

einen gegebenenfalls substituierten aliphatischen Rest mit 1 - 21 C-Atomen,

einen gegebenenfalls substituierten alicyclischen Rest mit 3 - 20 C-Atomen,

einen gegebenenfalls substituierten aromatischen Rest mit 6 - 12 C-Atomen,

einen gegebenenfalls substituierten araliphatischen Rest mit 7 - 32 C-Atomen,

einen gegebenenfalls substituierten alicyclisch-aliphatischen Rest mit 4 - 20 C-Atomen,

einen gegebenenfalls substituierten heteroaromatischen oder heteroaromatisch- ($C_1$-$C_8$)-aliphatischen Rest mit 5 - 12 Ringatomen, oder

einen Rest $OR^a$ oder $SR^a$ bedeutet, worin

$R^a$ für einen gegebenenfalls substituierten aliphatischen Rest mit 1 - 4 C-Atomen, für einen gegebenenfalls substituierten aromatischen Rest mit 6 - 12 C-Atomen oder einen gegebenenfalls substituierten heteroaromatischen Rest mit 5 - 12 Ringatomen steht,

$R^1$ Wasserstoff,

einen gegebenenfalls substituierten aliphatischen Rest mit 1 - 21 C-Atomen,

einen gegebenenfalls substituierten alicyclischen Rest mit 3 - 20 C-Atomen,

einen gegebenenfalls substituierten alicyclisch-aliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-($C_1$-$C_8$)-aliphatischen Rest mit 5 - 12 Ringatomen oder,
falls nicht von vorstehenden Definitionen schon umfaßt, die erforderlichenfalls geschützte Seitenkette einer natürlich vorkommenden α-Aminosäure bedeutet,
$R^2$ Wasserstoff,
einen gegebenenfalls substituierten aliphatischen Rest mit 1 - 21 C-Atomen, einen gegebenenfalls substituierten alicyclischen Rest mit 3 - 20 C-Atomen, einen gegebenenfalls substituierten aromatischen Rest mit 6 - 12 C-Atomen,
einen gegebenenfalls substituierten araliphatischen Rest mit 7 - 32 C-Atomen,
einen Rest der Formel

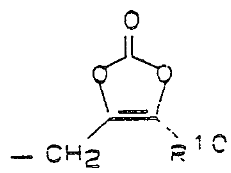

worin $R^{10}$ Wasserstoff, ein aliphatischer Rest mit 1 - 6 C-Atomen oder ein gegebenenfalls substituierter aromatischer Rest mit 6 - 12 C-Atomen ist,
einen Rest der Formel

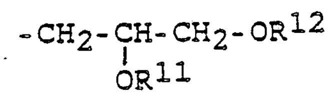

oder

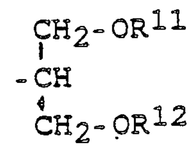

worin $R^{11}$ und $R^{12}$ gleich oder verschieden unabhängig voneinander Wasserstoff, einen gegebenenfalls substituierten Alkylrest mit 1 - 23 C-Atomen oder einen gegebenenfalls substituierten Acylrest mit 1 - 23 C-Atomen bedeuten,
bedeutet,
$R^3$ einen Rest der Formel

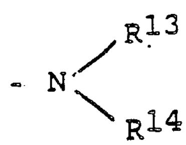

worin $R^{13}$ und $R^{14}$ gleich oder verschieden unabhängig voneinander Wasserstoff, einen gegebenenfalls substituierten aliphatischen Rest mit 1 - 21 C-Atomen, einen gegebenenfalls substituierten alicyclischen Rest mit 3 - 20 C-Atomen, einen gegebenenfalls substituierten aromatischen Rest mit 6 - 12 C-Atomen oder einen gegebenenfalls substituierten araliphatischen Rest mit 7 - 32 C-Atomen bedeuten, oder zusammen mit dem sie tragenden Stickstoffatom einen gegebenenfalls substituierten, gegebenenfalls zugleich teilweise oder vollständig hydrierten, heteroaromatischen Rest mit 2 - 24 C-Atomen bilden, bedeutet
$R^4$ für Wasserstoff oder ($C_1$-$C_6$)Alkyl und
$R^5$ für ($C_1$-$C_6$)Alkyl, ($C_3$-$C_6$)-Cycloalkyl oder

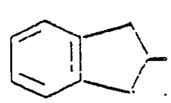

stehen oder
$R^4$ und $R^5$ zusammen mit den sie tragenden Atomen ein mono-, bi-, oder tricyclisches heterocyclisches

Ringsystem mit 3 bis 15 Ring-C-Atomen bilden,
$R^6$ Wasserstoff, Amino, $(C_1-C_6)$-Alkyl, $(C_6-C_{12})$-Aryl oder $(C_7-C_{13})$-Aralkyl,
$R^7$ $(C_1-C_6)$-Alkyl oder $(C_7-C_{13})$-Aralkyl, vorzugsweise $-(CH_2)_4-C_6H_5$,
$R^8$ $(C_1-C_6)$-Alkyl, das gegebenenfalls durch $(C_1-C_6)$-Alkanoyloxy monosubstituiert ist, vorzugsweise 2-Methyl-1-propionyloxy-propyl, und
$R^9$ Wasserstoff oder $(C_1-C_6)$-Alkyl bedeuten;
wie Verbindungen der Formel II,

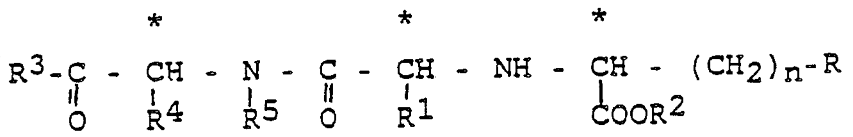 (II)

in welcher
n = 1 oder 2 ist,
R = Wasserstoff
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten alicyclisch-aliphatischen Rest mit 7 - 14 C-Atomen,
einen gegebenenfalls substituierten heteroaromatischen oder heteroaromatisch-$(C_1-C_8)$-aliphatischen Rest mit 5 - 12 Ringatomen, oder
einen Rest $OR^a$ oder $SR^a$ bedeutet, worin
$R^a$ für einen gegebenenfalls substituierten aliphatischen Rest mit 1 - 4 C-Atomen, für einen gegebenenfalls substituierten aromatischen Rest mit 6 - 12 C-Atomen oder einen gegebenenfalls substituierten heteroaromatischen Rest mit 5 - 12 Ringatomen steht,
$R^1$ Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten alicyclisch-aliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-$(C_1-C_8)$-aliphatischen Rest mit 5 - 12 Ringatomen oder,
falls nicht von vorstehenden Definitionen schon umfaßt, die erforderlichenfalls geschützte Seitenkette einer natürlich vorkommenden α-Aminosäure bedeutet,
$R^2$ Wasserstoff,
einen gegebenenfalls substituierten aliphatischen Rest mit 1 - 21 C-Atomen, einen gegebenenfalls substituierten alicyclischen Rest mit 3 - 20 C-Atomen, einen gegebenenfalls substituierten aromatischen Rest mit 6 - 12 C-Atomen,
einen gegebenenfalls substituierten araliphatischen Rest mit 7 - 32 C-Atomen,
einen Rest der Formel

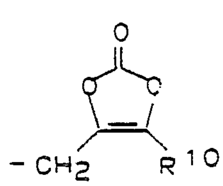

worin $R^{10}$ Wasserstoff, ein aliphatischer Rest mit 1 - 6 C-Atomen oder ein gegebenenfalls substituierter aromatischer Rest mit 6 - 12 C-Atomen ist,
einen Rest der Formel

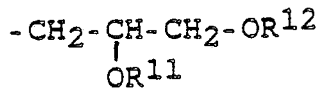 oder 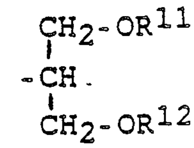

worin $R^{11}$ und $R^{12}$ gleich oder verschieden unabhängig voneinander Wasserstoff, einen gegebenenfalls substituierten Alkylrest mit 1 - 23 C-Atomen oder einen gegebenenfalls substituierten Acylrest mit 1 - 23 C-Atomen bedeuten,
bedeutet,
$R^3$ einen Rest der Formel

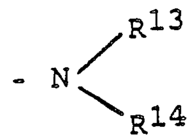

worin $R^{13}$ und $R^{14}$ gleich oder verschieden unabhängig voneinander Wasserstoff, einen gegebenenfalls substituierten aliphatischen Rest mit 1 - 21 C-Atomen, einen gegebenenfalls substituierten alicyclischen Rest mit 3 - 20 C-Atomen, einen gegebenenfalls substituierten aromatischen Rest mit 6 - 12 C-Atomen oder einen gegebenenfalls substituierten araliphatischen Rest mit 7 - 32 C-Atomen bedeuten, oder zusammen mit dem sie tragenden Stickstoffatom einen gegebenenfalls substituierten, gegebenenfalls zugleich teilweise oder vollständig hydrierten, heteroaromatischen Rest mit 2 - 24 C-Atomen bildet, bedeuten, und
$R^4$ und $R^5$ zusammen mit den sie tragenden Atomen ein mono-, bi-, oder tricyclisches heterocyclisches Ringsystem mit 3 bis 15 Ring-C-Atomen bilden,
sowie deren physiologisch verträgliche Salze mit Säuren und Basen.

Unter einem gegebenenfalls substituierten aliphatischen Rest versteht man einen aliphatischen acyclischen Rest, d.h. einen Rest mit einer offenen, geraden oder verzweigten Kohlenstoffkette, wie beispielsweise gegebenenfalls substituertes Alkyl, Alkenyl; Alkinyl und entsprechende mehrfach ungesättigte Reste. Er ist unsubstituiert oder, wie unten beispielsweise bei Carboxy, Carbamoyl, Aminoalkyl, Alkanoylaminoalkyl, Alkoxycarbonylaminoalkyl, Arylalkoxycarbonylaminoalkyl, Arylalkylaminoalkyl, Alkylaminoalkyl, Dialkylaminoalkyl, Alkylthioalkyl, Arylthioalkyl, Carboxyalkyl, Carbamoylalkyl, Alkoxycarbonylalkyl, Alkanoyloxyalkyl, Alkoxycarbonyloxyalkyl, Aroyloxyalkyl oder Aryloxycarbonyloxyalkyl beschrieben, monosubstituiert.

Ein gegebenenfalls substituierter alicyclischer Rest und der über eine offene Kohlenstoffkette verknüpfte entsprechende gegebenenfalls substituierte alicyclisch-aliphatische Rest ist ein vorzugsweise mono- bis pentacyclischer isocyclischer, nicht aromatischer Rest mit Einfach- oder unsymmetrisch verteilten Doppelbindungen, der auch verzweigt sein (d.h. offenkettige aliphatische Seitenketten tragen) kann, und der über ein Ring-C-Atom oder ein Seitenketten-C-Atom verknüpft ist. Er ist vorzugsweise unsubstituiert. Mehrere Ringe als Komponenten eines solchen Restes sind kondensiert, spiroverknüpft oder isoliert. Beispiele für solche Reste sind Cycloalkyl, Cycloalkenyl, Cycloalkylalkyl, Bicycloalkyl, Tricycloalkyl und von mono-, bi- oder oligocyclischen Terpenen abgeleitete Reste, wie Menthyl, Isomenthyl, Bornyl, Caranyl, Epibornyl, Epiisobornyl, Isobornyl, Norbonyl, Neomenthyl, Neoisomenthyl, Pinanyl, Thujanyl; sie sind vorzugsweise unsubstituiert (aliphatische Seitenketten sind nach vorliegender Definition keine Substituenten).

Ein gegebenenfalls substituierter aromatischer Rest ist vorzugsweise Aryl wie Phenyl, Biphenylyl oder Naphthyl, das gegebenenfalls wie unten bei Aryl angegeben mono-, di- oder trisubstituiert ist. Von Aryl abgeleitete Reste, wie Aralkyl, Aryloxy, Arylthio oder Aroyl, vorzugsweise Benzoyl, können wie Aryl substituiert sein.

Ein gegebenenfalls substituierter heteroaromatischer Rest ist vorzugsweise ein aromatischer mono- bzw. bicyclischer Heterocyclen-Rest mit 5 bis 7 bzw. 8 bis 12, bevorzugt bis 10 Ringatomen, wovon 1 bis 2 Ringatome Schwefel- oder Sauerstoffatome und/oder wovon 1 bis 4 Ringatome Stickstoffatome darstellen, wie beispielsweise Thienyl, Benzo[b]thienyl, Furyl, Pyranyl, Benzofuryl, Pyrrolyl, Imidazolyl, Pyrazolyl, Pyridyl, Pyrimidinyl, Pyridazinyl, Indazolyl, Isoindolyl, Indolyl, Purinyl, Chinolizinyl, Isochinolinyl, Phthalazinyl, Naphthyridinyl, Chinoxalinyl, Chinazolyl, Cinnolinyl, Pteridinyl, Oxazolyl, Isoxazolyl, Thiazolyl oder Isothiazolyl. Diese Reste können auch teilweise oder vollständig hydriert sein. Ein heteroaromatischer Rest und der entsprechende heteroaromatisch-aliphatische Rest kann wie unten definiert, substituiert sein.

Unter einem gegebenenfalls substituierten araliphatischen Rest werden insbesondere Phthalidyl und Arylalkyl, Diarylalkyl, Indanyl oder Fluorenyl verstanden, worin Aryl wie oben definiert ist und in der dort

angegebenen Weise substituiert sein kann.

Ein gegebenenfalls substituierter Acylrest ist vorzugsweise ein geradkettiger oder verzweigter, gesättigter oder ungesättigter aliphatischer Rest, insbesondere gesättigtes oder ungesättigtes, vorzugsweise unsubstituiertes Alkanoyl, wie Formyl, Acetyl, Propionyl, Butyryl, Isobutyryl, Valeryl, Isovaleryl, Pivaloyl, Lauroyl, Myristoyl, Palmitoyl, Stearoyl, Acryloyl, Propiolyl, Methacryloyl, Crotonoyl, Isocrotonoyl, Oleoyl, Elaioyl, Arachidonoyl, Caprinoyl, Caproyl, Capryloyl oder Ricinoloyl. Bevorzugt sind Reste von Fettsäuren, wie sie in natürlichen Triglyceriden vorkommen.

$R^4$ und $R^5$ können mit den diese tragenden Atomen ein mono-, bi- oder tricyclisches heterocyclisches Ringsystem mit 3 bis 15 Ring-C-Atomen bilden, das im Ring vorzugsweise bis zu 2 S-Atome und bis zu 2 N-Atome, insbesondere bis zu 1 S-Atom aufweist, welches durch $(C_1-C_4)$-Alkyl, $(C_1-C_4)$- Alkoxy, Hydroxy, Halogen, Nitro, Amino, Aminomethyl, $(C_1-C_4)$-Alkylamino, Di-$(C_1-C_4)$-alkylamino, $(C_1-C_4)$-Alkanoylamino, Methylendioxy, Carboxy, Cyano, Sulfanoyl, $(C_6-C_{12})$-Aryl und/oder $(C_1-C_9)$-Heteroaryl mono-, di- oder trisubstituiert sein kann, wobei Aryl und/oder Heteroaryl seinerseits gegebenenfalls durch $(C_1-C_4)$-Alkyl, $(C_1-C_4)$-Alkoxy, Hydroxy, Halogen, Nitro, Amino, Aminomethyl, $(C_1-C_4)$-Alkylamino, Di-$(C_1-C_4)$-alkylamino, $(C_1-C_4)$-Alkanoylamino, Methylendioxy, Carboxy, Cyano oder Sulfamoyl substituiert ist.

Als solche Ringsysteme kommen insbesondere jene aus der folgenden Gruppe in Betracht: Tetrahydroisochinolin (A); Decahydroisochinolin (B); Octahydroindol (C); Octahydrocyclopenta-[b]pyrrol (D); 2-Azaspiro[4.5]decan (E); 2-Azaspiro[4.4]-nonan (F); Spiro[(bicyclo[2.2.1]heptan)-2,3′-pyrrolidin] (G); Spiro[-(bicyclo[2.2.2]octan)-2,3′-pyrrolidin] (H); 2-Azatricyclo[4.3.0.1$^{6,9}$]decan (I); Decahydrocyclohepta[b]pyrrol (J); Hexahydrocyclopropa[b]pyrrol (K); Octahydroisoindol (L); Octahydrocyclopenta[c]pyrrol (M) 2,3,3a,4,5,7a-Hexahydroindol (N); 1,2,3,3a,4,6a-Hexahydrocyclopenta[b]pyrrol (O); Pyrrolidin (P); Indolin (Q); Thiazolidin (R), die alle gegebenenfalls substituiert sein können. Pyrrolidin (P) und Thiazolidin (R) können z.B. durch $(C_6-C_{12})$-Aryl (Phenyl, 2-Hydroxyphenyl, u.a.), $(C_6-C_{12})$-Arylmercapto (wie Phenylmercapto) oder $(C_3-C_7)$-Cycloalkyl (wie Cyclohexyl) monosubstituiert sein. Tetrahydroisochinolin (A) kann z.B. im Arylteil bis zu 2 $(C_1-C_6)$Alkoxyreste, vorzugsweise Methoxyreste tragen. Entsprechendes gilt für die anderen Ringsysteme. Bevorzugt sind jedoch die unsubstituierten Systeme.

Die in Betracht kommenden heterocyclischen Ringsysteme weisen die folgenden Strukturformeln auf:

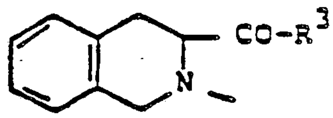

A

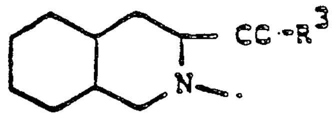

B

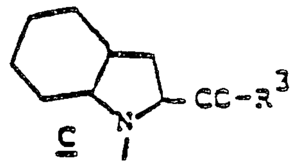

C

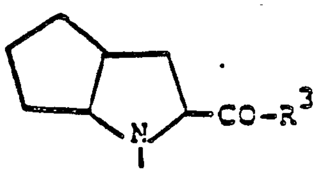

D

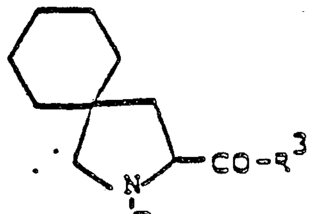

E

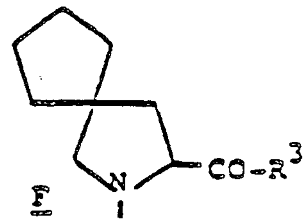

F

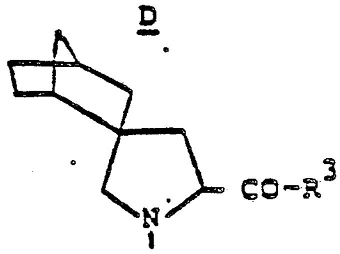

G

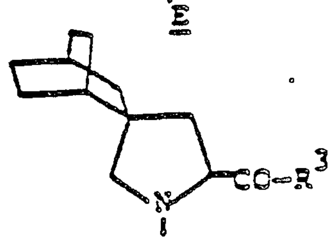

H

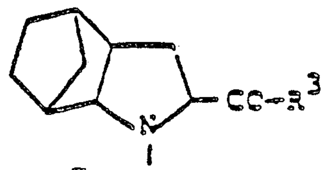

I

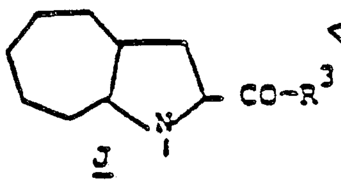

J

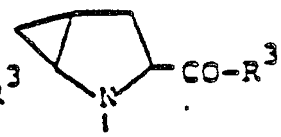

K

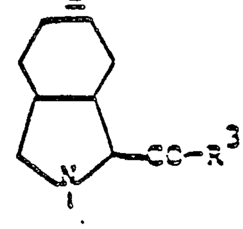

L

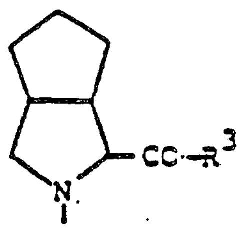

M

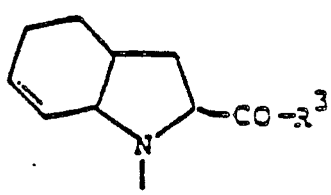

N

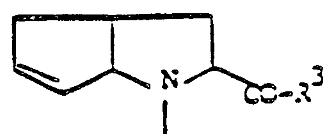

O

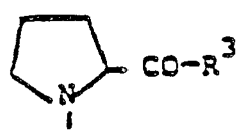

P

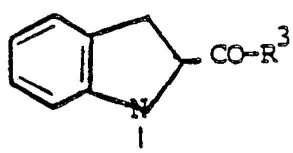

Q

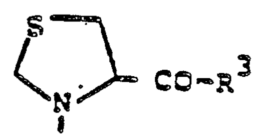

R

Natürlich vorkommende α-Aminosäuren sind beispielsweise Ala, Ser, Thr, Val, Leu, Ile, Asp, Asn, Glu, Gln, Arg, Lys, Hyl, Orn, Cit, Tyr, Phe, Trp und His.

Falls $R^1$ für eine Seitenkette einer geschützten natürlich vorkommenden α-Aminosäure steht, wie z.B. geschütztes Ser, Thr, Asp, Asn, Glu, Gln, Arg, Lys, Hyl, Cys, Orn, Cit, Tyr, Trp oder His, sind als Schutzgruppen die in der Peptidchemie üblichen Gruppen bevorzugt (vgl. Houben-Weyl, Bd. XV/1 und XV/2). Im Falle, daß $R^1$ die geschützte Lysin-Seitenkette bedeutet, werden die bekannten Amino-Schutzgruppen, insbesondere aber Z, Boc oder ($C_1$-$C_6$) Alkanoyl bevorzugt. Als O-Schutzgruppen für Tyrosin kommen bevorzugt ($C_1$-$C_6$)-Alkyl, insbesondere Methyl oder Ethyl in Frage.

Die Verbindungen der Formel I bzw. II weisen asymmetrische Kohlenstoffatome auf und können daher als Enantiomere und Diastereomere auftreten. Die Erfindung umfaßt sowohl die reinen Enantiomeren als

auch die Racemate.

Bei Verbindungen der Formel I bzw. II, die mehrere chirale Atome besitzen, kommen alle möglichen Diastereomere als Racemate oder Enantiomere, oder Gemische verschiedener Diastereomere in Betracht.

Eine bevorzugte Ausführungsform ist dadurch gekennzeichnet, daß Verbindungen der Formel I, vorzugsweise solche der Formel II verwendet werden, in welchen

a) n = 1 oder 2 ist;

b) R

1. Wasserstoff bedeutet;

2. Alkyl mit 1 - 18 C-Atomen bedeutet;

3. einen aliphatischen acyclischen Rest der Formel $C_aH_{(2a-b+1)}$ bedeutet, worin Doppelbindungen, falls ihre Zahl 1 übersteigt, nicht kumuliert sind, a für eine ganze Zahl 2 bis 18 und b für eine gerade Zahl 2 bis a stehen;

4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (C-2) stehen;

5. Aryl mit 6 - 12 C-Atomen bedeutet,
das durch ($C_1$-$C_8$)-Alkyl, ($C_1$-$C_4$)-Alkoxy, Hydroxy, Halogen, Nitro, Amino, Aminomethyl, ($C_1$-$C_4$)-Alkylamino, Di-($C_1$-$C_4$)-alkylamino, ($C_1$-$C_4$)-Alkanoylamino, Methylendioxy, Carboxy, Cyano und/oder Sulfamoyl, mono-, di- oder trisubstituiert sein kann;

6. falls n = 2 ist, ($C_6$-$C_{12}$)-Aryl-($C_1$-$C_8$)-alkyl oder Di-($C_6$-$C_{12}$)-aryl-($C_1$-$C_8$)-alkyl bedeutet, die im Arylteil jeweils wie unter b) 5. beschrieben substituiert sein können; oder

7. Alkoxy mit 1 - 4 C-Atomen;

8. Aryloxy mit 6 - 12 C-Atomen,
das wie unter b) 5. beschrieben substituiert sein kann;

9. mono- bzw. bicyclisches Heteroaryloxy oder Heteroaryl-($C_1$-$C_8$)-alkyl mit 5 - 7 bzw. 8 - 10 Ringatomen, wovon bis zu 9 Ringatome Kohlenstoff und 1 bis 2 Ringatome Schwefel oder Sauerstoff und/oder 1 bis 4 Ringatome Stickstoff darstellen, die im Heteroaryl wie unter b) 5. beschrieben substituiert sein können;

10. Amino-($C_1$-$C_8$)-alkyl;

11. ($C_1$-$C_4$)-Alkanoylamino-($C_1$-$C_8$)-alkyl;

12. ($C_7$-$C_{13}$)-Aroylamino-($C_1$-$C_8$)-alkyl;

13. ($C_1$-$C_4$)-Alkoxy-carbonylamino-($C_1$-$C_8$)-alkyl;

14. ($C_6$-$C_{12}$)-Aryl-($C_1$-$C_4$)-alkoxycarbonylamino-($C_1$-$C_8$)-alkyl;

15. ($C_6$-$C_{12}$)-Aryl-($C_1$-$C_4$)-alkylamino-($C_1$-$C_8$)-alkyl;

16. ($C_1$-$C_4$)-Alkylamino-($C_1$-$C_8$)-alkyl;

17. Di-($C_1$-$C_4$)-alkylamino-($C_1$-$C_8$)-alkyl;

18. Guanidino-($C_1$-$C_8$)-alkyl;

19. Imidazolyl;

20. Indolyl;

21. ($C_1$-$C_4$)-Alkylthio;

22. falls n = 2 ist, ($C_1$-$C_4$)-Alkylthio-($C_1$-$C_8$)-alkyl;

23. ($C_6$-$C_{12}$)-Arylthio-($C_1$-$C_8$)-alkyl,
das im Arylteil wie unter b) 5. beschrieben, substituiert sein kann;

24. ($C_6$-$C_{12}$)-Aryl-($C_1$-$C_8$)-alkylthio,
das im Arylteil wie unter b) 5. beschrieben substituiert sein kann;

25. falls n = 2 ist, Carboxy-($C_1$-$C_8$)-alkyl;

26. Carboxy;

27. Carbamoyl;

28. falls n = 2 ist, Carbamoyl-($C_1$-$C_8$)-alkyl;

29. ($C_1$-$C_4$)-Alkoxy-carbonyl-($C_1$-$C_8$)-alkyl;

30. falls n = 2 ist, ($C_6$-$C_{12}$)-Aryloxy-($C_1$-$C_8$)-alkyl,
das im Arylteil wie unter b) 5. beschrieben substituiert sein kann; oder

31. ($C_6$-$C_{12}$)-Aryl-($C_1$-$C_8$)-alkoxy,
das im Arylteil wie unter b) 5. beschrieben substituiert sein kann, bedeutet;

c) $R^1$

1. Wasserstoff bedeutet;

2. Alkyl mit 1 - 18 C-Atomen bedeutet;

3. einen aliphatischen acyclischen Rest der Formel $C_aH_{(2a-b+1)}$ bedeutet, worin Doppelbindungen, falls ihre Zahl 1 übersteigt, nicht kumuliert sind, a für eine ganze Zahl 2 bis 18 und b für eine gerade Zahl 2 bis a stehen;

4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (c-2) stehen; oder

5. Aryl mit 6 - 12 C-Atomen,
das wie unter b) 5. beschrieben substituiert sein kann;

6. $(C_6-C_{12})$-Aryl-$(C_1-C_8)$-alkyl oder $(C_7-C_{14})$-Aroyl-$(C_1-C_8)$-alkyl,
die beide im Arylteil wie unter b) 5. beschrieben substituiert sein können;

7. mono- bzw. bicyclisches, gegebenenfalls teilhydriertes Heteroaryl oder Heteroaryl-$(C_1-C_8)$-alkyl mit 5 - 7 bzw. 8 - 10 Ringatomen, wovon bis zu 9 Ringatome Kohlenstoff und 1 bis 2 Ringatome Schwefel- oder Sauerstoffatome und/oder 1 bis 4 Ringatome Stickstoffatome darstellen, die im Heteroaryl wie bei Aryl unter b) 5. beschrieben substituiert sein können; oder

8. falls von c) 1. -7. noch nicht umfaßt, die gegebenenfalls geschützte Seitenkette einer natürlich vorkommenden α-Aminosäure der Formel $R^1$-CH($NH_2$)-COOH bedeutet;

d) $R^2$

1. Wasserstoff bedeutet;
2. Alkyl mit 1 - 18 C-Atomen bedeutet;
3. einen aliphatischen acyclischen Rest der Formel $c_aH_{(2a-b+1)}$ bedeutet, worin Doppelbindungen, falls ihre Zahl 1 übersteigt, nicht kumuliert sind, a für eine ganze Zahl 2 bis 18 und b für eine gerade Zahl 2 bis a stehen;
4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist; worin c für eine ganze Zahl 3 bis 20 und d für eine gerade Zahl 0 bis (c-2) stehen; oder

5. Di-$(C_1-C_4)$-alkylamino-$(C_1-C_8)$-alkyl;

6. $(C_1-C_5)$-Alkanoyloxy-$(C_1-C_8)$-alkyl;

7. $(C_1-C_6)$-Alkoxy-carbonyloxy-$(C_1-C_8)$-alkyl;

8. $(C_7-C_{13})$-Aroyloxy-$(C_1-C_8)$-alkyl;

9. $(C_6-C_{12})$-Aryloxycarbonyloxy$(C_1-C_8)$-alkyl;

10. Aryl mit 6 - 12 C-Atomen;

11. $(C_7-C_{20})$-Aralkyl;

12. Phthalidyl;

13. einen Rest der Formel

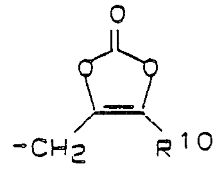

worin $R^{10}$ Wasserstoff, $(C_1-C_6)$-Alkyl oder Aryl mit 6 - 12 C-Atomen ist;

14. einen Rest der Formel

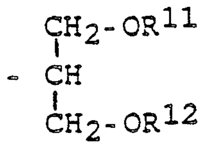

bedeutet,
worin $R^{11}$ und $R^{12}$ gleich oder verschieden unabhängig voneinander Wasserstoff, einen gegebenenfalls substituierten Alkylrest mit 1 - 23 C-Atomen oder einen gegebenenfalls substituierten Acylrest mit 1 - 23 C-Atomen bedeuten
wobei die unter d) 8., 9., 10., 11. und 12. genannten Reste wie unter b) 5. beschrieben im Arylteil substituiert sein können;

e) $R^3$ einen Rest der Formel

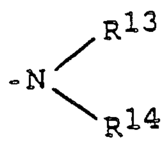

bedeutet,
worin $R^{13}$ und $R^{14}$ gleich oder verschieden unabhängig voneinander
1. Wasserstoff bedeuten;
2. Alkyl mit 1 - 21 C-Atomen bedeuten;
3. einen aliphatischen acyclischen Rest der Formel $C_aH_{(2a-b+1)}$ bedeuten, worin a für eine ganze Zahl 2 bis 21 und b für eine gerade Zahl 2 bis a stehen;
4. einen mono-, di-, tri-, tetra- oder pentacyclischen, nicht aromatischen Kohlenwasserstoffrest der Formel $C_cH_{(2c-d-1)}$ bedeuten, der gegebenenfalls verzweigt ist, worin c für eine ganze Zahl 3 - 20 und d für eine gerade Zahl 0 bis (c-2) stehen; oder
5. Amino-($C_1$-$C_{16}$)-alkyl;
6. Mono-($C_1$-$C_4$)-alkylamino-($C_1$-$C_{16}$)alkyl;
7. Di-($C_1$-$C_4$)-alkylamino-($C_1$-$C_{13}$)alkyl;
8. ($C_1$-$C_5$)-Alkanoyloxy-($C_1$-$C_{16}$)-alkyl;
9. ($C_1$-$C_6$)-Alkoxy-carbonyloxy-($C_1$-$C_{15}$)-alkyl;
10. ($C_7$-$C_{13}$)-Aroyloxy-($C_1$-$C_8$)-alkyl;
11. ($C_6$-$C_{12}$)-Aryloxy-carbonyloxy-($C_1$-$C_9$)alkyl;
12. Aryl mit 6 - 12 C-Atomen, oder
13. ($C_7$-$C_{20}$)-Aralkyl bedeuten

oder zusammen mit dem sie tragenden Stickstoffatom einen gegebenenfalls substituierten, gegebenenfalls teilweise oder vollständig hydrierten heteroaromatischen Rest mit 2 - 24 C-Atomen bilden
und

f) $R^4$ und $R^5$ zusammen mit den sie tragenden Atomen ein mono-, bi- oder tricyclisches heterocyclisches Ringsystem mit 3 bis 15 Ring C-Atomen bilden.

Eine besonders bevorzugte Ausführungsform ist dadurch gekennzeichnet, daß Verbindungen der Formel I, vorzugsweise solche der Formel II verwendet werden, in welchen
n = 1 oder 2 ist
R = Wasserstoff,
Alkyl mit 1 - 8 C-Atomen,
Alkenyl mit 2 - 6 C-Atomen,
Cycloalkyl mit 3 9 C-Atomen,
Aryl mit 6 - 12 C-Atomen,
das durch ($C_1$-$C_4$)-Alkyl, ($C_1$-$C_4$)-Alkoxy, Hydroxy, Halogen, Nitro, Amino, Aminomethyl, ($C_1$-$C_4$)-Alkylamino, Di-($C_1$-$C_4$)-alkylamino, ($C_1$-$C_4$)-Alkanoylamino, Methylendioxy, Carboxy, Cyano und/oder Sulfamoyl, mono-, di- oder trisubstituiert sein kann,
Alkoxy mit 1 - 4 C-Atomen,
Aryloxy mit 6 - 12 C-Atomen,

das wie oben bei Aryl beschrieben substituiert sein kann,
mono- bzw. bicyclisches Heteroaryloxy mit 5 - 7 bzw. 8 - 10 Ringatomen, wovon 1 bis 2 Ringatome Schwefel-oder Sauerstoffatome und/oder 1 bis 4 Ringatome Stickstoff darstellen,
das wie oben bei Aryl beschrieben substituiert sein kann,
Amino-($C_1$-$C_4$)-alkyl,
($C_1$-$C_4$)-Alkanoylamino-($C_1$-$C_4$)alkyl,
($C_7$-$C_{13}$)-Aroylamino-($C_1$-$C_4$)-alkyl,
($C_1$-$C_4$)-Alkoxy-carbonylamino-($C_1$-$C_4$)-alkyl,
($C_6$-$C_{12}$)-Aryl-($C_1$-$C_4$)-alkoxycarbonylamino-($C_1$-$C_4$)-alkyl,
($C_6$-$C_{12}$)-Aryl-($C_1$-$C_4$)-alkylamino-($C_1$-$C_4$)-alkyl,
($C_1$-$C_4$)-Alkylamino-($C_1$-$C_4$)-alkyl,
Di-($C_1$-$C_4$)-alkylamino-($C_1$-$C_4$)-alkyl,
Guanidino-($C_1$-$C_4$)-alkyl,
Imidazolyl, Indolyl,
($C_1$-$C_4$)-Alkylthio,
($C_1$-$C_4$)-Alkylthio-($C_1$-$C_4$)-alkyl,
($C_6$-$C_{12}$)-Arylthio-($C_1$-$C_4$)-alkyl,
das im Arylteil wie oben bei Aryl beschrieben, substituiert sein kann,
($C_6$-$C_{12}$)-Aryl-($C_1$-$C_4$)-alkylthio,
das im Arylteil wie oben bei Aryl beschrieben substituiert sein kann,
Carboxy-($C_1$-$C_4$)-alkyl,
Carboxy, Carbamoyl,
Carbamoyl-($C_1$-$C_4$)-alkyl,
($C_1$-$C_4$)-Alkoxy-carbonyl-($C_1$-$C_4$)-alkyl,
($C_6$-$C_{12}$)-Aryloxy-($C_1$-$C_4$)-alkyl,
das im Arylteil wie oben bei Aryl beschrieben substituiert sein kann oder
($C_6$-$C_{12}$)-Aryl-($C_1$-$C_4$)-alkoxy,
das im Arylteil wie oben bei Aryl beschrieben substituiert sein kann,
$R^1$ Wasserstoff,
Alkyl mit 1 - 6 C-Atomen,
Alkenyl mit 2 - 6 C-Atomen,
Alkinyl mit 2 - 6 C-Atomen,
Cycloalkyl mit 3 - 9 C-Atomen,
Cycloalkenyl mit 5 - 9 C-Atomen,
($C_3$-$C_9$)-Cycloalkyl-($C_1$-$C_4$)-alkyl,
($C_5$-$C_9$)-Cycloalkenyl-($C_1$-$C_4$)-alkyl,
gegebenenfalls teilhydriertes Aryl mit 6 - 12 C-Atomen,
das wie oben bei R beschrieben substituiert sein kann,
($C_6$-$C_{12}$)-Aryl-($C_1$-$C_4$)-alkyl oder ($C_7$-$C_{13}$)-Aroyl-($C_1$ oder $C_2$)alkyl,
die beide wie das vorstehende Aryl substituiert sein können,
mono- bzw. bicyclisches, gegebenenfalls teilhydriertes Heteroaryl mit 5 - 7 bzw. 8 - 10 Ringatomen, wovon 1 bis 2 Ringatome Schwefel- oder Sauerstoffatome und/oder 1 bis 4 Ringatome Stickstoffatome darstellen,
das wie das vorstehende Aryl substituiert sein kann oder
die gegebenenfalls geschützte Seitenkette einer natürlich vorkommenden α-Aminosäure $R^1$-$CH(NH_2)$-$COOH$ bedeutet,
$R^2$
Wasserstoff,
Alkyl mit 1 - 6 C-Atomen,
Alkenyl mit 2 - 6 C-Atomen,
Di-($C_1$-$C_4$)-alkylamino-($C_1$-$C_4$)-alkyl,
($C_1$-$C_5$)-Alkanoyloxy-($C_1$-$C_4$)-alkyl,
($C_1$-$C_6$)Alkoxy-carbonyloxy-($C_1$-$C_4$)-alkyl,
($C_7$-$C_{13}$)-Aroyloxy-($C_1$-$C_4$)-alkyl,
($C_6$-$C_{12}$)-Aryloxycarbonyloxy($C_1$-$C_4$)-alkyl,
Aryl mit 6 - 12 C-Atomen,
($C_6$-$C_{12}$)-Aryl-($C_1$-$C_4$)-alkyl,
($C_3$-$C_9$)-Cycloalkyl oder

$(C_3-C_9)$-Cycloalkyl-$(C_1-C_4)$-alkyl,
bedeutet,
$R^3$ einen Rest der Formel

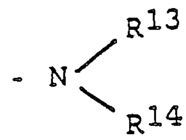

bedeutet,
worin $R^{13}$ und $R^{14}$ gleich oder verschieden unabhängig voneinander Wasserstoff,
Alkyl mit 1 - 16 C-Atomen,
Alkenyl mit 2 - 16 C-Atomen,
Alkinyl mit 2 - 16 C-Atomen,
Cycloalkyl mit 3 - 9 C-Atomen,
$(C_3-C_9)$-Cycloalkyl-$(C_1-C_4)$-alkyl,
Amino-$(C_1-C_{16})$-alkyl,
Mono-$(C_1-C_4)$-alkylamino-$(C_1-C_{12})$alkyl,
Di-$(C_1-C_4)$-alkylamino-$(C_1-C_8)$alkyl,
$(C_1-C_5)$-Alkanoyloxy-$(C_1-C_{11})$-alkyl,
$(C_1-C_6)$-Alkoxycarbonyloxy-$(C_1-C_{10})$-alkyl,
$(C_7-C_{13})$-Aroyloxy-$(C_1-C_6)$-alkyl,
$(C_6-C_{12})$-Aryloxycarbonyloxy-$(C_1-C_6)$-alkyl,
Aryl mit 6 - 12 C-Atomen oder $(C_6-C_{12})$-Aryl-$(C_1-C_6)$-alkyl bedeuten
oder zusammen mit dem sie tragenden Stickstoffatom, gegebenenfalls durch Amino, $(C_1-C_4)$-Alkylamino, Di-$(C_1-C_4)$-alkylamino, $(C_1-C_5)$-Alkanoyloxy, $(C_1-C_6)$-Alkoxycarbonyloxy, $(C_7-C_{13})$-Aryloxy oder $(C_6-C_{12})$-Aryloxycarbonyloxy monosubstituiertes
Pyrrolidinyl,
Piperidinyl,
Piperazinyl,
Homopiperazinyl,
Morpholinyl,
Thiomorpholinyl,
Imidazolyl,
Pyridyl,
Pyrimidinyl,
sym.-Triazinyl bedeuten
und
$R^4$ und $R^5$ die oben angegebene Bedeutung haben.

Eine ganz besonders bevorzugte Ausführungsform ist dadurch gekennzeichnet, daß Verbindungen der Formel I, vorzugsweise solche der Formel II verwendet werden, in welchen
n = 1 oder 2 ist,
R $(C_1-C_6)$-Alkyl, $(C_2-C_6)$-Alkenyl, $(C_3-C_9)$-Cycloalkyl, Amino-$(C_1-C_4)$-alkyl, $(C_2-C_5)$-Acylamino-$(C_1-C_4)$-alkyl, $(C_7-C_{13})$-Aroylamino-$(C_1-C_4)$-alkyl, $(C_1-C_4)$-Alkoxycarbonylamino-$(C_1-C_4)$-alkyl, $(C_6-C_{12})$-Aryl-$(C_1-C_4)$-alkoxycarbonylamino-$(C_1-C_4)$-alkyl, $(C_6-C_{12})$-Aryl, das durch $(C_1-C_4)$-Alkyl, $(C_1-C_4)$-Alkoxy, Hydroxy, Halogen, Nitro, Amino, $(C_1-C_4)$-Alkylamino, Di-$(C_1-C_4)$-alkylamino und/oder Methylendioxy mono-, di- oder trisubstituiert sein kann, oder 3-Indolyl, insbesondere Methyl, Ethyl, Cyclohexyl, tert. Butoxycarbonylamino-$(C_1-C_4)$-alkyl, Benzyloxycarbonylamino-$(C_1-C_4)$-alkyl oder Phenyl, das durch Phenyl, $(C_1-C_2)$-Alkyl, ($C_1$ oder $C_2$)-Alkoxy, Hydroxy, Fluor, Chlor, Brom, Amino, $(C_1-C_4)$-Alkylamino, Di-$(C_1-C_4)$alkylamino, Nitro und/oder Methylendioxy mono- oder disubstituiert oder im Falle von Methoxy, trisubstituiert sein kann, bedeutet,
$R^1$ Wasserstoff oder $(C_1-C_6)$-Alkyl, das gegebenenfalls durch Amino, $(C_1-C_6)$-Acylamino oder Benzoylamino substituiert sein kann, $(C_2-C_6)$-Alkenyl, $(C_3-C_9)$-Cycloalkyl, $(C_5-C_9)$-Cycloalkenyl, $(C_3-C_7)$-Cycloalkyl-$(C_1-C_4)$-alkyl, $(C_6-C_{12})$-Aryl oder teilhydriertes Aryl, das jeweils durch $(C_1-C_4)$-Alkyl, ($C_1$ oder $C_2$)-Alkoxy oder Halogen substituiert sein kann, $(C_6-C_{12})$-Aryl-($C_1$ bis $C_4$)-alkyl oder $(C_7-C_{13})$-Aroyl-$(C_1-C_2)$alkyl, die beide wie vorstehend definiert im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder 1 bis 4 Ringatome Stickstoffatome darstellen, oder eine Seitenkette eine natürlich vorkommenden, gegebenenfalls geschützten α-Aminosäure, insbesondere aber Wasserstoff, $(C_1$-$C_3)$-Alkyl, ($C_2$ oder $C_3$)-Alkenyl, die gegebenenfalls geschützte Seitenkette von Lysin, Phenylalanin oder Tyrosin, Benzyl, 4-Methoxybenzyl, 4-Ethoxybenzyl, Phenethyl, 4-Amino-butyl oder Benzoylmethyl bedeutet,
$R^2$ Wasserstoff,
$(C_1$-$C_6)$-Alkyl, $(C_2$-$C_6)$-Alkenyl, $(C_6$-$C_{12})$-Aryl-$(C_1$-$C_4)$-alkyl, insbesondere aber Wasserstoff, $(C_1$-$C_4)$-Alkyl oder Benzyl bedeutet,
$R^3$ einen Rest der Formel

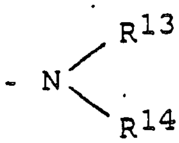

bedeutet,
worin $R^{13}$ und $R^{14}$ gleich oder verschieden unabhängig voneinander Wasserstoff,
$(C_4$-$C_{12})$-Alkyl, $(C_4$-$C_{12})$-Alkenyl, $(C_5$-$C_7)$-Cycloalkyl, $(C_5$-$C_7)$-Cycloalkyl-$(C_1$-$C_4)$-alkyl, Amino-$(C_4$-$C_{12})$-alkyl, $(C_1$-$C_4)$-Alkylamino-$(C_4$-$C_{12})$-alkyl, Di-$(C_1$-$C_4)$-Alkylamino-$(C_1$-$C_8)$alkyl, $(C_6$-$C_{12})$-Aryl-$(C_1$-$C_6)$-alkyl bedeuten oder zusammen mit dem sie tragenden Stickstoffatom gegebenenfalls durch Amino, $(C_1$-$C_4)$-Alkylamino oder Di-$(C_1$-$C_4)$-alkylamino substituiertes Pyrrolidinyl, Piperidinyl, Piperazinyl, Homopiperazinyl, Morpholinyl oder Thiomorpholinyl bedeuten,
und
$R^4$ und $R^5$ die oben angegebene Bedeutung haben.

Besonders vorteilhaft können die folgenden Verbindungen erfindungsgemäß verwendet werden:
2-[N-(1S-Ethoxycarbonyl-3-phenylpropyl)-S-alanyl]-(1S,3S,5S)-2-azabicyclo[3.3.0]octan-3-carbonsäureoctylamid;
2-[N-(1S-Ethoxycarbonyl-3-phenylpropyl)-S-alanyl]-(1S,3S,5S)-2-azabicyclo[3.3.0]octan-3-carbonsäure-(8-amino-octyl)amid;
1-[N-(1S-Ethoxycarbonyl-3-phenylpropyl)-S-alanyl]-3′S-spiro[(bicyclo{2.2.2]octan)-2,3′-pyrrolidin]-5′S-yl-carbonsäure-piperidid;
1-[N-(1S-Ethoxycarbonyl-3-phenylpropyl)-S-alanyl]-3′S-spiro[(bicyclo[2.2.2]octan)-2,3′-pyrrolidin]-5′S-yl-carbonsäure-cyclohexylamid;
1-[N-(1S-Ethoxycarbonyl-3-phenylpropyl)-S-alanyl]-3′S-spiro[(bicyclo[2.2.2]octan)-2,3′-pyrrolidin]-5′S-yl-carbonsäure-N,N-dibutylamid;
1-[N-(1S-Ethoxycarbonyl-3-phenylpropyl)-S-alanyl]-3′S-spiro[(bicyclo[2.2.2]octan)-2,3′-pyrrolidin]-5′S-yl-carbonsäure-octylamid;
1-[N-(1S-Ethoxycarbonyl-3-phenylpropyl)-S-alanyl]-3′S-spiro[(bicyclo[2.2.2]octan)-2,3′-pyrrolidin]-5′S-yl-carbonsäure-hexylamid;
1-[N-(1S-Ethoxycarbonyl-3-phenylpropyl)-S-alanyl]-3′S-spiro[(bicyclo[2.2.2]octan)-2,3′-pyrrolidin]-5′S-yl-carbonsäure-decylamid.
2-[N-(1-S-Carbethoxy-3-phenyl-propyl)-S-alanyl]-S-1,2,3,4-tetrahydroisochinolin-3-carbonsäure-octylamid
2-[N-(1-S-Carbethoxy-3-cyclohexyl-propyl)-S-alanyl]-S-1,2,3,4-tetrahydroisochinolin-3-carbonsäure-hexylamid
2-[N-(1-S-Carbethoxy-3-phenyl-propyl)-S-lysyl]-S-1,2,3,4-tetrahydroisochinolin-3-carbonsäure-(8-amino-octyl)amid
2-[N-(1-S-Carbethoxy-3-phenyl-propyl)-O-ethyl-S-tyrosyl]-S-1,2,3,4-tetrahydroisochinolin-3-carbonsäure-decylamid
2-[N-(1-S-Carbethoxy-3-phenyl-propyl)-S-alanyl]-(3S)-decahydroisochinolin-3-carbonsäure-N,N-dibutylamid
1-[N-(1-S-Carbethoxy-3-phenyl-propyl)-S-alanyl]-(2S,3aS,7aS)-octahydroindol-2-carbonsäure-cyclohexylamid
1-[N-(1-S-Carbethoxy-3-cyclohexyl-propyl)-S-alanyl]-(2S,3aS,7aS)-octahydroindol-2-carbonsäure-piperidid
1-[N-(1-S-Carbethoxy-3-phenyl-propyl)-S-lysyl]-(2S,3aS,7aS)-octahydroindol-2-carbonsäure-octylamid
1-[N-(1-S-Carbethoxy-3-cyclohexyl-propyl)-S-lysyl]-(2S,3aS,7aS)-octahydroindol-2-carbonsäure-hexylamid
1-[N-(1-S-Carbethoxy-3-cyclohexyl-propyl)-S-lysyl]-(2S,3aS,7aS)-octahydroindol-2-carbonsäure-(8-amino-octyl)amid
1-[N-(1-S-Carbethoxy-3-phenyl-propyl)-O-methyl-S-tyrosyl]-(2S,3aS,7aS)-octahydroindol-2-carbonsäure-decylamid
1-[N-(1-S-Carbethoxy-3-phenyl-propyl)-O-ethyl-S-tyrosyl]-(2S,3aS,7aS)-octahydroindol-2-carbonsäure-N,N-dibutylamid

1-[N-(1-S-Carbethoxy-3-(3,4-dimethylphenyl-propyl)-S-alanyl]-(2S,3aS,7aS)-octahydroindol-2-carbonsäure-cyclohexylamid
1-[N-(1-S-Carbethoxy-3-(4-fluorphenyl)-propyl]-S-alanyl]-(2S,3aS,7aS)-octahydroindol-2-carbonsäure-piperidid
1-[N-[1-S-Carbethoxy-3-(4-methoxyphenyl)-propyl]-S-alanyl]-(2S,3aS,7aS)-octahydroindol-2-carbonsäure-octylamid
1-[N-(1-S-Carbethoxy-3-(3,4-dimethoxyphenyl)-propyl]-S-alanyl]-(2S,3aS,7aS)-octahydroindol-2-carbonsäure-hexylamid
1-[N-(1-S-Carbethoxy-3-cyclopentylpropyl)-S-alanyl]-(2S,3aS,7aS)-octahydroindol-2-carbonsäure-(8-amino-octyl)amid
1-[N-(1-S-Carbethoxy-3-phenyl-propyl)-S-alanyl]-(2S,3aR,7aS)-octahydroindol-2-carbonsäure-decylamid
1-[N-(1-S-Carbethoxy-3-cyclohexyl-propyl]-S-alanyl]-(2S,3aR,7aS)-octahydroindol-carbonsäure-N,N-dibutylamid
1-[N-(1-S-Carbethoxy-3-phenyl-propyl]-S-lysyl]-(2S,3aR,7aS)-octahydroindol-2-carbonsäure-cyclohexylamid
1-[N-(1-S-Carbethoxy-3-cyclohexyl-propyl)-S-lysyl]-(2S,3aR,7aS)-octahydroindol-2-carbonsäure-piperidid
1-[N-(1-S-Carbethoxy-3-phenyl-propyl]-O-ethyl-S-tyrosyl]-(2S,3aS,7aR)-octahydroindol-2-carbonsäure-octylamid
1-[N-(1-S-Carbethoxy-3-phenyl-propyl]-S-alanyl]-(2S,3aR,7aR)-octahydroindol-2-carbonsäure-hexylamid
1-[N-(1-S-Carbethoxy-3-phenyl-propyl)-S-lysyl]-(2S,3aR,7aS)-octahydroindol-2-carbonsäure-(8-amino-octyl)-amid
1-[N-(1-S-Carbethoxy-3-cyclohexyl-propyl)-S-alanyl]-(2S,3aR,7aR)-octahydroindol-2-carbonsäure-decylamid
1-[N-(1-S-Carbethoxy-3-cyclohexyl-propyl]-O-ethyl]-S-tyrosyl]-(2S,3aR,7aR)-octahydroindol-2-carbonsäure-N,N-dibutylamid
1-[N-(1-S-Carbethoxy-3-phenyl-propyl)-S-alanyl]-(2S,3aS,7aR)-octahydroindol-2-carbonsäure-cyclohexylamid
1-[N-(1-S-Carbethoxy-3-phenyl-propyl]-O-ethyl-S-tyrosyl]-(2S,3aS,7aS)-octahydroindol-2-carbonsäure-piperidid
1-[N-(1-S-Carbethoxy-3,4-dimethylphenyl-propyl]-S-alanyl]-(2S,3aS,7aS)-octahydroindol-2-carbonsäure-octylamid
1-[N-[1-S-Carbethoxy-3-(4-fluorphenyl)-propyl]-S-alanyl]-(2S,3aS,7aS)-octahydroindol-2-carbonsäure-hexylamid
1-[N-[1-S-Carbethoxy-3-(4-methoxyphenyl)-propyl]-S-alanyl]-(2S,3aS,7aS)-octahydroindol-2-carbonsäure-(8-amino-octyl)amid
1-[N-[1-S-Carbethoxy-3-(3,4-dimethoxyphenyl)-propyl]-S-alanyl]-(2S,3aS,7aS)-octahydroindol-2-carbonsäure-decylamid
1-[N-(1-S-Carbethoxy-3-cyclopentylpropyl)-S-alanyl]-(2S,3aS,7aS)-octahydroindol-2-carbonsäure-N,N-dibutylamid
2-[N-(1-S-Carbethoxy-3-phenyl-propyl)-S-alanyl]-cis-endo-2-azabicyclo[3.3.0]octan-3-S-carbonsäure-cyclohexylamid
2-[N-(1-S-Carbethoxy-3-phenyl-propyl)-S-lysyl]-cis-endo-2-azabicyclo[3.3.0]octan-3-S-carbonsäure-piperidid
2-[N-(1-S-Carbethoxy-3-cyclohexyl-propyl)-S-alanyl]-cis-endo-2-azabicyclo[3.3.0]octan-3-S-carbonsäure-octylamid
2-[N-(1-S-Carboxy-3-cyclohexyl-propyl)-S-alanyl]-cis-endo-2-azabicyclo[3.3.0]octan-3-S-carbonsäure-hexylamid
2-[N-(1-S-Carbethoxy-butyl)-S-alanyl]-cis-endo-2-azabicyclo-[3.3.0]octan-3-S-carbonsäure-(8-amino-octyl)-amid
2-[N-(1-S-Carbethoxy-3-(3,4-dimethoxyphenylpropyl]-S-alanyl]-cis-endo-2-azabicyclo[3.3.0]octan-3-S-carbonsäure-decylamid
2-[N-(1-S-Carbethoxy-3-cyclopentyl-propyl)-S-alanyl]-cis-endo-azabicyclo-[3.3.0]octan-3-S-carbonsäure-N,N-dibutylamid
2-[N-(1-S-Carbethoxy-3-phenyl-propyl]-O-methyl-S-tyrosyl]-cis-endo-2-azabicyclo[3.3.0]octan-3-S-carbonsäure-cyclohexylamid
2-[N-(1-S-Carbethoxy-3-phenyl-propyl]-O-ethyl-S-tyrosyl]-cis-endo-2-azabicyclo[3.3.0]octan-3-S-carbonsäure-piperidid
2-[N-(1-S-Carbethoxy-3-(4-fluorphenyl-propyl]-S-alanyl]-cis-endo-2-azabicyclo[3.3.0]octan-3-S-carbonsäure-octylamid
2-[N-(1-S-Carbethoxy-3-(4-methoxyphenyl-propyl]-S-alanyl]-cis-endo-2-azabicyclo[3.3.0]octan-3-S-carbonsäure-hexylamid
1-[N-(1-S-Carbethoxy-3-phenyl-propyl)-S-lysyl]-(2S,3aR,6aS)-octahydrocylopenta[b]pyrrol-2-carbonsäure-(8-amino-octyl)amid
1-[N-(1-S-Carbethoxy-3-cyclohexylpropyl)-S-lysyl]-(2S,3aR,6aS)-octahydrocyclopenta[b]pyrrol-2-carbonsäure-decylamid
1-[N-(1-S-Carbethoxy-3-phenyl-propyl)-O-ethyl-S-tyrosyl]-(2S,3aR,6aS)-octahydrocyclopenta[b]pyrrol-2-carbonsäure-N,N-dibutylamid
1-[N-(1-S-Carbethoxy-3-phenyl-propyl)-S-alanyl]-2-(2S,3aR,6aS)-octahydrocyclopenta[b]pyrrol-2-carbonsäure-cyclohexylamid
2-[N-(1-S-Carbethoxy-3-phenyl-propyl)-S-alanyl]-2-azaspiro-[4.5]decan-3-S-carbonsäure-piperidid
2-[N-(1-S-Carbethoxy-3-phenyl-propyl)-O-ethyl-2-tyrosyl]-2-azaspiro-[4.5]decan-3-S-carbonsäure-octylamid
2-[N-(1-S-Carbethoxy-3-phenyl-propyl)-S-lysyl]-2-azaspiro-[4.5]decan-3-S-carbonsäure-hexylamid
2-[N-(1-S-Carbethoxy-3-cyclohexylpropyl)-S-alanyl]-2-azaspiro[4.5]decan-3-S-carbonsäure-(8-amino-octyl)-amid
2-[N-(1-S-Carbethoxy-3-cyclohexylpropyl)-S-lysyl]-2-azaspiro[4.5]decan-3-S-carbonsäure-decylamid
2-[N-(1-S-Carbethoxy-3-phenyl-propyl)-S-alanyl]-2-azaspiro-[4.4]nonan-3-S-carbonsäure-N,N-dibutylamid
2-[N-(1-S-Carbethoxy-3-phenyl-propyl)-O-ethyl-S-tyrosyl]-2-azaspiro[4.4]nonan-3-S-carbonsäure-cyclohexylamid
2-[N-(1-S-Carbethoxy-3-phenyl-propyl)-S-lysyl]-2-azaspiro-[4.4]nonan-3-S-carbonsäure-piperidid
2-[N-(1-S-Carbethoxy-3-cyclohexyl-propyl)-S-alanyl]-2-azaspiro[4.4]nonan-3-S-carbonsäure-octylamid
2-[N-(1-S-Carbethoxy-3-cyclopentyl-propyl)-S-alanyl]-2-azaspiro[4.4]nonan-3-S-carbonsäure-hexylamid
2-[N-(1-S-Carbethoxy-3-cyclopentyl-propyl)-S-lysyl]-2-azaspiro[4.4]nonan-3-S-carbonsäure-(8-amino-octyl)-amid
1'-[N-(1-S-Carbethoxy-3-phenyl-propyl)-S-alanyl]-spiro[bicyclo[2.2.1]heptan-2,3'-pyrrolidin]-5'-S-carbonsäure-decylamid
1'-[N-(1-S-Carbethoxy-3-phenyl-propyl)-O-ethyl-S-tyrosyl]-spiro[bicyclo[2.2.1]heptan-2,3'-pyrrolidin]-5'-S-carbonsäure-N,N-dibutylamid
1'-[N-(1-S-Carbethoxy-3-phenyl-propyl)-S-lysyl]-spiro[bicyclo[2.2.1]heptan-2,3'-pyrrolidin]-5'-S-carbonsäure-cyclohexylamid
1'-[N-(1-S-Carbethoxy-3-cyclohexyl-propyl)-S-alanyl]-spiro[bicyclo[2.2.1]heptan-2,3'-pyrrolidin]5'-S-carbonsäure-piperidid
1'-[N-(1-S-Carbethoxy-3-cyclohexyl-propyl)-S-lysyl]-spiro[bicyclo[2.2.1]heptan-2,3'-pyrrolidin]-5'-S-carbonsäure-octylamid
1'-[N-(1-S-Carbethoxy-3-phenyl-propyl)-S-alanyl]-spiro[bicyclo[2.2.2]octan-2,3'-pyrrolidin]-5'-S-carbonsäure-hexylamid
1'-[N-(1-S-Carbethoxy-3-phenyl-propyl)-O-ethyl-S-tyrosyl]-spiro-[bicyclo[2.2.2]octan-2,3'-pyrrolidin]-5'-S-carbonsäure-(8-amino-octyl)amid
1'-[N-(1-S-Carbethoxy-3-phenyl-propyl)-S-lysyl]-spiro[bicyclo[2.2.2]octan-2,3'-pyrrolidin]-5'-S-carbonsäure-decylamid
1'-[N-(1-S-Carbethoxy-3-cyclohexyl-propyl)-S-alanyl]-spiro[bicyclo[2.2.2]octan-2,3'-pyrrolidin]-5'-S-carbonsäure-N,N-dibutylamid
2-[N-(1-S-Carbethoxy-3-phenyl-propyl)-S-alanyl]-2-azatricyclo[4.3.0.$1^{6,9}$]decan-3-S-carbonsäure-cyclohexylamid
2-[N-(1-S-Carbethoxy-3-phenyl-propyl)-O-ethyl-S-tyrosyl]-2-azatricyclo[4.3.0.$1^{6,9}$]decan-3-S-carbonsäure-piperidid
2-[N-(1-S-Carbethoxy-3-phenyl-propyl)-S-lysyl]-2-azatricyclo[4.3.0.$1^{6,9}$]decan-3-S-carbonsäure-octylamid
2-[N-(1-S-Carbethoxy-3-cyclohexyl-propyl)-S-alanyl]-2-azatricyclo[4.3.0.$1^{6,9}$]decan-3-S-carbonsäure-hexylamid
2-[N-(1-S-Carbethoxy-3-cyclohexyl-propyl)-S-lysyl]-2-azatricyclo[4.3.0.$1^{6,9}$]decan-3-S-carbonsäure-(8-amino-octyl)amid
1-[N-(1-S-Carbethoxy-3-phenyl-propyl)-S-alanyl]-decahydrocyclohepta[b]pyrrol-2-S-carbonsäure-decylamid
1-[N-(1-S-Carbethoxy-3-phenyl-propyl)-O-ethyl-S-tyrosyl]-decahydrocyclohepta[b]pyrrol-2-S-carbonsäure-N,N-dibutylamid
1-[N-(1-S-Carbethoxy-3-phenyl-propyl)-S-lysyl]-decahydrocyclohepta[b]pyrrol-2-S-carbonsäure-cyclohexylamid
1-[N-(1-S-Carbethoxy-3-cyclohexyl-propyl)-S-alanyl]-decahydrocyclohepta[b]pyrrol-2-S-carbonsäure-piperidid
1-[N-(1-S-Carbethoxy-3-cyclohexyl-propyl)-S-lysyl]-decahydrocyclohepta[b]pyrrol-2-S-carbonsäure-octylamid
2-[N-(1-S-Carbethoxy-3-phenyl-propyl)-S-alanyl]-trans-octahydroisoindol-1-S-carbonsäure-hexylamid

2-[N-(1-S-Carbethoxy-3-phenyl-propyl)-S-alanyl]-cis-octahydroisoindol-1-S-carbonsäure-(8-amino-octyl)amid
2-[N-(1-S-Carbethoxy-3-cyclohexyl-propyl)-S-alanyl]-trans-octahydroisoindol-1-S-carbonsäure-decylamid
2-[N-(1-S-Carbethoxy-3-cyclohexyl-propyl)-S-alanyl]-cis-octahydroisoindol-1-S-carbonsäure-N,N-dibutylamid
2-[N-(1-S-Carbethoxy-3-phenyl-propyl)-S-alanyl]-cis-octahydrocyclopenta[c]pyrrol-1-S-carbonsäure-cyclohexylamid
2-[N-(1-S-Carbethoxy-3-cyclohexyl-propyl)-S-alanyl]-cis-octahydrocyclopenta[c]pyrrol-1-S-carbonsäure-benzylester
2-[N-(1-S-Carbethoxy-3-cyclohexyl-propyl)-S-lysyl]-cis-octahydrocyclopenta[c]pyrrol-1-S-carbonsäure-piperidid
1-[N-(1-S-Carbethoxy-3-phenyl-propyl)-S-alanyl]-2,3,3a,4,5,7a-hexahydroindol-cis-endo-1-S-carbonsäure-octylamid
1-[N-(1-S-Carbethoxy-3-phenyl-propyl)-S-lysyl]-2,3,3a,4,5,7a-hexahydroindol-cis-endo-1-S-carbonsäure-hexylamid
2-[N-(1-S-Carbethoxy-3-cyclohexyl-propyl)-S-lysyl]-2-azabicyclo[3.1.0]hexan-3-S-carbonsäure-(8-amino-octyl)amid
2-[N-(1-S-Carboxy-3-phenyl-propyl)-S-lysyl]-2-azabicylo[3.1.0]hexan-cis-endo-3-S-carbonsäure-decylamid
2-[N-(1-S-Carbethoxy-3-cyclopentyl-propyl)-S-alanyl]-2-azabicyclo[3.1.0]hexan-3-carbonsäure-N,N-dibutylamid
2-[N-(1-S-Carbethoxy-3-phenyl-propyl)-S-alanyl]-cis-endo-2-azabicyclo[3.1.0]hexan-3-S-carbonsäure-cyclohexylamid
2-[N-(1-S-Carbethoxy-3-cyclohexyl-propyl)-S-alanyl]-cis-endo-2-azabicyclo[3.1.0]hexan-3-S-carbonsäure-piperidid
1′-[N-(1-S-Carbethoxy-3-phenylpropyl)-S-alanyl]-(3′S,5′S)-spiro-bicyclo[2.2.2]-octan-2,3′-pyrrolidin-5′-carbonsäure-octylamid

Die Verbindungen der Formel I und II sind zum Teil als Hemmstoffe des Angiotensin-Converting-Enzymes (ACE) beziehungsweise Zwischenprodukte bei der Herstellung von solchen Hemmstoffen bekannt und können auch zur Bekämpfung des Bluthochdrucks verschiedener Genese eingesetzt werden. Die Verbindungen der Formeln I und II und Verfahren zu ihrer Herstellung sind teilweise bekannt, beispielsweise aus EP-A-12401 und EP-A-50800. Neue Verbindungen der Formeln I und II werden in analoger Weise hergestellt.

Die Erfindung betrifft auch neue Verbindungen der Formel II,

$$R^3-\underset{\underset{O}{\|}}{C}-\underset{R^4}{\overset{*}{CH}}-\underset{R^5}{N}-\underset{\underset{O}{\|}}{C}-\underset{R^1}{\overset{*}{CH}}-NH-\underset{COOR^2}{\overset{*}{CH}}-(CH_2)_n-R \qquad (II)$$

in welcher

I.

a) n = 1 oder 2 ist;

b) R 1. Wasserstoff bedeutet;

2. Alkyl mit 1 - 18 C-Atomen bedeutet;

3. einen aliphatischen acylischen Rest der Formel $C_aH_{(2a-b+1)}$ bedeutet, worin Doppelbindungen, falls ihre Zahl 1 übersteigt, nicht kumuliert sind, a für eine ganze Zahl 2 bis 18 und b für eine gerade Zahl 2 bis a stehen;

4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (c-2) stehen;

5. Aryl mit 6 - 12 C-Atomen bedeutet, das durch ($C_1$-$C_8$)-Alkyl, ($C_1$-$C_4$)-Alkoxy, Hydroxy, Halogen, Nitro, Amino, Aminomethyl, ($C_1$-$C_4$)-Alkylamino, Di-($C_1$-$C_4$)-alkylamino, ($C_1$-$C_4$)- Alkanoylamino, Methylendioxy, Carboxy, Cyano oder Sulfamoyl monosubstituiert sein kann;

6. falls n = 2 ist, ($C_6$-$C_{12}$)-Aryl-($C_1$-$C_8$)-alkyl oder Di-($C_6$-$C_{12}$)-aryl-($C_1$-$C_8$)-alkyl bedeutet, die im Arylteil jeweils wie unter I. b) 5. beschrieben mono-, di- oder trisubstituiert sein können; oder

7. Alkoxy mit 1 - 4 C-Atomen;

8. Aryloxy mit 6 - 12 C-Atomen,

das wie unter I. b) 6. beschrieben substituiert sein kann,

9. mono- bzw. bicylisches Heteroaryloxy mit 5 - 7 bzw. 8 - 10 Ringatomen, wovon bis zu 9 Ringatome Kohlenstoff und 1 bis 2 Ringatome Schwefel oder Sauerstoff und/oder 1 bis 4 Ringatome Stickstoff darstellen,
die im Heteroaryl wie unter I. b) 6. beschrieben substituiert sein können;
10. Amino-($C_1$-$C_8$)-alkyl;
11. ($C_1$-$C_4$)-Alkanoylamino-($C_1$-$C_8$)-alkyl;
12. ($C_7$-$C_{13}$)-Aroylamino-($C_1$-$C_8$)-alkyl, das im Arylteil wie unter I. b) 5. monosubstituiert sein kann;
13. ($C_1$-$C_4$)-Alkoxy-carbonylamino-($C_1$-$C_8$)-alkyl;
14. ($C_6$-$C_{12}$)-Aryl-($C_1$-$C_4$)-alkoxycarbonylamino-($C_1$-$C_8$)alkyl;
15. ($C_6$-$C_{12}$)-Aryl-($C_1$-$C_4$)-alkylamino-($C_1$-$C_8$)-alkyl;
16. ($C_1$-$C_4$)-Alkylamino-($C_1$-$C_8$)-alkyl;
17. Di-($C_1$-$C_4$)-alkylamino-($C_1$-$C_8$)-alkyl;
18. Guanidino-($C_1$-$C_8$)-alkyl;
19. Imidazolyl;
20. Indolyl;
21. ($C_1$-$C_4$)-Alkylthio;
22. falls n = 2 ist, ($C_1$-$C_4$)-Alkylthio-($C_1$-$C_8$)-alkyl;
23. ($C_6$-$C_{12}$)-Arylthio-($C_1$-$C_8$)-alkyl,
das im Arylteil wie unter I. b) 6. beschrieben substituiert sein kann;
24. ($C_6$-$C_{12}$)-Aryl-($C_1$-$C_8$)-alkylthio,
das im Arylteil wie unter I. b) 6. beschrieben substituiert sein kann;
25. falls n = 2 ist, Carboxy-($C_1$-$C_8$)-alkyl;
26. Carboxy;
27. Carbamoyl;
28. falls n = 2 ist, Carbamoyl-($C_1$-$C_8$)-alkyl;
29. ($C_1$-$C_4$)-Alkoxy-carbonyl-($C_1$-$C_8$)-alkyl;
30. falls n = 2 ist, ($C_6$-$C_{12}$)-Aryloxy-($C_1$-$C_8$)-alkyl, das im Arylteil wie unter I. b) 6. beschrieben substituiert sein kann; oder
31. ($C_6$-$C_{12}$)-Aryl-($C_1$-$C_8$)-alkoxy,
das im Arylteil wie unter I. b) 6. beschrieben substituiert sein kann, bedeutet;

c) $R^1$ 1. Wasserstoff bedeutet;
2. Alkyl mit 1 - 18 C-Atomen bedeutet;
3. einen aliphatischen acylischen Rest der Formel $C_aH_{(2a-b+1)}$ bedeutet, worin Doppelbindungen, falls ihre Zahl 1 übersteigt, nicht kumuliert sind, a für eine ganze Zahl 2 bis 18 und b für eine gerade Zahl 2 bis a stehen;
4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (c-2) stehen; oder
5. Aryl mit 6 - 12 C-Atomen, das wie unter I. b) 6. beschrieben substituiert sein kann;
6. ($C_6$-$C_{12}$)-Aryl-($C_1$-$C_8$)-alkyl oder ($C_7$-$C_{13}$)-Aroyl-($C_1$-$C_8$)-alkyl,
die beide im Arylteil wie unter I. b) 6. beschrieben substituiert sein können;
7. mono- bzw. bicyclisches, gegebenenfalls teilhydriertes Heteroaryl oder Heteroaryl-($C_1$-$C_8$)-alkyl mit 5 - 7 bzw. 8 - 10 Ringatomen, wovon bis zu 9 Ringatome Kohlenstoff und 1 bis 2 Ringatome Schwefel oder Sauerstoff und/oder 1 bis 4 Ringatome Stickstoff darstellen, die im Heteroaryl wie Aryl unter I. b) 6. beschreiben substituiert sein können;
8. falls von c) 1. - 7. noch nicht umfaßt, die gegebenenfalls geschützte Seitenkette einer natürlich vorkommenden α-Aminosäure der Formel $R^1$-CH($NH_2$)-COOH; oder
9. falls von c) 1.-8. noch nicht umfaßt, die gegebenenfalls geschützte Seitenkette von Cyclohexylalanin, 2-Thienylalanin, 3-Thienylalanin, O-($C_3$-$C_5$)-Alkyl-Tyrosin oder C-Phenylglycin bedeutet;

d) $R^2$
1. Wasserstoff bedeutet;
2. Alkyl mit 1 - 18 C-Atomen bedeutet;
3. einen aliphatischen acyclischen Rest der Formel $C_aH_{(2a-b+1)}$ bedeutet, worin Doppelbindungen, falls ihre Zahl 1 übersteigt, nicht kumuliert sind, a für eine ganze Zahl 2 bis 18 und b für eine gerade Zahl 2 bis a stehen;
4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (c-2) stehen; oder

5. Di-($C_1$-$C_4$)-alkylamino-($C_1$-$C_8$)-alkyl;
6. ($C_1$-$C_5$)-Alkanoyloxy-($C_1$-$C_8$)alkyl;
7. ($C_1$-$C_6$)-Alkoxy-carbonyl-($C_1$-$C_8$)-alkyl;
8. ($C_7$-$C_{13}$)-Aroyloxy-($C_1$-$C_8$)-alkyl;
9. ($C_6$-$C_{12}$)-Aryloxycarbonyloxy-($C_1$-$C_8$)-alkyl;
10. Aryl mit 6 - 12 C-Atomen;
11. ($C_7$-$C_{20}$)-Aralkyl,
12. Phthalidyl;
13. einen Rest der Formel

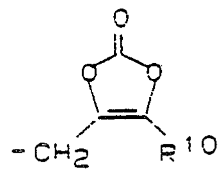

worin $R^{10}$ Wasserstoff, ($C_1$-$C_6$)-Alkyl oder Aryl mit 6 - 12 C-Atomen ist; oder
14. einen Rest der Formel

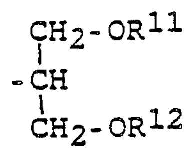

bedeutet,
worin $R^{11}$ und $R^{12}$ gleich oder verschieden unabhängig voneinander Wasserstoff, einen gegebenenfalls substituierten Alkylrest mit 1 - 23 C-Atomen oder einen gegebenenfalls substituierten Acylrest mit 1 - 23 C-Atomen bedeuten,
wobei die unter d) 8., 9., 10. und 11. genannten Reste wie unter I. b) 6. beschrieben im Arylteil substituiert sein können;

e) $R^3$ einen Rest der Formel

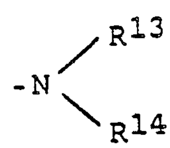

bedeutet,
worin $R^{13}$ und $R^{14}$ gleich oder verschieden unabhängig voneinander
1. Wasserstoff bedeuten;
2. Alkyl mit 1 - 21 C-Atomen bedeuten;
3. einen aliphatischen acyclischen Rest der Formel $C_aH_{(2a-b+1)}$ bedeuten, worin a für eine ganze Zahl 2 bis 21 und b für eine gerade Zahl 2 bis a stehen;
4. einen mono-, di-, tri-, tetra- oder pentacyclischen, nicht aromatischen Kohlenwasserstoffrest der Formel $C_cH_{(2c-d-1)}$ bedeuten, der gegebenenfalls verzweigt ist, worin c für eine ganze Zahl 3 - 20 und d für eine gerade Zahl 0 bis (c-2) stehen; oder
5. Amino-($C_1$-$C_{16}$)-alkyl;
6. Mono-($C_1$-$C_4$)-alkylamino-($C_1$-$C_{16}$)alkyl;
7. Di-($C_1$-$C_4$)-alkylamino-($C_1$-$C_{13}$)alkyl;
8. ($C_1$-$C_5$)-Alkanoyloxy-($C_1$-$C_{16}$)-alkyl;
9. ($C_1$-$C_6$)-Alkoxy-carbonyloxy-($C_1$-$C_{15}$)-alkyl;
10. ($C_7$-$C_{13}$)-Aroyloxy-($C_1$-$C_8$)-alkyl;
11. ($C_6$-$C_{12}$)-Aryloxy-carbonyloxy-($C_1$-$C_9$)alkyl;
12. Aryl mit 6 - 12 C-Atomen, oder
13. ($C_7$-$C_{20}$)-Aralkyl bedeuten
oder zusammen mit dem sie tragenden Stickstoffatom einen gegebenenfalls substituierten, gegebenenfalls

teilweise oder vollständig hydrierten heteroaromatischen Rest mit 2 - 24 C-Atomen bilden und

f) $R^4$ und $R^5$ zusammen mit den sie tragenden Atomen ein mono-, bi- oder tricyclisches heterocyclisches Ringsystem mit 3 bis 15 Ring C-Atomen bilden,

und deren physiologisch verträgliche Salze mit Säuren oder Basen,

II. ausgenommen Verbindungen der Formel II und deren Salze, worin

a) n = 1 oder 2 ist;

b) R 1. Wasserstoff;

2. Alkyl mit 1 - 8 C-Atomen;
3. Alkenyl mit 2 - 8 C-Atomen;
4. Cycloalkyl mit 3 - 9 C-Atomen;
5. Aryl mit 6 - 12 C-Atomen,
das durch ($C_1$-$C_4$)-Alkyl, ($C_1$-$C_4$)-Alkoxy, Hydroxy, Halogen, Nitro, Amino, Aminomethyl, ($C_1$-$C_4$)-Alkylamino, Di-($C_1$-$C_4$)-alkylamino, ($C_1$-$C_4$)-Alkanoylamino, Methylendioxy, Carboxy, Cyano und/oder Sulfamoyl mono-, di- oder trisubstituiert sein kann;
6. Alkoxy mit 1 - 4 C-Atomen;
7. Aryloxy mit 6 - 12 C-Atomen,
das wie unter II. b) 5. beschrieben substituiert sein kann;
8. mono- bzw. bicyclisches Heteroaryloxy mit 5-7 bzw. 8 - 10 Ringatomen, wovon 1 bis 2 Ringatome Schwefel- oder Sauerstoffatome und/oder 1 bis 4 Ringatome Stickstoff darstellen,
das wie unter II. b) 5. beschrieben substituiert sein kann;
9. Amino-($C_1$-$C_4$)-alkyl;
10. ($C_1$-$C_4$)-Alkanoylamino-($C_1$-$C_4$)-alkyl;
11. ($C_7$-$C_{13}$)-Aroylamino-($C_1$-$C_4$)-alkyl;
12. ($C_1$-$C_4$)-Alkoxy-carbonylamino-($C_1$-$C_4$)-alkyl;
13. ($C_6$-$C_{12}$)-Aryl-($C_1$-$C_4$)-alkoxycarbonylamino-($C_1$-$C_4$)-alkyl;
14. ($C_6$-$C_{12}$)-Aryl-($C_1$-$C_4$)-alkylamino-($C_1$-$C_4$)-alkyl;
15. ($C_1$-$C_4$)-Alkylamino-($C_1$-$C_4$)-alkyl;
16. Di-($C_1$-$C_4$)-alkylamino-($C_1$-$C_4$)-alkyl;
17. Guanidino-($C_1$-$C_4$)-alkyl;
18. Imidazolyl;
19. Indolyl;
20. ($C_1$-$C_4$)-Alkylthio;
21. ($C_1$-$C_4$)-Alkylthio-($C_1$-$C_4$)-alkyl;
22. ($C_6$-$C_{12}$)-Arylthio-($C_1$-$C_4$)-alkyl,
das im Arylteil wie unter II. b) 5. beschrieben substituiert sein kann;
23. ($C_6$-$C_{12}$)-Aryl-($C_1$-$C_4$)-alkylthio,
das im Arylteil wie unter II. b) 5. beschrieben substituiert sein kann;
24. Carboxy-($C_1$-$C_4$)-alkyl;
25. Carboxy;
26. Carbamoyl;
27. Carbamoyl-($C_1$-$C_4$)-alkyl;
28. ($C_1$-$C_4$)-Alkoxy-carbonyl-($C_1$-$C_4$)-alkyl;
29. ($C_6$-$C_{12}$)-Aryloxy-($C_1$-$C_4$)-alkyl,
das im Arylteil wie unter II. b) 5. beschrieben substituiert sein kann; oder
30. ($C_6$-$C_{12}$)-Aryl-($C_1$-$C_4$)-alkoxy,
das im Arylteil wie unter II. b) 5. beschrieben substituiert sein kann, bedeutet;

c) $R^1$ 1. Wasserstoff;

2. Alkyl mit 1 - 6 C-Atomen;
3. Alkenyl mit 2 - 6 C-Atomen;
4. Alkinyl mit 2 - 6 C-Atomen;
5. Cycloalkyl mit 3 - 9 C-Atomen;
6. Cycloalkenyl mit 5 - 9 C-Atomen;
7. ($C_3$-$C_9$)-Cycloalkyl-($C_1$-$C_4$)-alkyl;
8. ($C_5$-$C_9$)-Cycloalkenyl-($C_1$-$C_4$)-alkyl;
9. gegebenenfalls teilhydriertes Aryl mit 6 - 12 C-Atomen, das wie II. b) 5. beschrieben substituiert sein kann;

10. $(C_6\text{-}C_{12})$-Aryl-$(C_1\text{-}C_4)$-alkyl oder $(C_7\text{-}C_{13})$-Aroyl-($C_1$ oder $C_2$)-alkyl, die beide wie im Arylteil wie unter II. b) 5. beschrieben substituiert sein können;
11. mono- bzw. bicyclisches, gegebenenfalls teilhydriertes Heteroaryl mit 5 - 7 bzw. 8 - 10 Ringatomen, wovon 1 bis 2 Ringatome Schwefel-oder Sauerstoffatome und/oder 1 bis 4 Ringatome Stickstoffatome darstellen, das wie unter II. b) 5. beschrieben substituiert sein kann; oder
12. falls von den vorstehenden Definitionen nicht umfaßt, die gegebenenfalls geschützte Seitenkette einer natürlich vorkommenden α-Aminosäure der Formel $R^1$-CH($NH_2$)-COOH bedeutet;

d) $R^2$
1. Wasserstoff;
2. Alkyl mit 1 - 6 C-Atomen;
3. Alkenyl mit 2 - 6 C-Atomen;
4. Di-$(C_1\text{-}C_4)$-alkylamino-$(C_1\text{-}C_4)$-alkyl;
5. $(C_1\text{-}C_5)$-Alkanoyloxy-$(C_1\text{-}C_4)$-alkyl;
6. $(C_1\text{-}C_6)$-Alkoxy-carbonyloxy-$(C_1\text{-}C_4)$-alkyl;
7. $(C_7\text{-}C_{13})$-Aroyloxy-$(C_1\text{-}C_4)$-alkyl;
8. $(C_6\text{-}C_{12})$-Aryloxycarbonyloxy$(C_1\text{-}C_4)$-alkyl;
9. Aryl mit 6 - 12 C-Atomen;
10. $(C_6\text{-}C_{12})$-Aryl-$(C_1\text{-}C_4)$-alkyl;
11. $(C_3\text{-}C_9)$-Cycloalkyl; oder
12. $(C_3\text{-}C_9)$-Cycloalkyl-$(C_1\text{-}C_4)$-alkyl bedeutet,

e) $R^3$ einen Rest der Formel

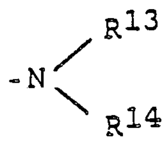

bedeutet,
worin $R^{13}$ und $R^{14}$ gleich oder verschieden unabhängig voneinander
1. Wasserstoff;
2. Alkyl mit 1 - 6 C-Atomen, oder
3. $(C_6\text{-}C_{12})$-Aryl-$(C_1\text{-}C_4)$-alkyl

bedeuten und

f) $R^4$ und $R^5$ zusammen mit den sie tragenden Atomen ein mono-, bi- oder tricyclisches heterocyclisches Ringsystem mit 3 bis 15 Ring-C-Atomen bilden.

Die Erfindung betrifft bevorzugt neue Verbindungen der Formel II, in welchen
n = 1 oder 2 ist
R = Wasserstoff,
Alkyl mit 1 - 8 C-Atomen,
Alkenyl mit 2 - 6 C-Atomen,
Cycloalkyl mit 3 - 9 C-Atomen,
Aryl mit 6 - 12 C-Atomen,
das durch $(C_1\text{-}C_4)$-Alkyl, $(C_1\text{-}C_4)$-Alkoxy, Hydroxy, Halogen, Nitro, Amino, Aminomethyl, $(C_1\text{-}C_4)$-Alkylamino, Di-$(C_1\text{-}C_4)$-alkylamino, $(C_1\text{-}C_4)$-Alkanoylamino, Methylendioxy, Carboxy, Cyano oder Sulfamoyl monosubstituiert sein kann,
Alkoxy mit 1 - 4 C-Atomen,
Aryloxy mit 6 - 12 C-Atomen,
das wie oben bei Aryl beschrieben mono-, di- oder trisubstituiert sein kann,
mono- bzw. bicyclisches Heteroaryloxy mit 5 - 7 bzw. 8 - 10 Ringatomen, wovon 1 bis 2 Ringatome Schwefel-oder Sauerstoffatome und/oder 1 bis 4 Ringatome Stickstoff darstellen,
das wie oben bei Aryloxy beschrieben substituiert sein kann,
Amino-$(C_1\text{-}C_4)$-alkyl,
$(C_1\text{-}C_4)$-Alkanoylamino-$(C_1\text{-}C_4)$alkyl,
$(C_7\text{-}C_{13})$-Aroylamino-$(C_1\text{-}C_4)$-alkyl, das wie oben bei Aryl beschrieben substituiert sein kann,

$(C_1-C_4)$-Alkoxy-carbonylamino-$(C_1-C_4)$-alkyl,
$(C_6-C_{12})$-Aryl-$(C_1-C_4)$-alkoxycarbonylamino-$(C_1-C_4)$-alkyl,
$(C_6-C_{12})$-Aryl-$(C_1-C_4)$-alkylamino-$(C_1-C_4)$-alkyl,
$(C_1-C_4)$-Alkylamino-$(C_1-C_4)$-alkyl,
Di-$(C_1-C_4)$-alkylamino-$(C_1-C_4)$-alkyl,
Guanidino-$(C_1-C_4)$-alkyl,
Imidazolyl, Indolyl,
$(C_1-C_4)$-Alkylthio,
$(C_1-C_4)$-Alkylthio-$(C_1-C_4)$-alkyl,
$(C_6-C_{12})$-Arylthio-$(C_1-C_4$-alkyl,
das im Arylteil wie oben bei Aryloxy beschrieben, substituiert sein kann,
$(C_6-C_{12})$-Aryl-$(C_1-C_4)$-alkylthio,
das im Arylteil wie oben bei Aryloxy beschrieben substituiert sein kann,
Carboxy-$(C_1-C_4)$-alkyl,
Carboxy, Carbamoyl,
Carbamoyl-$(C_1-C_4)$-alkyl,
$(C_1-C_4)$-Alkoxy-carbonyl-$(C_1-C_4)$-alkyl,
$(C_6-C_{12})$-Aryloxy-$(C_1-C_4)$-alkyl,
das im Arylteil wie oben bei Aryloxy beschrieben substituiert sein kann oder
$(C_6-C_{12})$-Aryl-$(C_1-C_4)$-alkoxy,
das im Arylteil wie oben bei Aryloxy beschrieben substituiert sein kann,
$R^1$ Wasserstoff,
Alkyl mit 1 - 6 C-Atomen,
Alkenyl mit 2 - 6 C-Atomen,
Alkinyl mit 2 - 6 C-Atomen,
Cycloalkyl mit 3 - 9 C-Atomen,
Cycloalkenyl mit 5 - 9 C-Atomen,
$(C_3-C_9)$-Cycloalkyl-$(C_1-C_4)$-alkyl,
$(C_5-C_9)$-Cycloalkenyl-$(C_1-C_4)$-alkyl,
gegebenenfalls teilhydriertes Aryl mit 6 - 12 C-Atomen,
das wie oben bei R beschrieben substituiert sein kann,
$(C_6-C_{12})$-Aryl-$(C_1-C_4)$-alkyl oder $(C_7-C_{13})$-Aroyl-($C_1$ oder $C_2$)alkyl,
die beide wie das vorstehende Aryl substituiert sein können,
mono- bzw. bicyclisches, gegebenenfalls teilhydriertes Heteroaryl mit 5 - 7 bzw. 8 - 10 Ringatomen, wovon 1 bis 2 Ringatome Schwefel- oder Sauerstoffatome und/oder 1 bis 4 Ringatome Stickstoffatome darstellen,
das wie das vorstehende Aryl substituiert sein kann oder
die gegebenenfalls geschützte Seitenkette einer natürlich vorkommenden α-Aminosäure $R^1$-$CH(NH_2)$-$COOH$ bedeutet,
$R^2$
Wasserstoff,
Alkyl mit 1 - 6 C-Atomen,
Alkenyl mit 2 - 6 C-Atomen,
Di-$(C_1-C_4)$-alkylamino-$(C_1-C_4)$-alkyl,
$(C_1-C_5)$-Alkanoyloxy-$(C_1-C_4)$-alkyl,
$(C_1-C_6)$-Alkoxy-carbonyloxy-$(C_1-C_4)$-alkyl,
$(C_7-C_{13})$-Aroyloxy-$(C_1-C_4)$-alkyl,
$(C_6-C_{12})$-Aryloxycarbonyloxy$(C_1-C_4)$-alkyl,
Aryl mit 6 - 12 C-Atomen,
$(C_6-C_{12})$-Aryl-$(C_1-C_4)$-alkyl,
$(C_3-C_9)$-Cycloalkyl oder
$(C_3-C_9)$-Cycloalkyl-$(C_1-C_4)$-alkyl
bedeutet,
$R^3$ einen Rest der Formel

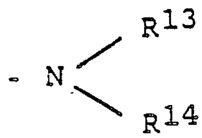

bedeutet,
worin $R^{13}$ und $R^{14}$ gleich oder verschieden unabhängig voneinander
Wasserstoff,
Alkyl mit 1 - 16 C-Atomen,
Alkenyl mit 2 - 16 C-Atomen,
Alkinyl mit 2 - 16 C-Atomen,
Cycloalkyl mit 3 - 9 C-Atomen,
$(C_3-C_9)$-Cycloalkyl-$(C_1-C_4)$-alkyl,
Amino-$(C_1-C_{16})$-alkyl,
Mono-$(C_1-C_4)$-alkylamino-$(C_1-C_{12})$alkyl,
Di-$(C_1-C_4)$-alkylamino-$(C_1-C_8)$-alkyl,
$(C_1-C_5)$-Alkanoyloxy-$(C_1-C_{11})$-alkyl,
$(C_1-C_6)$-Alkoxycarbonyloxy-$(C_1-C_{10})$-alkyl,
$(C_7-C_{13})$-Aroyloxy-$(C_1-C_6)$-alkyl,
$(C_6-C_{12})$-Aryloxycarbonyloxy$(C_1-C_6)$-alkyl,
Aryl mit 6 - 12 C-Atomen oder
$(C_6-C_{12})$-Aryl-$(C_1-C_6)$-alkyl bedeuten
oder zusammen mit dem sie tragenden Stickstoffatom, gegebenenfalls durch Amino, $(C_1-C_4)$-Alkylamino, Di-$(C_1-C_4)$-alkylamino, $(C_1-C_5)$-Alkanoyloxy, $(C_1-C_6)$-Alkoxycarbonyloxy, $(C_7-C_{13})$-Aroyloxy oder $(C_6-C_{12})$-Aryloxycarbonyloxy substituiertes
Pyrrolidinyl,
Piperidinyl,
Piperazinyl,
Homopiperazinyl,
Morpholinyl,
Thiomorpholinyl,
Imidazolyl,
Pyridyl,
Pyrimidinyl,
sym.-Triazinyl bedeuten
und
f) $R^4$ und $R^5$ zusammen mit den sie tragenden Atomen ein mono-, bi- oder tricyclisches heterocyclisches Ringsystem mit 3 bis 15 Ring-C-Atomen bilden
und deren physiologisch verträgliche Salze mit Säuren oder Basen,
ausgenommen Verbindungen der Formel II und deren Salze,
worin

a) n = 1 oder 2 ist;
b) R
1. Wasserstoff;
2. Alkyl mit 1 - 8 C-Atomen;
3. Alkenyl mit 2 - 8 C-Atomen;
4. Cycloalkyl mit 3 - 9 C-Atomen;
5. Aryl mit 6 - 12 C-Atomen,

das durch $(C_1-C_4)$-Alkyl, $(C_1-C_4)$-Alkoxy, Hydroxy, Halogen, Nitro, Amino, Aminomethyl, $(C_1-C_4)$-Alkylamino, Di-$(C_1-C_4)$-alkylamino, $(C_1-C_4)$-Alkanoylamino, Methylendioxy, Carboxy, Cyano und/oder Sulfamoyl mono-, di- oder trisubstituiert sein kann;

6. Alkoxy mit 1 - 4 C-Atomen;
7. Aryloxy mit 6 - 12 C-Atomen,

das wie unter II. b) 5. beschrieben substituiert sein kann;

8. mono- bzw. bicyclisches Heteroaryloxy mit 5 - 7 bzw. 8 - 10 Ringatomen, wovon 1 bis 2 Ringatome Schwefel- oder Sauerstoffatome und/oder 1 bis 4 Ringatome Stickstoff darstellen,

das wie unter II. b) 5. beschrieben substituiert sein kann;

9. Amino-($C_1$-$C_4$)-alkyl;
10. ($C_1$-$C_4$)-Alkanoylamino-($C_1$-$C_4$)-alkyl;
11. ($C_7$-$C_{13}$)-Aroylamino-($C_1$-$C_4$)-alkyl;
12. ($C_1$-$C_4$)-Alkoxy-carbonylamino-($C_1$-$C_4$)-alkyl;
13. ($C_6$-$C_{12}$)-Aryl-($C_1$-$C_4$)-alkoxycarbonylamino-($C_1$-$C_4$)-alkyl;
14. ($C_6$-$C_{12}$)-Aryl-($C_1$-$C_4$)-alkylamino-($C_1$-$C_4$)-alkyl;
15. ($C_1$-$C_4$)-Alkylamino-($C_1$-$C_4$)-alkyl;
16. Di-($C_1$-$C_4$)-alkylamino-($C_1$-$C_4$)-alkyl;
17. Guanidino-($C_1$-$C_4$)-alkyl;
18. Imidazolyl;
19. Indolyl;
20. ($C_1$-$C_4$)-Alkylthio;
21. ($C_1$-$C_4$)-Alkylthio-($C_1$-$C_4$)-alkyl;
22. ($C_6$-$C_{12}$)-Arylthio-($C_1$-$C_4$)-alkyl,
das im Arylteil wie unter II. b) 5. beschrieben substituiert sein kann;
23. ($C_6$-$C_{12}$)-Aryl-($C_1$-$C_4$)-alkylthio,
das im Arylteil wie unter II. b) 5. beschrieben substituiert sein kann;
24. Carboxy-($C_1$-$C_4$)-alkyl;
25. Carboxy;
26. Carbamoyl;
27. Carbamoyl-($C_1$-$C_4$)-alkyl;
28. ($C_1$-$C_4$)-Alkoxy-carbonyl-($C_1$-$C_4$)-alkyl;
29. ($C_6$-$C_{12}$)-Aryloxy-($C_1$-$C_4$)-alkyl,
das im Arylteil wie unter II. b) 5. beschrieben substituiert sein kann; oder
30. ($C_6$-$C_{12}$)-Aryl-($C_1$-$C_4$)-alkoxy,
das im Arylteil wie unter II. b) 5. beschrieben substituiert sein kann, bedeutet;

c) $R^1$
1. Wasserstoff;
2. Alkyl mit 1 - 6 C-Atomen;
3. Alkenyl mit 2 - 6 C-Atomen;
4. Alkinyl mit 2 - 6 C-Atomen;
5. Cycloalkyl mit 3 - 9 C-Atomen;
6. Cycloalkenyl mit 5 - 9 C-Atomen;
7. ($C_3$-$C_9$)-Cycloalkyl-($C_1$-$C_4$)-alkyl;
8. ($C_5$-$C_9$)-Cycloalkenyl-($C_1$-$C_4$)-alkyl;
9. gegebenenfalls teilhydriertes Aryl mit 6 - 12 C-Atomen, das wie unter II. b) 5. beschrieben substituiert sein kann;
10. ($C_6$-$C_{12}$)-Aryl-($C_1$-$C_4$)-alkyl oder ($C_7$-$C_{13}$)-Aroyl-($C_1$ oder $C_2$)-alkyl,
die beide wie im Arylteil wie unter II. b) 5. beschrieben substituiert sein können;
11. mono- bzw. bicyclisches, gegebenenfalls teilhydriertes Heteroaryl mit 5 - 7 bzw. 8 - 10 Ringatomen, wovon 1 bis 2 Ringatome Schwefel- oder Sauerstoffatome und/oder 1 bis 4 Ringatome Stickstoffatome darstellen,
das wie unter II. b) 5. beschrieben substituiert sein kann; oder
12. falls von den vorstehenden Definitionen nicht umfaßt, die gegebenenfalls geschützte Seitenkette einer natürlich vorkommenden α-Aminosäure der Formel $R^1$-CH($NH_2$)-COOH bedeutet;

d) $R^2$
1. Wasserstoff;
2. Alkyl mit 1 - 6 C-Atomen;
3. Alkenyl mit 2 - 6 C-Atomen;
4. Di-($C_1$-$C_4$)-alkylamino-($C_1$-$C_4$)-alkyl;
5. ($C_1$-$C_5$)-Alkanoyloxy-($C_1$-$C_4$)-alkyl;
6. ($C_1$-$C_6$)-Alkoxy-carbonyloxy-($C_1$-$C_4$)-alkyl;
7. ($C_7$-$C_{13}$)-Aroyloxy-($C_1$-$C_4$)-alkyl;
8. ($C_6$-$C_{12}$)-Aryloxycarbonyloxy($C_1$-$C_4$)-alkyl;
9. Aryl mit 6 - 12 C-Atomen;
10. ($C_6$-$C_{12}$)-Aryl-($C_1$-$C_4$)-alkyl;

11. $(C_3-C_9)$-Cycloalkyl; oder
12. $(C_3-C_9)$-Cycloalkyl-$(C_1-C_4)$-alkyl bedeutet,

e) $R^3$ einen der Rest der Formel

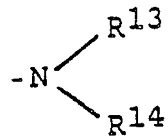

bedeutet, worin
$R^{13}$ und $R^{14}$ gleich oder verschieden unabhängig voneinander
1. Wasserstoff;
2. Alkyl mit 1 - 6 C-Atomen; oder
3. $(C_6-C_{12})$-Aryl-$(C_1-C_4)$-alkyl;
bedeuten und

f) $R^4$ und $R^5$ zusammen mit den sie tragenden Atomen ein mono-, bi- oder tricyclisches heterocyclisches Ringsystem mit 3 bis 15 Ring-C-Atomen bilden.

Die Erfindung betrifft insbesondere Verbindungen der Formel II, in welcher
n = 1 oder 2 ist,
R $(C_1-C_6)$-Alkyl, $(C_2-C_6)$-Alkenyl, $(C_3-C_9)$-Cycloalkyl, Amino-$(C_1-C_4)$-alkyl, $(C_2-C_5)$-Acylamino-$(C_1-C_4)$-alkyl, $(C_7-C_{13})$-Aroylamino-$(C_1-C_4)$-alkyl, $(C_1-C_4)$-Alkoxycarbonylamino-$(C_1-C_4)$-alkyl, $(C_6-C_{12})$-Aryl-$(C_1-C_4)$-alkoxycarbonylamino-$(C_1-C_4)$-alkyl, $(C_6-C_{12})$-Aryl, das durch $(C_1-C_4)$-Alkyl, $(C_1-C_4)$-Alkoxy, Hydroxy, Halogen, Nitro, Amino, $(C_1-C_4)$-Alkylamino, Di-$(C_1-C_4)$-alkylamino oder Methylendioxy monosubstituiert sein kann, oder 3-Indolyl, insbesondere Methyl, Ethyl, Cyclohexyl, tert. Butoxycarbonylamino-$(C_1-C_4)$-alkyl, Benzoyloxycarbonylamino-$(C_1-C_4)$-alkyl oder Phenyl, das durch Phenyl, $(C_1-C_2)$-Alkyl, ($C_1$ oder $C_2$)-Alkoxy, Hydroxy, Fluor, Chlor, Brom, Amino, $(C_1-C_4)$-Alkylamino, Di-$(C_1-C_4)$alkylamino oder Nitro monosubstituiert sein kann, bedeutet,
$R^1$ Wasserstoff oder $(C_1-C_6)$-Alkyl, das gegebenenfalls durch Amino, $(C_1-C_6)$-Acylamino oder Benzoylamino substituiert sein kann, $(C_2-C_6)$-Alkenyl, $(C_3-C_9)$-Cycloalkyl, $(C_5-C_9)$-Cycloalkenyl, $(C_3-C_7)$-Cycloalkyl-$(C_1-C_4)$-alkyl, $(C_6-C_{12})$-Aryl oder teilhydriertes Aryl, das jeweils durch $(C_1-C_4)$-Alkyl, ($C_1$ oder $C_2$)-Alkoxy oder Halogen substituiert sein kann, $(C_6-C_{12})$-Aryl-($C_1$ bis $C_4$)-alkyl oder $(C_7-C_{13})$-Aroyl-$(C_1-C_2)$-alkyl, die beide wie vorstehend definiert im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder 1 bis 4 Ringatome Stickstoffatome darstellen, oder eine Seitenkette einer natürlich vorkommenden, gegebenenfalls geschützten α-Aminosäure, insbesondere aber Wasserstoff, $(C_1-C_3)$-Alkyl, ($C_2$ oder $C_3$)-Alkenyl, die gegebenenfalls geschützte Seitenkette von Lysin, Benzyl, 4-Methoxybenzyl, 4-Ethoxybenzyl, Phenethyl, 4-Amino-butyl oder Benzoylmethyl bedeutet,
$R^2$ Wasserstoff,
$(C_1-C_6)$-Alkyl, $(C_2-C_6)$-Alkenyl oder $(C_6-C_{12})$-Aryl-$(C_1-C_4)$-alkyl, insbesondere aber Wasserstoff, $(C_1-C_4)$-Alkyl oder Benzyl bedeutet,
$R^3$ einen Rest der Formel

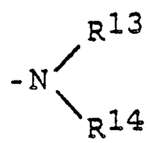

bedeutet,
worin $R^{13}$ und $R^{14}$ gleich oder verschieden unabhängig voneinander Wasserstoff, $(C_4-C_{12})$-Alkyl, $(C_4-C_{12})$-Alkenyl, $(C_5-C_7)$-Cycloalkyl, $(C_5-C_7)$-Cycloalkyl-$(C_1-C_4)$-alkyl, Amino-$(C_4-C_{12})$-alkyl, $(C_1-C_4)$-Alkylamino-$(C_4-C_{12})$-alkyl, Di-$(C_1-C_4)$-Alkylamino-$(C_1-C_8)$alkyl, $(C_6-C_{12})$-Aryl-$(C_1-C_6)$-alkyl, bedeuten oder zusammen mit dem sie tragenden Stickstoffatom gegebenenfalls durch Amino, $(C_1-C_4)$-Alkylamino oder Di-$(C_1-C_4)$-alkylamino substituiertes Pyrrolidinyl, Piperidinyl, Piperazinyl, Homopiperazinyl, Morpholinyl oder Thiomorpholinyl bedeuten,
und

$R^4$ und $R^5$ zusammen mit den sie tragenden Atomen ein mono-, bi- oder tricylisches Ringsystem mit 3 bis 15 Ring-C-Atomen bilden

und deren physiologisch verträgliche Salze mit Säuren und Basen,

ausgenommen Verbindungen der Formel II und deren Salze, worin

a) n = 1 oder 2 ist;

b) R

1. Wasserstoff;
2. Alkyl mit 1 - 8 C-Atomen;
3. Alkenyl mit 2 - 8 C-Atomen;
4. Cycloalkyl mit 3 - 9 C-Atomen;
5. Aryl mit 6 - 12 C-Atomen,

das durch ($C_1$-$C_4$)-Alkyl, ($C_1$-$C_4$)-Alkoxy, Hydroxy, Halogen, Nitro, Amino, Aminomethyl, ($C_1$-$C_4$)-Alkylamino, Di-($C_1$-$C_4$)-alkylamino, ($C_1$-$C_4$)-Alkanoylamino, Methylendioxy, Carboxy, Cyano und/oder Sulfamoyl mono-, di- oder trisubstituiert sein kann;

6. Alkoxy mit 1 - 4 C-Atomen;
7. Aryloxy mit 6 - 12 C-Atomen,

das wie unter II. b) 5. beschrieben substituiert sein kann;

8. mono- bzw. bicyclisches Heteroaryloxy mit 5 - 7 bzw. 8 - 10 Ringatomen, wovon 1 bis 2 Ringatome Schwefel- oder Sauerstoffatome und/oder 1 bis 4 Ringatome Stickstoff darstellen,

das wie unter II. b) 5. beschrieben substituiert sein kann;

9. Amino-($C_1$-$C_4$)-alkyl;
10. ($C_1$-$C_4$)-Alkanoylamino-($C_1$-$C_4$)-alkyl;
11. ($C_7$-$C_{13}$)-Aroylamino-($C_1$-$C_4$)-alkyl;
12. ($C_1$-$C_4$)-Alkoxy-carbonylamino-($C_1$-$C_4$)-alkyl;
13. ($C_6$-$C_{12}$)-Aryl-($C_1$-$C_4$)-alkoxycarbonylamino-($C_1$-$C_4$)-alkyl;
14. ($C_6$-$C_{12}$)-Aryl-($C_1$-$C_4$)-alkylamino-($C_1$-$C_4$)-alkyl;
15. ($C_1$-$C_4$)-Alkylamino-($C_1$-$C_4$)-alkyl;
16. Di-($C_1$-$C_4$)-alkylamino-($C_1$-$C_4$)-alkyl;
17. Guanidino-($C_1$-$C_4$)-alkyl;
18. Imidazolyl;
19. Indolyl;
20. ($C_1$-$C_4$)-Alkylthio;
21. ($C_1$-$C_4$)-Alkylthio-($C_1$-$C_4$)-alkyl;
22. ($C_6$-$C_{12}$)-Arylthio-($C_1$-$C_4$)-alkyl,

das im Arylteil wie unter II. b) 5. beschrieben substituiert sein kann;

23. ($C_6$-$C_{12}$)-Aryl-($C_1$-$C_4$)-alkylthio,

das im Arylteil wie unter II. b) 5. beschrieben substituiert sein kann;

24. Carboxy-($C_1$-$C_4$)-alkyl;
25. Carboxy;
26. Carbamoyl;
27. Carbamoyl-($C_1$-$C_4$)-alkyl;
28. ($C_1$-$C_4$)-Alkoxy-carbonyl-($C_1$-$C_4$)-alkyl;
29. ($C_6$-$C_{12}$)-Aryloxy-($C_1$-$C_4$)-alkyl,

das im Arylteil wie unter II. b) 5. beschrieben substituiert sein kann; oder

30. ($C_6$-$C_{12}$)-Aryl-($C_1$-$C_4$)-alkoxy,

das im Arylteil wie unter II. b) 5. beschrieben substituiert sein kann, bedeutet;

c) $R^1$

1. Wasserstoff;
2. Alkyl mit 1 - 6 C-Atomen;
3. Alkenyl mit 2 - 6 C-Atomen;
4. Alkinyl mit 2 - 6 C-Atomen;
5. Cycloalkyl mit 3 - 9 C-Atomen;
6. Cycloalkenyl mit 5 - 9 C-Atomen;
7. ($C_3$-$C_9$)-Cycloalkyl-($C_1$-$C_4$)-alkyl;
8. ($C_5$-$C_9$)-Cycloalkenyl-($C_1$-$C_4$)-alkyl;
9. gegebenenfalls teilhydriertes Aryl mit 6 - 12 C-Atomen, das wie unter II. b) 5. beschrieben substituiert sein kann;

10. ($C_6$-$C_{12}$)-Aryl-($C_1$-$C_4$)-alkyl oder ($C_7$-$C_{13}$)-Aroyl-($C_1$ oder $C_2$)-alkyl, die beide wie im Arylteil wie unter II. b) 5. beschrieben substituiert sein können;

11. mono- bzw. bicyclisches, gegebenenfalls teilhydriertes Heteroaryl mit 5 - 7 bzw. 8 - 10 Ringatomen, wovon 1 bis 2 Ringatome Schwefel-oder Sauerstoffatome und/oder 1 bis 4 Ringatome Stickstoffatome darstellen, das wie unter II. b) 5. beschrieben substituiert sein kann; oder

12. falls von den vorstehenden Definitionen nicht umfaßt, die gegebenenfalls geschützte Seitenkette natürlich vorkommenden α-Aminosäure der Formel $R^1$-CH($NH_2$)-COOH bedeutet;

d) $R^2$

1. Wasserstoff;
2. Alkyl mit 1 - 6 C-Atomen;
3. Alkenyl mit 2 - 6 C-Atomen;
4. Di-($C_1$-$C_4$)-alkylamino-($C_1$-$C_4$)-alkyl;
5. ($C_1$-$C_5$)-Alkanoyloxy-($C_1$-$C_4$)-alkyl;
6. ($C_1$-$C_6$)-Alkoxy-carbonyloxy-($C_1$-$C_4$)-alkyl;
7. ($C_7$-$C_{13}$)-Aroyloxy-($C_1$-$C_4$)-alkyl;
8. ($C_6$-$C_{12}$)-Aryloxycarbonyloxy($C_1$-$C_4$)-alkyl;
9. Aryl mit 6 - 12 C-Atomen;
10. ($C_6$-$C_{12}$)-Aryl-($C_1$-$C_4$)-alkyl;
11. ($C_3$-$C_9$)-Cycloalkyl; oder
12. ($C_3$-$C_9$)-Cycloalkyl-($C_1$-$C_4$)-alkyl bedeutet,

e) $R^3$ einen Rest der Formel

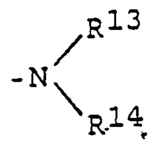

bedeutet, worin $R^{13}$ und $R^{14}$ gleich oder verschieden unabhängig voneinander

1. Wasserstoff,
2. Alkyl mit 1 - 6 C-Atomen oder
3. ($C_6$-$C_{12}$)-Aryl-($C_1$-$C_4$)-alkyl

bedeuten und

f) $R^4$ und $R^5$ zusammen mit den sie tragenden Atomen ein mono-, bi- oder tricyclisches heterocyclisches Ringsystem mit 3 bis 15 Ring-C-Atomen bilden.

Als solche Ringsysteme, die die Reste $R^4$ und $R^5$ in den neuen Verbindungen der Formel II bilden, kommen insbesondere jene aus der folgenden Gruppe in Betracht:

Tetrahydroisochinolin (A); Decahydroisochinolin (B); Octahydroindol (C); Octahydrocyclopenta-[b]pyrrol (D); 2-Azaspiro[4.5]decan (E); 2-Azaspiro[4.4]-nonan (F); Spiro[(bicyclo[2.2.1]heptan)-2,3′-pyrrolidin] (G); Spiro[(bicyclo[2.2.2]octan)-2,3′-pyrrolidin] (H); 2-Azatricyclo[4.3.0.1$^{6,9}$]decan (I); Decahydrocyclohepta-[b]-pyrrol (J); Hexahydrocyclopropa[b]pyrrol (K);

Octahydroisoindol (L); Octahydrocyclopenta[c]pyrrol (M) 2,3,3a,4,5,7a-Hexahydroindol (N); 1,2,3,3a,4,6a-Hexahydrocyclopenta[b]pyrrol (O); Pyrrolidin (P); Indolin (Q); Thiazolidin (R), die alle gegebenenfalls substituiert sein können.

Besonders bevorzugt sind die Ringsysteme A, C, D, H und O in welchen das die CO-$R^3$-Gruppe tragende C-Atom vorzugsweise die S-Konfiguration aufweist.

Die neuen Verbindungen der Formel II weisen asymmetrische Kohlenstoffatome auf und können daher als Enantiomere und Diastereomere auftreten. Die Erfindung umfaßt sowohl die reinen Enantiomeren als auch die Racemate.

Bei den Verbindungen, die mehrere chirale Atome besitzen, kommen alle möglichen Diastereomere als Racemate oder Enantiomere, oder Gemische verschiedener Diastereomere in Betracht. Die Racemate können nach gebräuchlichen Methoden, zum Beispiel durch Salzbildung mit optisch aktiven Säuren wie Camphersulfonsäure oder Di-benzoylweinsäure, fraktionierte Kristallisation und anschließende Freisetzung der Basen aus ihren Salzen, oder durch Derivatisierung mit geeigneten optisch aktiven Reagenzien,

Trennung der diastereomeren Derivate durch fraktionierte Kristallisation oder Chromatographie an Kieselgel oder Aluminiumoxid und Rückspaltung in die Enantiomeren aufgetrennt werden. Die Diastereomeren können nach gebräuchlichen Methoden, wie fraktionierte Kristallisation oder Chromatographie an Säulen, getrennt werden.

Als Salze der Verbindungen der Formeln I und II kommen, je nach saurer oder basischer Natur diese Verbindungen, Alkali-oder Erdalkalisalze oder Salze mit physiologisch verträglichen Aminen oder Salze mit anorganischen oder organischen Säuren wie z.B. HCl, HBr, $H_2SO_4$, Maleinsäure, Fumarsäure, Weinsäure, Citronensäure in Frage.

Die Erfindung betrifft weiterhin ein Verfahren zur Herstellung einer Verbindung der Formel II, das dadurch gekennzeichnet ist, daß man ihre Fragmente in einem geeigneten Lösungsmittel gegebenenfalls in Gegenwart einer Base und/oder eines Kupplungshilfsmittels bei einer Temperatur bis zum Siedepunkt des Reaktionsgemisches miteinander umsetzt, gegebenenfalls intermediär entstehende ungesättigte Verbindungen, wie Schiffsche Basen, reduziert, zum Schutz reaktiver Gruppen temporär eingeführte Schutzgrupppen abspaltet, Verbindungen der Formel II mit freier(en) Carboxylgruppe(n) gegebenenfalls verestert oder in Amide überführt und die erhaltenen Verbindungen gegebenenfalls in ihre physiologisch verträglichen Salze überführt.

Das erfindungsgemäße Verfahren ist beispielsweise dadurch gekennzeichnet, daß man

a) eine Verbindung der Formel III

$$HOOC - \underset{R^4}{\overset{*}{CH}} - \underset{R^5}{N} - \underset{\overset{\|}{O}}{C} - \underset{R^1}{\overset{*}{CH}} - NH - \underset{COOR^2}{\overset{*}{CH}} - (CH_2)_n - R \qquad (III)$$

worin R, $R^1$, $R^2$, $R^4$, $R^5$ und n die gleiche Bedeutung wie in Formel II haben, mit einem Amin der Formel IV

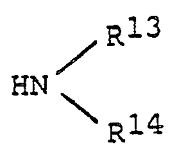

(IV)

worin $R^{13}$ und $R^{14}$ die gleiche Bedeutung wie in Formel II haben, umsetzt, beispielsweise in Analogie zu bekannten Peptidkupplungsverfahren in einem organischen Lösungsmittel wie DMF, $CH_2Cl_2$, DMA in Gegenwart von Kupplungshilfsmitteln, wie Carbodiimiden (z.B. Dicyclohexylcarbodiimid), Diphenylphosphorylazid, Alkanphosphonsäureanhydriden, Dialkylphosphinsäureanhydriden oder N,N-Succinimidoylcarbonat in einem Lösungsmittel wie $CH_3CN$, wobei Aminogruppen in Verbindungen der Formel IV mit Tetraethyldiphosphit aktiviert werden können und Verbindungen der Formel III in Aktivester (z.B. mit 1-Hydroxybenzotriazol), gemischte Anhydride (z.B. mit Chlorameisensäureestern), Azide oder Carbodiimid-Derivate überführt und damit aktiviert werden können (vgl. Schröder, Lübke, The Peptides, Band 1, New York 1965, Seiten 76 - 136) und die Reaktion vorzugsweise zwischen -20°C und dem Siedepunkt des Reaktionsgemisches durchgeführt wird,

oder daß man

b) eine Verbindung der Formel V

$$R^3 - \underset{\overset{\|}{O}}{C} - \underset{R^4}{\overset{*}{CH}} - \underset{R^5}{NH} \qquad (V)$$

worin $R^3$, $R^4$ und $R^5$ die gleiche Bedeutung wie in Formel II haben, mit einer Verbindung der Formel VI

$$HOOC - \underset{R^1}{\overset{*}{CH}} - NH - \underset{COOR^2}{\overset{*}{CH}} - (CH_2)_n - R \qquad (VI)$$

worin R, $R^1$, $R^2$ und n die gleiche Bedeutung wie in Formel II haben, nach einem Kupplungsverfahren, wie unter der Verfahrensvariante a) beschrieben, umsetzt, oder daß man

c) eine Verbindung der Formel VII

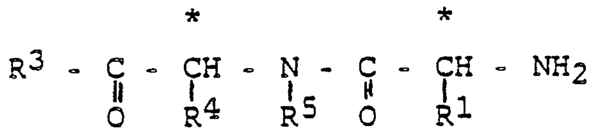 (VII)

worin $R^1$, $R^3$, $R^4$ und $R^5$ die gleiche Bedeutung wie in Formel II haben, mit einer Verbindung der Formel VIII

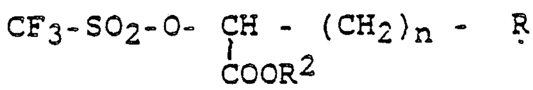 (VIII)

worin R, $R^2$ und n die gleiche Bedeutung wie in Formel II haben, z.B. analog der in US-Patent 4525301 beschriebenen Verfahrensweise umsetzt,
oder daß man

d) eine Verbindung der Formel IX,

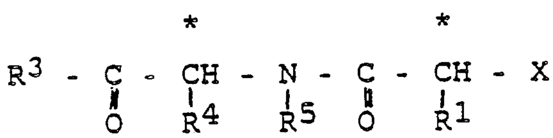 (IX)

worin $R^1$, $R^3$, $R^4$ und $R^5$ die gleiche Bedeutung wie in Formel II haben und
in der X eine Abgangsgruppe, die nucleophil verdrängt werden kann, insbesondere ein Cl, Br, J-Atom oder einen Sulfonsäurerest, bedeutet,
mit einer Verbindung der Formel X

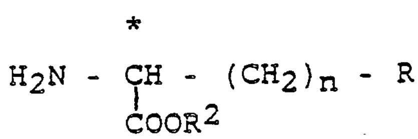 (X)

in der R, $R^2$ und n die gleiche Bedeutung wie in Formel II haben, unter Bedingungen einer nucleophilen Substitution, vorzugsweise in einem polaren organischen Lösungsmittel, wie einem Alkohol, vorzugsweise Methanol, Ethanol, Propanol oder Isopropanol oder einem niederen Keton, vorzugsweise Aceton, Methylethylketon oder Methylisobutylketon oder in Acetonitril, Dimethylformamid, Dimethylsulfoxid oder Sulfolan oder einem Kohlenwasserstoff, vorzugsweise Toluol, mit oder ohne Gegenwart einer Hilfsbase zum Abfangen der sich bildenden Säure, vorzugsweise in Gegenwart von Kaliumhydrogencarbonat, Kaliumcarbonat, Natriumhydrogencarbonat, Natriumcarbonat, Triethylamin, Pyridin, 1,5-Diazabicyclo[5,4,0]undec-5-en oder 1,5-Diazabicyclo[4,3,0]non-5-en, sowie mit oder ohne Gegenwart eines Alkalihalogenids, vorzugsweise Natriumjodid oder Kaliumjodid, bei einer Temperatur zwischen -50 und +100° C, vorzugsweise zwischen -20 und +60° C, umsetzt, oder daß man

e) eine Verbindung der Formel XI

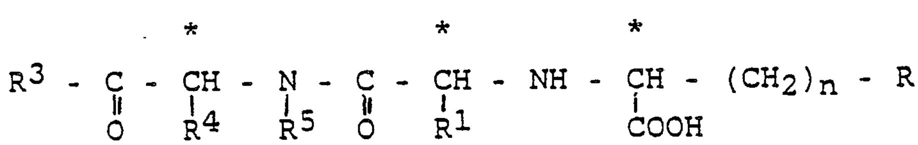 (XI)

in der R, $R^1$, $R^3$, $R^4$, $R^5$ und n die gleiche Bedeutung wie in Formel II haben, unter Anwendung einer der gebräuchlichen Veresterungsmethoden (siehe z.B. Buchler, Pearson, Survey of Organic Syntheses, Vol. 1,

New York 1970, S. 802 - 825; Houben-Weyl, Methoden der Organischen Chemie, Band E5, 1985, S. 656 - 773) in Verbindungen der Formel II überführt beispielsweise durch Umsetzung mit einem Alkohol $R^2$-OH unter saurer Katalyse oder nach Aktivierung der Carbonsäurefunktion von XI oder nach Aktivierung der Hydroxyfunktion von $R^2OH$, z.B. unter den Bedingungen einer Mitsunobu-Reaktion,
oder durch Umsetzung mit einer Verbindung der Formel $R^2$ X, worin $R^2$ die gleiche Bedeutung wie in Formel II und X die gleiche Bedeutung wie in Formel IX hat unter den Bedingungen einer nucleophilen Substitution, wie sie unter der Verfahrensvariante d) beschrieben sind, oder durch Umsetzung mit einem Diazoalkan in einem inerten organischen Lösungsmittel,
oder daß man

f) eine Verbindung der Formel XII

$R^2OOC\text{-}CH=CH\text{-}CO\text{-}R$ (XII),

in welcher R und $R^2$ die gleiche Bedeutung wie in Formel II haben, mit einer Verbindung der Formel VII, in bekannter Weise in einer Michael-Reaktion (Organikum, 6. Auflage, S. 492, 1967) umsetzt, die Carbonylgruppe z.B. in sauer alkoholischer Lösung mit einem Edelmetallkatalysator, insbesondere Palladium oder Platin auf Aktivkohle bei einem Druck von 20 bis 120 bar hydriert,
oder daß man

g) eine Verbindung der Formel VII mit einer Verbindung der Formel XIII

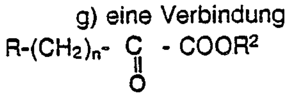 (XIII)

worin R, $R^2$ und n die gleiche Bedeutung wie in Formel II haben, beispielsweise gemäß der in J. Amer. Chem. Soc. 93, 2897 (1971) beschriebenen Verfahrensweise umsetzt und die erhaltenen Schiff-Basen reduziert, vorzugsweise unter Verwendung eines komplexen Hydrides, z.B. Natriumcyanborhydrid,
und die so erhaltenen Verbindungen der Formel II gegebenenfalls in ihre physiologisch verträglichen Salze überführt.

Verbindungen der Formel III sind bekannt (siehe z.B. EP-A 79022, EP-A 105102, EP-A 113880, EP-A 116270, EP-A 84164, EP-A 90362).

Verbindungen der Formel IV sind bekannt und größtenteils kommerziell erhältlich.

Verbindungen der Formel V erhält man beispielsweise aus den bekannten Verbindungen der Formel XIV

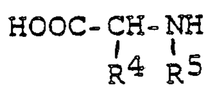 (XIV),

worin $R^4$ und $R^5$ die gleiche Bedeutung wie in Formel II besitzen, durch Schutz der Aminogruppe, Umsetzung mit einen Amin der Formel IV unter Bedingungen, wie sie unter Verfahrensvariante a) beschrieben sind, und anschließende Abspaltung der Amino-Schutzgruppe.

Verbindungen der Formel VI sind als Zwischenprodukte zur Herstellung von ACE-Hemmern bekannt bzw. können auf analogem Wege hergestellt werden.

Verbindungen der Formel VII erhält man beispielsweise aus Verbindungen der Formel XV,

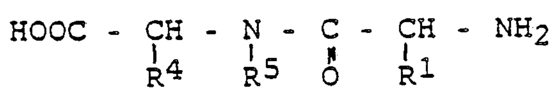 (XV)

worin $R^1$, $R^4$ und $R^5$ die gleiche Bedeutung wie in Formel II besitzen, durch Schutz der Aminogruppe, Umsetzung mit einem Amin der Formel IV unter Bedingungen wie sie unter der Verfahrensvariante a) beschrieben sind, und anschließende Abspaltung der Amino-Schutzgruppe
oder
aus bekannten Verbindungen der Formel XVI

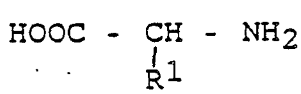 (XVI)

worin $R^1$ die gleiche Bedeutung wie in Formel II besitzt, durch Schutz der Aminogruppe, Umsetzung mit

einer Verbindung der Formel V unter Bedingungen, wie sie unter der Verfahrensvariante a) beschrieben sind, und anschließende Abspaltung der Amino-Schutzgruppe.

Verbindungen der Formel VIII sind bekannt oder auf analogem Wege aus den entsprechenden Ausgangsmaterialien erhältlich.

Verbindungen der Formel IX können beispielsweise erhalten werden durch Umsetzung von Verbindungen der Formel V mit bekannten Verbindungen der Formel XVII,

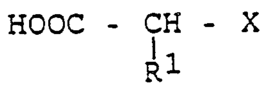 (XVII)

worin $R^1$ die gleiche Bedeutung wie in Formel II und X die gleiche Bedeutung wie in Formel IX besitzt unter Bedingungen, wie sie unter der Verfahrensvariante a) beschrieben sind,
oder aus Verbindungen der Formel XVIII

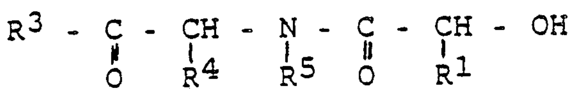 (XVIII)

worin $R^1$, $R^3$, $R^4$ und $R^5$ die gleiche Bedeutung wie in Formel II besitzen, durch Überführung der Hydroxygruppe in die Abgangsgruppe X nach gängigen Methoden.

Verbindungen der Formel X sind bekannt bzw. auf analogem Wege aus den entsprechenden Ausgangsmaterialien erhältlich.

Verbindungen der Formel XI lassen sich beispielsweise herstellen aus Verbindungen der Formel II, in denen $R^2$ eine leicht entfernbare Estergruppe darstellt, die ohne Beeinflussung des übrigen Molekülrestes selektiv abspaltbar ist wie z.B. die Benzylgruppe, die durch Hydrogenolyse entfernt werden kann.

Verbindungen der Formeln XII und XIII sind bekannt.

Verbindungen der Formel XV sind Dipeptide, die aus den einzelnen Aminosäurekomponenten nach an sich bekannten Methoden der Peptidchemie (siehe z.B. Houben-Weyl, Methoden der Organischen Chemie, Bd. XV, Teil II, S. 1 - 364) aufgebaut werden können.

Verbindungen der Formel XVIII sind beispielsweise erhältlich durch Umsetzung von Verbindungen der Formel V mit bekannten Hydroxycarbonsäuren der Formel XIX

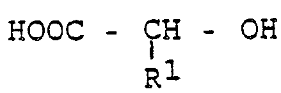 (XIX)

worin $R^1$ die gleiche Bedeutung wie in Formel II besitzt und denen die Hydroxyfunktion gegebenenfalls geschützt ist unter Bedingungen, wie sie unter der Verfahrensvariante a) beschrieben sind, gegebenenfalls gefolgt von einer Abspaltung der Hydroxy-Schutzgruppe.

Die Erfindung betrifft ferner neue Zwischenprodukte der Formel V

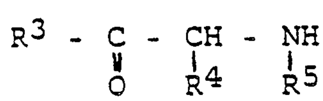 (V)

worin
$R^3$ einen Rest der Formel

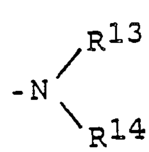

bedeutet,

worin $R^{13}$ und $R^{14}$ gleich oder verschieden unabhängig voneinander
Wasserstoff
Alkyl mit 7 - 16 C-Atomen,
Alkenyl mit 7 - 16 C-Atomen,
Alkinyl mit 7 - 16 C-Atomen
Cycloalkyl mit 3 - 9 C-Atomen,
$(C_3-C_9)$-Cycloalkyl-$(C_1-C_4)$-alkyl,
Amino-$(C_1-C_{16})$-alkyl,
Mono-$(C_1-C_4)$-alkylamino-$(C_1-C_{12})$alkyl,
Di-$(C_1-C_4)$-alkylamino-$(C_1-C_8)$alkyl,
$(C_1-C_5)$-Alkanoyloxy-$(C_1-C_{11})$-alkyl,
$(C_1-C_6)$-Alkoxycarbonyloxy-$(C_1-C_{10})$-alkyl,
$(C_7-C_{13})$-Aroyloxy-$(C_1-C_6)$-alkyl,
$(C_6-C_{12})$-Aryloxycarbonyloxy-$(C_1-C_6)$-alkyl,
Aryl mit 6 - 12 C-Atomen oder
$(C_6-C_{12})$-Aryl-$(C_5-C_6)$-alkyl bedeuten
oder zusammen mit dem sie tragenden Stickstoffatom, gegebenenfalls durch Amino, $(C_1-C_4)$-Alkylamino, Di-$(C_1-C_4)$-alkylamino, $(C_1-C_5)$-Alkanoyloxy, $(C_1-C_6)$-Alkoxycarbonyloxy, $(C_7-C_{13})$-Aroyloxy oder $(C_6-C_{12})$-Aryloxycarbonyloxy substituiertes
Pyrrolidinyl,
Piperidinyl,
Piperazinyl,
Homopiperazinyl,
Morpholinyl,
Thiomorpholinyl,
Imidazolyl,
Pyridyl,
Pyrimidinyl,
sym.-Triazinyl bedeuten
und
$R^4$ und $R^5$ zusammen mit den sie tragenden Atomen ein mono-, bi- oder tricyclisches heterocyclisches Ringsystem mit 3 bis 15 Ring-C-Atomen bilden.

Die Erfindung betrifft ferner ein Verfahren zur Herstellung der neuen Zwischenprodukte V, dadurch gekennzeichnet, daß man in Verbindungen der Formel XIV, worin $R^4$ und $R^5$ die gleiche Bedeutung wie in Formel V besitzen, zunächst die Aminogruppe schützt, dann mit einem Amin der Formel IV unter Bedingungen, wie sie unter der Verfahrensvariante a) beschrieben sind, umsetzt, und anschließend die Amino-Schutzgruppe wieder abspaltet.

Die Verbindungen der Formeln I und II sind, soweit sie bekannt sind, Inhibitoren des Angiotensin-Converting-Enzyms (ACE-Hemmer).

Die Verbindungen der Formeln I und II und deren Salze besitzen lang andauernde, intensive blutdrucksenkende Wirkung. Sie können zur Bekämpfung des Bluthochdrucks verschiedener Genese eingesetzt werden. Auch ihre Kombination mit anderen blutdrucksenkenden, gefäßerweiternden oder diuretisch wirksamen Verbindungen ist möglich. Typische Vertreter dieser Wirkklassen sind z.B. in Erhardt-Ruschig, Arzneimittel, 2. Auflage, Weinheim, 1972, beschrieben. Die Anwendung kann intravenös, subcutan oder peroral erfolgen.

Die Dosierung bei oraler Gabe liegt bei 1-100 mg, vorzugsweise bei 1-40 mg je Einzeldosis bei einem normalgewichtigen erwachsenen Patienten: dies entspricht etwa 15-1300 µg/kg/Tag, vorzugsweise 15-500 µg/kg/Tag. Sie kann in schweren Fällen auch erhöht werden, da toxische Eigenschaften bisher nicht beobachtet wurden. Auch eine Herabsetzung der Dosis ist möglich und vor allem dann angebracht, wenn gleichzeitig Diuretika verabreicht werden.

Überraschend wurde nun gefunden, daß die Verbindungen der Formeln I und II auch eine psychotrope Wirkung aufweisen, insbesondere eine nootrope Wirkung und eine anxiolytische Wirkung.

Die nootrope Wirkung der erfindungsgemäßen Verbindungen wurde an Mäusen, die ein Körpergewicht von 20-25 g besaßen, im inhibitory (passive) avoidance test (step-through-Modell) geprüft. Eine modifizierte Form der von J. KOPP, Z. BODANECKY und M.E. JARVIK beschriebenen Testmethode wurde von J. BURES, O. BURESOVA und J. HUSTON in "Techniques and Basic Experiments for the Study of Brain and Behavior", Elsevier Scientific Publishers, Amsterdam (1983) beschrieben.

Entsprechend diesen Literaturangaben wird eine Substanz dann als nootrop wirksam bezeichnet, wenn

sie bei den Versuchstieren die mittels eines elektroconvulsiven Schocks erzeugte Amnesie oder die mittels Scopolamin induzierte Amnesie aufzuheben vermag.

Die Versuche wurden nach modifizierten Testmethoden durchgeführt. Als Vergleichsverbindung diente das bekannte Nootropikum 2-Oxo-1-pyrrolidinylessigsäureamid (Piracetam). Die deutliche Überlegenheit der erfindungsgemäßen Verbindungen über die Vergleichssubstanz zeigte sich darin, daß die Scopolamininduzierte Amnesie im inhibitory avoidance-Test sich mit einer MED (minimal effective Dosis) von 0,03 - 30 mg/kg p.o. aufheben läßt. Die Vergleichssubstanz hat eine ME von ca 500-1000 mg/kg p.o.

Die erfindungsgemäßen Verbindungen der Formeln I und II sind aufgrund ihrer pharmakologischen Eigenschaften neben der Behandlung des Bluthochdruckes auch für die Behandlung cognitiver Dysfunktionen unterschiedlicher Genese, wie sie z.B. bei der Alzheimer'schen Krankheit oder der senilen Demenz auftreten, geeignet.

Die Erfindung betrifft daher weiter die Anwendung der erfindungsgemäßen Verbindungen bei der Behandlung von Störungen des zentralen Nervensystems, speziell bei der Behandlung und Propylaxe cognitiver Dysfunktionen.

Die Erfindung umfaßt weiterhin die genannten Verbindungen enthaltende Arzneimittel, Verfahren zu deren Herstellung sowie die Verwendung der erfindungsgemäßen Verbindungen bei der Herstellung von Arzneimitteln, die zur Behandlung und Prophylaxe der vorstehend genannten Krankheiten eingesetzt werden.

In Ausübung der erfindungsgemäßen Methode können die oben beschriebenen Verbindungen der Formeln I und II an Säugern wie Affen, Hunden, Katzen, Ratten, Menschen etc. angewendet werden.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoff) entweder als solche oder vorzugsweise in Kombination mit geeigneten pharmazeutischen Hilfsstoffen in Form von Tabletten, Dragees, Kapseln, Suppositorien, Emulsionen, Suspensionen oder Lösungen eingesetzt, wobei der Wirkstoffgehalt bis etwa 95 %, vorteilhafterweise zwischen 10 und 75 %, beträgt.

Welche Hilfsstoffe für die gewünschte Arzneimittelformulierung geeignet sind, ist dem Fachmann aufgrund seines Fachwissens geläufig. Neben Lösungsmitteln, Gelbildnern, Suppositoriengrundlagen, Tabletten-Hilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Die Wirkstoffe können beispielsweise oral, rektal oder parenteral (z.B. intravenös oder subcutan) appliziert werden, wobei die orale Applikation bevorzugt ist.

Für eine orale Anwendungsform werden die aktiven Verbindungen mit den dafür geeigneten Zusatzstoffen wie Trägerstoffen, Stabilisatoren oder inerten Verdünnungsmitteln vermischt und durch die üblichen Methoden in geeignete Darreichungsform gebracht wie Tabletten, Dragees, Steckkapseln, wässrige, alkoholische oder ölige Suspensionen oder wässrige, alkoholische oder ölige Lösungen. Als inerte Träger können z.B. Gummi arabicum, Magnesia, Magnesiumcarbonat, Milchzucker, Glucose oder Stärke, insbesondere Maisstärke verwendet werden. Dabei kann die Zubereitung sowohl als Trocken- als auch als Feuchtgranulat erfolgen. Als ölige Trägerstoffe oder Lösemittel kommen beispielsweise pflanzliche oder tierische Öle in Betracht wie Sonnenblumenöl oder Lebertran.

Zur subcutanen oder intravenösen Applikation werden die aktiven Verbindungen der deren physiologisch verträglichen Salze, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z.B. in Frage Wasser, physiologische Kochsalzlösung oder Alkohole, z.B. Ethanol, Propanol, Glycerin, daneben auch Zuckerlösungen wie Glucose-oder Mannitlösungen oder auch eine Mischung aus den verschiedenen genannten Lösungsmitteln.

Die nachfolgenden Beispiele sollen die erfindungsgemäßen Verbindungen und Verfahren erläutern, ohne die Erfindung auf die hier stellvertretend genannten Substanzen zu beschränken.

**Beispiel 1**

2-[N-(1S-Ethoxycarbonyl-3-phenylpropyl)-S-alanyl]-(1S,3S,5S)-2-azabicyclo[3.3.0]octan-3-carbonsäure-octylamid

1a) 2-(Tert.butyloxycarbonyl)-(1S,3S,5S)-2-azabicyclo[3.3.0]octan-3-carbonsäure-octylamid

Zu einer Lösung von 1,28 g (5 mmol) 2-(tert.Butyloxycarbonyl)-(1S,3S,5S)-2-azabicyclo[3.3.0]octan-3-carbonsäure in 50 ml absolutem Dimethylformamid werden nacheinander 645 mg (5 mmol) 1-Octylamin und 675 mg (5 mmol) 1-Hydroxybenzotriazol gegeben, auf 0°C abgekühlt und anschließend 1,03 g (5 mmol) Dicyclohexylcarbodiimid in drei Portionen zugegeben. Nach 60 Stunden Rühren bei Raumtemperatur wird vom Niederschlag abgesaugt, eingeengt, der Rückstand in Essigester aufgenommen, filtriert, nacheinander zweimal mit 10 %iger Zitronensäure, zweimal mit gesättigter Natriumbicarbonatlösung, Wasser und gesättigter Natriumchloridlösung gewaschen, über Magnesiumsulfat getrocknet und eingedampft. Der Rückstand wird in 15 ml Ether gelöst, über Nacht in der Tiefkühltruhe aufbewahrt, noch einmal vom ausgefallenen Niederschlag abgesaugt und erneut eingeengt,
Ausbeute: 1,34 g (73 %) öliges Produkt.

1b) (1S,3S,5S)-2-Azabicyclo[3.3.0]octan-3-carbonsäureoctylamid

1,3 g (3,55 mmol) Verbindung aus 1a) werden bei 0°C mit 3,5 ml Trifluoressigsäure übergossen und 2,5 Stunden bei Raumtemperatur gerührt. Überschüssige Säure wird in Vakuum abgezogen, der Rückstand in Wasser gelöst, mit gesättigter Natriumbicarbonatlösung alkalisch gestellt und zweimal mit Essigester extrahiert. Die vereinigten organischen Phasen werden mit Wasser und gesättigter Natriumchloridlösung gewaschen, über Magnesiumsulfat getrocknet und eingeengt. Ausbeute: 0,85 g (90 %) öliges Produkt.

1c) 2-[N-(1S-Ethoxycarbonyl-3-phenylpropyl)-S-alanyl]-(1S,3S,5S)-2-azabicyclo[3.3.0]octan-3-carbonsäure-octylamid

0,83 g (3,1 mmol) Verbindung aus 1b), 0,865 g (3,1 mmol) N-(1S-Ethoxycarbonyl-3-phenylpropyl)-S-alanin und 0,313 g (15,5 mmol) Triethylamin werden in 12 ml absolutem Dimethylformamid gelöst und bei -5°C 3,1 ml einer 50 %igen Lösung von Propanphosphonsäureanhydrid in Methylenchlorid zugetropft, eine Stunde bei -5°C und eine weitere Stunde bei Raumtemperatur nachgerührt. Das Reaktionsgemisch wird zwischen Wasser und Essigester verteilt, die wäßrige Phase noch einmal mit Essigester extrahiert und die vereinigten organischen Phasen mit 10 %iger Zitronensäurelösung, gesättigter Natriumbicarbonatlösung und gesättigter Natriumchloridlösung gewaschen, über Magnesiumsulfat getrocknet und eingeengt. Das Rohprodukt wird durch Säulenchromatographie an 80 g Kieselgel (Laufmittel Cyclohexan/Essigester 8:2 und 1:1) gereinigt. Ausbeute: 1,14 g (70 %) farbloses Öl.
$[\alpha]_D^{25}$ = -27,2° (c = 1, Methanol).

**Beispiel 2**

2-[N-(1S-Ethoxycarbonyl-3-phenylpropyl)-S-alanyl]-(1S,3S,5S)-2-azabicyclo[3.3.0]octan-3-carbonsäure-octylamid

Zu einer Lösung von 416 mg (1 mmol) 2-[N-(1S-Ethoxycarbonyl-3-phenylpropyl)-S-alanyl]-(1S,3S,5S)-2-azabicyclo[3.3.0]octan-3-carbonsäure (Ramipril) in 10 ml absolutem Dimethylformamid werden nacheinander 129 mg (1 mmol) 1-Octylamin, 135 mg (1 mmol) 1-Hydroxybenzotriazol und bei 0°C in drei Portionen 206 mg (1 mmol) Dicyclohexylcarbodiimid gegeben. Nach 48 Stunden bei Raumtemperatur wird abgesaugt, mit Wasser verdünnt und zweimal mit Essigester extrahiert. Die vereinigten organischen Phasen werden mit 10 %iger Zitronensäurelösung, gesättigter Natriumbicarbonatlösung und gesättigter Natriumchloridlösung gewaschen, über Natriumsulfat getrocknet und eingeengt. Der Rückstand wird mit Diisopropylether verrieben, vom Niederschlag abgesaugt und das Filtrat eingeengt und durch Säulenchromatographie an 25 g Kieselgel (Laufmittel Cyclohexan/Essigester 7:3) 100 mg öliges Produkt erhalten, das in jeder Hinsicht mit dem in Beispiel 1 beschriebenen Produkt identisch ist.

Die nachfolgenden Beispiele geben die Anwendungsformen zur Propylaxe und Behandlung cognitiver Dysfunktionen nach der erfindungsgemäßen Methode an. Die erfindungsgemäßen Verbindungen können analog den Beispielen in die entsprechenden Anwendungsformen gebracht werden.

**Beispiel 3**

Herstellung des erfindungsgemäß verwendeten Mittels zur oralen Anwendung in der Behandlung von Störungen des zentralen Nervensystems.

1000 Tabletten, die je 10 mg 2-[N-(1-S-Ethoxycarbonyl-3-phenyl-propyl)-S-alanyl]-1S,3S,5S-2-azabicyclo[3.3.0]octan-3-carbonsäure-octylamid enthalten, werden mit den folgenden Hilfsmitteln hergestellt:

| | |
|---|---|
| 2-[N-(1-S-Ethoxycarbonyl-3-phenyl-propyl)-S-alanyl]-(1S,3S,5S)-2-azabicyclo[3.3.0]octan-3-carbonsäure-octylamid | 10 g |
| Maisstärke | 140 g |
| Gelatine | 7,5 g |
| Mikrokristalline Cellulose | 2,5 g |
| Magnesiumstearat | 2,5 g |

2-[N-(1-S-Ethoxycarbonyl-3-phenyl-propyl)-S-alanyl]-(1S,3S,5S)-2-azabicyclo[3.3.0]octan-3-carbonsäure-octylamid und Maisstärke werden mit einer wäßrigen Gelatine-Lösung gemischt. Die Mischung wird getrocknet und zu einem Granulat vermahlen. Mikrokristalline Cellulose und Magnesiumstearat werden mit dem Granulat vermischt. Das entstandene Granulat wird zu 1000 Tabletten gepreßt, wobei jede Tablette 10 mg der Wirksubstanz enthält.

**Beispiel 4**

Gelatine-Kapseln, die je 10 mg 1′-[N-(1-S-Ethoxycarbonyl-3-phenylpropyl)-S-alanyl]-(3′S,5′S)-spirobicyclo[2.2.2]octan-2,3′-pyrrolidin-5′-carbonsäure-octylamid enthalten, werden mit der folgenden Mischung gefüllt:

| | |
|---|---|
| 1'-[N-(1-S-Ethoxycarbonyl-3-phenylpropyl)-S-alanyl]-(3'S,5'S)-spirobicyclo[2.2.2]octan-2,3'-pyrrolidin-5'-carbonsäure-octylamid | 10 mg |
| Magnesiumstearat | 1mg |
| Lactose | 214 mg |

Diese Kapseln können zur Behandlung von Störungen des zentralen Nervensystems verwendet werden. Unter Verwendung entsprechender Ausgangsmaterialien wurden die folgenden Verbindungen auf analogem Wege, wie in Beispiel 1 und 2 dargelegt, hergestellt:

**Beispiel 5**

1′-[N-(1-S-Ethoxycarbonyl-3-phenylpropyl)-S-alanyl]-(3′S,5′S)-spirobicyclo[2.2.2]octan-2,3′-pyrrolidin-5′carbonsäure-n-hexylamid $[\alpha]_D^{20}$ = -39,3° (c = 1, Methanol)

**Beispiel 6**

1′-[N-(1-S-Ethoxycarbonyl-3-phenylpropyl)-S-alanyl]-(3′S,5′S)-spirobicyclo[2.2.2]octan-2,3′-pyrrolidin-5′carbonsäure--n-octylamid $[\alpha]_D^{20}$ = -35,5° (c = 1, Methanol)

**Beispiel 7**

1′-[N-(1-S-Ethoxycarbonyl-3-phenylpropyl)-S-alanyl]-(3′S,5′S)-spirobicyclo[2.2.2]octan-2,3′-pyrrolidin-5′-carbonsäure-n-decylamid $[\alpha]_D^{20}$ = -36,6° (c = 1, Methanol)

**Beispiel 8**

1′-[N-(1-S-Ethoxycarbonyl-3-phenylpropyl)-S-alanyl]-(3′S,5′S)-spirobicyclo[2.2.2]octan-2,3′-pyrrolidin-5′-carbonsäure-(N,N-di-n-butylamid) $[\alpha]_D^{20}$ = -38,6° (c = 1, Methanol)

**Beispiel 9**

1′-[N-(1-S-Ethoxycarbonyl-3-phenylpropyl)-S-alanyl]-(3′S,5′S)-spirobicyclo[2.2.2]octan-2,3′-pyrrolidin-5′-carbonsäure-cyclohexylamid $[\alpha]_D^{20}$ = -37,1° (c = 1, Methanol)

**Beispiel 10**

1′-[N-(1-S-Ethoxycarbonyl-3-phenylpropyl)-S-alanyl]-(3′S,5′S)-spirobicyclo[2.2.2]octan-2,3′-pyrrolidin-5′-carbonsäure-piperidid $[\alpha]_D^{20}$ = -36,9° (c = 1, Methanol)

**Beispiel 11**

2-[N-(1S-Ethoxycarbonyl-3-phenylpropyl)-S-alanyl]-(1S,3S,5S)-2-azabicyclo[3.3.0]-3-carbonsäure-N-(8-aminoacetyl)amid

11a) 2-Benzyloxycarbonyl-(1S,3S,5S)-2-azabicyclo[3.3.0]carbonsäure

Zu 6,5 g (42 mmol) (1S,3S,5S)-2-azabicyclo[3.3.0]octan-3-carbonsäure in einem Gemisch aus 65 ml Dioxan und 42 ml 1N Natronlauge wird eine Lösung von 10,7 g (42 mmol) N-(Benzyloxycarbonyloxy)-succinimid in 50 ml Dioxan zugetropft, der Reaktionsansatz über Nacht bei Raumtemperatur gerührt und eingeengt. Der Rückstand wird mit Wasser versetzt und mit 10 %iger Natriumhydrogensulfatlösung auf pH 4 gestellt, die Lösung mit Essigester extrahiert und der Extrakt eingeengt. Das so erhaltene Rohprodukt wird erneut in Wasser aufgenommen, mit 2N Natronlauge alkalisch gestellt und im Vakuum eingeengt. Der Rückstand wird mit Diethylether verrührt, abgesaugt, in Wasser/Essigester suspendiert und mit 2N Salzsäure angesäuert. Die Essigester-Phase wird abgetrennt, getrocknet und eingeengt.
Man erhält das Produkt als gelbes Öl (12,6 g).
$[\alpha]_D^{20}$ = -29,3° (c = 1, Methanol)

11b) 2-Benzyloxycarbonyl-(1S,3S,5S)-2-azabicyclo[3.3.0]octan-3-carbonsäure-N-(8-tert.-butyl-oxycarbonylaminooctyl)-amid

Zu 5,0 g (17.3 mmol) Carbonsäure aus Beispiel 11a) in 100 ml absolutem Methylenchlorid gibt man nacheinander 4,9 g (17,3 mmol)
1-Amino-8-(tert.-butyloxycarbonylamino)octan-hydrochlorid und 6,6 ml (52 mmol) N-Ethylmorpholin. Bei Raumtemperatur tropft man 21,5 ml einer Propanphosphonsäureanhydridlösung (50 % in Methylenchlorid) zu und rührt 3 Stunden. Dann gibt man auf Eiswasser, trennt die organische Phase ab, wäscht mit gesättigter Natriumbicarbonatlösung, 10 %iger Citronensäurelösung und gesättigter Natriumchloridlösung, trocknet, engt ein und reinigt das Rohprodukt (67 g) durch Flash-Säulenchromatographie an Kieselgel (Laufmittel Cyclohexan/Essigester 1:1).
Man erhält 4.94 g des Produktes.
$R_f$ (Cyclohexan/Essigester 1:1) = 0,27; MS (DCI) = 516(M+1)

11c) (1S,3S,5S)-2-Azabicyclo[3.3.0]octan-3-carbonsäure-N-(8-tert.-butyloxycarbonylamino-octyl)amid

5,4 g (10,5 mmol) der Benzyloxycarbonyl-geschützten Verbindung aus Beispiel 11b) werden an 0,56 g Palladium-Kohle (10 %) in 140 ml THF bei Raumtemperatur und Normaldruck hydriert. Man saugt vom Katalysator ab, engt ein, nimmt den Rückstand in Essigester auf, wäscht mit gesättigter Natriumbicarbonatlösung, trocknet und engt ein. Man erhält 35 g (87 %) des Produktes.
$[\alpha]_D^{20}$ = -33,2° (c=1, Methanol)

11d) 2-[N-(1S-Ethoxycarbonyl-3-phenylpropyl)-S-alanyl]-(1S,3S,5S)-2-azabicyclo[3.3.0]octan-3-carbonsäure-N-(8-tert.-butyloxycarbonylamino-octyl)amid

Aus 3,0 g (7,89 mmol) Aminderivat aus Beispiel 11c) und 2,2 g (7.89 mmol) N-(1S-Ethoxycarbonyl-3-phenylpropyl)-S-alanin werden analog dem in Beispiel 1c beschriebenen Verfahren umgesetzt.
Man erhält (ohne säulenchromatographische Reinigung) 4,6 g (91 %) öliges Produkt.
$[\alpha]_D^{20}$ = -21,9° (c=1, Methanol)

11e) 2-[N-(1S-Ethoxycarbonyl-3-phenylpropyl)-S-alanyl]-(1S,3S,5S)-2-azabicyclo[3.3.0]octan-3-carbonsäure-N-(8-aminooctyl)amid

4,5 g (7 mmol) der tert.-Butyloxycarbonyl-geschützten Verbindung aus Beispiel 11d) werden bei -5° C mit 18 ml eiskalter Trifluoressigsäure versetzt und 2 Stunden bei dieser Temperatur gerührt. Die Reaktionsmischung wird eingeengt, der Rückstand in Methylenchlorid aufgenommen, mit gesättigter Natriumbicarbonatlösung gewaschen, getrocknet und eingeengt.
Man erhält 3,8 g (99 %) öliges Produkt.
MS (DCI) = 543 (M+1)
Aus Aceton/Diethylether wird hieraus durch Zusatz von 2 Äquivalenten Oxalsäure das Bis-Hydrogenoxalat hergestellt,
$[\alpha]^{20}$ = +5,0° (c=1, Methanol)

**Ansprüche**

1. Verbindung der Formel II

$$R^3-\underset{\underset{O}{\|}}{C}-\overset{*}{\underset{\underset{R^4}{|}}{CH}}-\underset{\underset{R^5}{|}}{N}-\underset{\underset{O}{\|}}{C}-\overset{*}{\underset{\underset{R^1}{|}}{CH}}-NH-\overset{*}{\underset{\underset{COOR^2}{|}}{CH}}-(CH_2)_n-R \qquad (II)$$

in welcher

I. a) n = 1 oder 2 ist

b) R 1. Wasserstoff bedeutet,

2. Alkyl mit 1 - 18 C-Atomen bedeutet,

3. einen aliphatischen acyclischen Rest der Formel $C_aH_{(2a-b+1)}$ bedeutet, worin Doppelbindungen, falls ihre Zahl 1 übersteigt, nicht kumuliert sind, a für eine ganze Zahl 2 bis 18 und b für eine gerade Zahl 2 bis a stehen;

4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (C-2) stehen;

5. Aryl mit 6 - 12 C-Atomen bedeutet, das durch ($C_1$-$C_8$)-Alkyl, ($C_1$-$C_4$)-Alkoxy, Hydroxy, Halogen, Nitro, Amino, Aminomethyl, ($C_1$-$C_4$)-Alkylamino, Di-($C_1$-$C_4$)-alkylamino, ($C_1$-$C_4$)-Alkanoylamino, Methylendioxy, Carboxy, Cyano oder Sulfamoyl, monosubstituiert sein kann;

6. falls n = 2 ist, ($C_6$-$C_{12}$)-Aryl-($C_1$-$C_8$)-alkyl oder Di-($C_6$-$C_{12}$)-aryl-($C_1$-$C_8$)-alkyl bedeutet, die im Arylteil jeweils wie unter I. b) 5. beschrieben mono-, di- oder trisubstituiert sein können; oder

7. Alkoxy mit 1 - 4 C-Atomen;

8. Aryloxy mit 6 - 12 C-Atomen,
das wie unter I. b) 6. beschrieben substituiert sein kann;

9. mono- bzw. bicyclisches Heteroaryloxy mit 5 - 7 bzw. 8 - 10 Ringatomen, wovon bis zu 9 Ringatome Kohlenstoff und 1 bis 2 Ringatome Schwefel oder Sauerstoff und/oder 1 bis 4 Ringatome Stickstoff darstellen, die im Heteroaryl wie unter I. b) 6. beschrieben substituiert sein können.

10. Amino-($C_1$-$C_8$)-alkyl;

11. ($C_1$-$C_4$)-Alkanoylamino-($C_1$-$C_8$)-alkyl;

12. ($C_7$-$C_{13}$)-Aroylamino-($C_1$-$C_8$)-alkyl, das im Arylteil wie unter I. b) 5. monosubstituiert sein kann;

13. ($C_1$-$C_4$)-Alkoxy-carbonylamino-($C_1$-$C_8$)-alkyl;

14. ($C_6$-$C_{12}$)-Aryl-($C_1$-$C_4$)-alkoxycarbonylamino-($C_1$-$C_8$)-alkyl;

15. ($C_6$-$C_{12}$)-Aryl-($C_1$-$C_4$)-alkylamino-($C_1$-$C_8$)-alkyl;

16. ($C_1$-$C_4$)-Alkylamino-($C_1$-$C_8$)-alkyl;

17. Di-($C_1$-$C_4$)-alkylamino-($C_1$-$C_8$)-alkyl;

18. Guanidino-($C_1$-$C_8$)-alkyl;

19. Imidazolyl;

20. Indolyl;

21. ($C_1$-$C_4$)-Alkylthio;

22. falls n = 2 ist, ($C_1$-$C_4$)-Alkylthio-($C_1$-$C_8$)-alkyl;

23. ($C_6$-$C_{12}$)-Arylthio-($C_1$-$C_8$)-alkyl;
das im Arylteil wie unter I. b) 6. beschrieben, substituiert sein kann,

24. ($C_6$-$C_{12}$)-Aryl-($C_1$-$C_8$)-alkylthio,
das im Arylteil wie unter I. b) 6. beschrieben substituiert sein kann;

25. falls n = 2 ist, Carboxy-($C_1$-$C_8$)-alkyl;

26. Carboxy;

27. Carbamoyl;

28. falls n = 2 ist, Carbamoyl-($C_1$-$C_8$)-alkyl;

29. ($C_1$-$C_4$)-Alkoxy-carbonyl-($C_1$-$C_8$)-alkyl;

30. falls n = 2 ist, ($C_6$-$C_{12}$)-Aryloxy-($C_1$-$C_8$)-alkyl, das im Arylteil unter I. b) 6. beschrieben substituiert sein kann; oder

31. ($C_6$-$C_{12}$)-Aryl-($C_1$-$C_8$)-alkoxy,
das im Arylteil wie unter I. b) 6. beschrieben substituiert sein kann, bedeutet;

c) $R^1$ 1. Wasserstoff bedeutet;

2. Alkyl mit 1 - 18 C-Atomen bedeutet;

3. einen aliphatischen acyclischen Rest der Formel $C_aH_{(2a-b+1)}$ bedeutet, worin Doppelbindungen, falls ihre Zahl 1 übersteigt, nicht kumuliert sind, a für eine ganze Zahl 2 bis 18 und b für eine gerade Zahl 2 bis a stehen;

4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (c-2) stehen; oder

5. Aryl mit 6 - 12 C-Atomen,
das wie unter I. b) 6. beschrieben substituiert sein kann;

6. ($C_6$-$C_{12}$)-Aryl-($C_1$-$C_8$)-alkyl oder ($C_7$-$C_{13}$)-Aroyl-($C_1$-$C_8$)-alkyl,
die beide im Arylteil wie unter I. b) 6. beschrieben substituiert sein können;

7. mono- bzw. bicyclisches, gegebenenfalls teilhydriertes Heteroaryl oder Heteroaryl-($C_1$-$C_8$)-alkyl mit 5 - 7 bzw. 8 - 10 Ringatomen, wovon bis zu 9 Ringatome Kohlenstoff und 1 bis 2 Ringatome Schwefel-oder Sauerstoffatome und/oder 1 bis 4 Ringatome Stickstoffatome darstellen, die im Heteroaryl wie bei Aryl unter I. b) 6. beschrieben substituiert sein können;

8. falls von c) 1. -7. noch nicht umfaßt, die gegebenenfalls geschützte Seitenkette einer natürlich vorkommenden α-Aminosäure der Formel $R^1$-$CH(NH_2)$-COOH; oder

9. Falls von c) 1.-8. noch nicht umfaßt, die gegebenenfalls geschützte Seitenkette von Cyclohexylalanin, 2-Thienylalanin, 3-Thienylalanin, O-($C_3$-C5)-Alkyl-Tyrosin oder C-Phenylglycin bedeutet.

d) $R^2$

1. Wasserstoff bedeutet;

2. Alkyl mit 1 - 18 C-Atomen bedeutet;

3. einen aliphatischen acyclischen Rest der Formel $C_aH_{(2a-b+1)}$ bedeutet, worin Doppelbindungen, falls ihre Zahl 1 übersteigt, nicht kumuliert sind, a für eine ganze Zahl 2 bis 18 und b für eine gerade Zahl 2 bis a stehen;

4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (c-2) stehen; oder

5. Di-($C_1$-$C_4$)-alkylamino-($C_1$-$C_8$)-alkyl;

6. ($C_1$-$C_5$)-Alkanoyloxy-($C_1$-$C_8$)-alkyl;

7. ($C_1$-$C_6$)-Alkoxy-carbonyloxy-($C_1$-$C_8$)-alkyl;

8. ($C_7$-$C_{13}$)-Aroyloxy-($C_1$-$C_8$)-alkyl;

9. ($C_6$-$C_{12}$)-Aryloxycarbonyloxy($C_1$-$C_8$)-alkyl;

10. Aryl mit 6 - 12 C-Atomen;

11. ($C_7$-$C_{20}$)-Aralkyl;

12. Phthalidyl;

13. einen Rest der Formel

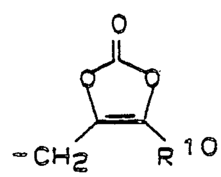

worin $R^{10}$ Wasserstoff, ($C_1$-$C_6$)-Alkyl oder Aryl mit 6 - 12 C-Atomen ist; oder

14. einen Rest der Formel

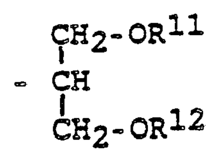

bedeutet,

worin $R^{11}$ und $R^{12}$ gleich oder verschieden unabhängig voneinander Wasserstoff, einen gegebenenfalls substituierten Alkylrest mit 1 - 23 C-Atomen oder einen gegebenenfalls substituierten Acylrest mit 1 - 23 C-Atomen bedeuten

wobei die unter d) 8., 9., 10., 11. und 12. genannten Reste wie unter I. b) 6. beschrieben im Arylteil substituiert sein können;

e) $R^3$ einen Rest der Formel

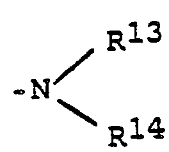

bedeutet,
worin $R^{13}$ und $R^{14}$ gleich oder verschieden unabhängig voneinander
1. Wasserstoff bedeuten;
2. Alkyl mit 1 - 21 C-Atomen bedeuten;
3. einen aliphatischen acyclischen Rest der Formel $C_aH_{(2a-b+1)}$ bedeuten, worin a für eine ganze Zahl 2 bis 21 und b für eine gerade Zahl 2 bis a stehen;
4. einen mono-, di-, tri-, tetra- oder pentacyclischen, nicht aromatischen Kohlenwasserstoffrest der Formel $C_cH_{(2c-d-1)}$ bedeuten, der gegebenenfalls verzweigt ist, worin c für eine ganze Zahl 3 - 20 und d für eine gerade Zahl 0 bis (c-2) stehen; oder
5. Amino-($C_1$-$C_{16}$)-alkyl;
6. Mono-($C_1$-$C_4$)-alkylamino-($C_1$-$C_{16}$)alkyl;
7. Di-($C_1$-$C_4$)-alkylamino-($C_1$-$C_{13}$)alkyl;
8. ($C_1$-$C_5$)-Alkanoyloxy-($C_1$-$C_{16}$)-alkyl;
9. ($C_1$-$C_6$)-Alkoxy-carbonyloxy-($C_1$-$C_{15}$)-alkyl;
10. ($C_7$-$C_{13}$)-Aroyloxy-($C_1$-$C_8$)-alkyl;
11. ($C_6$-$C_{12}$)-Aryloxy-carbonyloxy-($C_1$-$C_9$)alkyl;
12. Aryl mit 6 - 12 C-Atomen, oder
13. ($C_7$-$C_{20}$)-Aralkyl bedeuten
oder zusammen mit dem sie tragenden Stickstoffatom einen gegebenenfalls substituierten, gegebenenfalls teilweise oder vollständig hydrierten heteroaromatischen Rest mit 2 - 24 C-Atomen bilden und
f) $R^4$ und $R^5$ zusammen mit den sie tragenden Atomen ein mono-, bi- oder tricyclisches heterocyclisches Ringsystem mit 3 bis 15 Ring C-Atomen bilden
oder deren physiologisch verträgliche Salze mit Säuren oder Basen,

II. <u>ausgenommen</u> Verbindungen der Formel II und deren Salze, worin
a) n = 1 oder 2 ist;
b) R 1. Wasserstoff;
2. Alkyl mit 1 - 8 C-Atomen;
3. Alkenyl mit 2 - 8 C-Atomen;
4. Cycloalkyl mit 3 - 9 C-Atomen;
5. Aryl mit 6 - 12 C-Atomen,
das durch ($C_1$-$C_4$)-Alkyl, ($C_1$-$C_4$)-Alkoxy, Hydroxy, Halogen, Nitro, Amino, Aminomethyl, ($C_1$-$C_4$)Alkylamino, Di-($C_1$-$C_4$)-alkylamino, ($C_1$-$C_4$)Alkanoylamino, Methylendioxy, Carboxy, Cyano und/oder Sulfamoyl mono-, di- oder trisubstituiert sein kann;
6. Alkoxy mit 1 - 4 C-Atomen;
7. Aryloxy mit 6 - 12 C-Atomen,
das wie unter II. b) 5. beschrieben substituiert sein kann;
8. mono- bzw. bicyclisches Heteroaryloxy mit 5-7 bzw. 8 - 10 Ringatomen, wovon 1 bis 2 Ringatome Schwefel- oder Sauerstoffatome und/oder 1 bis 4 Ringatome Stickstoff darstellen, das wie unter II. b) 5. beschrieben substituiert sein kann;
9. Amino-($C_1$-$C_4$)-alkyl;
10. ($C_1$-$C_4$)-Alkanoylamino-($C_1$-$C_4$)-alkyl;
11. ($C_7$-$C_{13}$)-Aroylamino-($C_1$-$C_4$)-alkyl;
12. ($C_1$-$C_4$)-Alkoxy-carbonylamino-($C_1$-$C_4$)-alkyl;
13. ($C_6$-$C_{12}$)-Aryl-($C_1$-$C_4$)-alkoxycarbonylamino-($C_1$-$C_4$)-alkyl;
14. ($C_6$-$C_{12}$)-Aryl-($C_1$-$C_4$)-alkylamino-($C_1$-$C_4$)-alkyl;
15. ($C_1$-$C_4$)-Alkylamino-($C_1$-$C_4$)-alkyl;
16. Di-($C_1$-$C_4$)-alkylamino-($C_1$-$C_4$)-alkyl;
17. Guanidino-($C_1$-$C_4$)-alkyl;
18. Imidazolyl;
19. Indolyl;
20. ($C_1$-$C_4$)-Alkylthio;
21. ($C_1$-$C_4$)-Alkylthio-($C_1$-$C_4$)-alkyl;
22. ($C_6$-$C_{12}$)-Arylthio-($C_1$-$C_4$)-alkyl,
das im Arylteil wie unter II. b) 5. beschrieben substituiert sein kann;
23. ($C_6$-$C_{12}$)-Aryl-($C_1$-$C_4$)-alkylthio,
das im Arylteil wie unter II. b) 5. beschrieben substituiert sein kann;
24. Carboxy-($C_1$-$C_4$)-alkyl;
25. Carboxy;

26. Carbamoyl;
27. Carbamoyl-($C_1$-$C_4$)-alkyl;
28. ($C_1$-$C_4$)-Alkoxy-carbonyl-($C_1$-$C_4$)-alkyl;
29. ($C_6$-$C_{12}$)-Aryloxy-($C_1$-$C_4$)-alkyl,
das im Arylteil wie unter II. b) 5. beschrieben substituiert sein kann; oder
30. ($C_6$-$C_{12}$)-Aryl-($C_1$-$C_4$)-alkoxy,
das im Arylteil wie unter II. b) 5. beschrieben substituiert sein kann, bedeutet;
c) $R^1$ 1. Wasserstoff;
2. Alkyl mit 1 - 6 C-Atomen;
3. Alkenyl mit 2 - 6 C-Atomen;
4. Alkinyl mit 2 - 6 C-Atomen;
5. Cycloalkyl mit 3 - 9 C-Atomen;
6. Cycloalkenyl mit 5 - 9 C-Atomen;
7. ($C_3$-$C_9$)-Cycloalkyl-($C_1$-$C_4$)-alkyl;
8. ($C_5$-$C_9$)-Cycloalkenyl-($C_1$-$C_4$)-alkyl;
9. gegebenenfalls teilhydriertes Aryl mit 6 - 12 C-Atomen, das wie II. b) 5. beschrieben substituiert sein kann;
10. ($C_6$-$C_{12}$)-Aryl-($C_1$-$C_4$)-alkyl oder ($C_7$-$C_{13}$)-Aroyl-($C_1$ oder $C_2$)-alkyl,
die beide wie im Arylteil wie unter II. b) 5. beschrieben substituiert sein können;
11. mono- bzw. bicyclisches, gegebenenfalls teilhydriertes Heteroaryl mit 5 - 7 bzw. 8 - 10 Ringatomen, woven 1 bis 2 Ringatome Schwefel- oder Sauerstoffatome und/oder 1 bis 4 Ringatome Stickstoffatome darstellen, das wie unter II. b) 5. beschrieben substituiert sein kann; oder
12. Falls von den vorstehenden Definitionen nicht umfaßt, die gegebenenfalls geschützte Seitenkette einer natürlich vorkommenden α-Aminosäure der Formel $R^1$-$CH(NH_2)$-$COOH$ bedeutet;
d) $R^2$
1. Wasserstoff;
2. Alkyl mit 1 - 6 C-Atomen;
3. Alkenyl mit 2 - 6 C-Atomen;
4. Di-($C_1$-$C_4$)-alkylamino-($C_1$-$C_4$)-alkyl;
5. ($C_1$-$C_5$)-Alkanoyloxy-($C_1$-$C_4$)-alkyl;
6. ($C_1$-$C_6$)-Alkoxy-carbonyloxy-($C_1$-$C_4$)-alkyl;
7. ($C_7$-$C_{13}$)-Aroyloxy-($C_1$-$C_4$)-alkyl;
8. ($C_6$-$C_{12}$)-Aryloxycarbonyloxy($C_1$-$C_4$)-alkyl;
9. Aryl mit 6 - 12 C-Atomen;
10. ($C_6$-$C_{12}$)-Aryl-($C_1$-$C_4$)-alkyl;
11. ($C_3$-$C_9$)-Cycloalkyl; oder
12. ($C_3$-$C_9$)-Cycloalkyl-($C_1$-$C_4$)-alkyl bedeutet ,
e) $R^3$ einen Rest der Formel

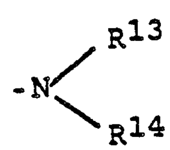

bedeutet,
worin $R^{13}$ und $R^{14}$ gleich oder verschieden unabhängig voneinander
1. Wasserstoff;
2. Alkyl mit 1 - 6 C-Atomen; oder
3. ($C_6$-$C_{12}$)-Aryl-($C_1$-$C_4$)-alkyl bedeuten und
f) $R^4$ und $R^5$ zusammen mit den sie tragenden Atomen ein mono-, bi- oder tricyclisches heterocyclisches Ringsystem mit 3 bis 15 Ring-C-Atomen bilden.

2. Verbindung der Formel II gemäß Anspruch 1, in welcher
n = 1 oder 2 ist
R = Wasserstoff,
Alkyl mit 1 - 8 C-Atomen,
Alkenyl mit 2 - 6 C-Atomen,

Cycloalkyl mit 3 - 9 C-Atomen,
Aryl mit 6 - 12 C-Atomen,
das durch ($C_1$-$C_4$)-Alkyl, ($C_1$-$C_4$)-Alkoxy, Hydroxy, Halogen, Nitro, Amino, Aminomethyl, ($C_1$-$C_4$)-Alkylamino, Di-($C_1$-$C_4$)-alkylamino, ($C_1$-$C_4$)-Alkanoylamino, Methylendioxy, Carboxy, Cyano oder Sulfamoyl, monosubstituiert sein kann,
Alkoxy mit 1 - 4 C-Atomen,
Aryloxy mit 6 - 12 C-Atomen,
das wie oben bei Aryl beschrieben mono-, di- oder trisubstituiert sein kann,
mono- bzw. bicyclisches Heteroaryloxy mit 5 - 7 bzw. 8 - 10 Ringatomen, wovon 1 bis 2 Ringatome Schwefel-oder Sauerstoffatome und/oder 1 bis 4 Ringatome Stickstoff darstellen, das wie oben bei Aryloxy beschrieben substituiert sein kann,
Amino-($C_1$-$C_4$)-alkyl,
($C_1$-$C_4$)-Alkanoylamino-($C_1$-$C_4$)alkyl,
($C_7$-$C_{13}$)-Aroylamino-($C_1$-$C_4$)-alkyl, das wie oben bei Aryl beschrieben substituiert sein kann,
($C_1$-$C_4$)-Alkoxy-carbonylamino-($C_1$-$C_4$)-alkyl,
($C_6$-$C_{12}$)-Aryl-($C_1$-$C_4$)-alkoxycarbonylamino-($C_1$-$C_4$)-alkyl,
($C_6$-$C_{12}$)-Aryl-($C_1$-$C_4$)-alkylamino-($C_1$-$C_4$)-alkyl,
($C_1$-$C_4$)-Alkylamino-($C_1$-$C_4$)-alkyl,
Di-($C_1$-$C_4$)-alkylamino-($C_1$-$C_4$)-alkyl,
Guanidino-($C_1$-$C_4$)-alkyl,
Imidazolyl, Indolyl,
($C_1$-$C_4$)-Alkylthio,
($C_1$-$C_4$)-Alkylthio-($C_1$-$C_4$)-alkyl,
($C_6$-$C_{12}$)-Arylthio-($C_1$-$C_4$-alkyl,
das im Arylteil wie oben bei Aryloxy beschrieben, substituiert sein kann,
($C_6$-$C_{12}$)-Aryl-($C_1$-$C_4$)-alkylthio,
das im Arylteil wie oben bei Aryloxy beschrieben substituiert sein kann,
Carboxy-($C_1$-$C_4$)-alkyl,
Carboxy, Carbamoyl,
Carbamoyl-($C_1$-$C_4$)-alkyl,
($C_1$-$C_4$)-Alkoxy-carbonyl-($C_1$-$C_4$)-alkyl,
($C_6$-$C_{12}$)-Aryloxy-($C_1$-$C_4$)-alkyl,
das im Arylteil wie oben bei Aryloxy beschrieben substituiert sein kann oder
($C_6$-$C_{12}$)-Aryl-($C_1$-$C_4$)-alkoxy,
das im Arylteil wie oben bei Aryloxy beschrieben substituiert sein kann,
$R^1$ Wasserstoff,
Alkyl mit 1 - 6 C-Atomen,
Alkenyl mit 2 - 6 C-Atomen,
Alkinyl mit 2 - 6 C-Atomen,
Cycloalkyl mit 3 - 9 C-Atomen,
Cycloalkenyl mit 5 - 9 C-Atomen,
($C_3$-$C_9$)-Cycloalkyl-($C_1$-$C_4$)-alkyl,
($C_5$-$C_9$)-Cycloalkenyl-($C_1$-$C_4$)-alkyl,
gegebenenfalls teilhydriertes Aryl mit 6 - 12 C-Atomen, das wie oben bei R beschrieben mono-, bi-oder trisubstituiert sein kann, ($C_6$-$C_2$)-Aryl-($C_1$-$C_4$)-alkyl oder ($C_7$-$C_{13}$)-Aroyl-($C_1$ oder $C_2$)-alkyl
die beide wie das vorstehende Aryl substituiert sein können,
mono- bzw. bicyclisches, gegebenenfalls teilhydriertes Heteroaryl mit 5 - 7 bzw. 8 - 10 Ringatomen, wovon 1 bis 2 Ringatome Schwefel- oder Sauerstoffatome und/oder 1 bis 4 Ringatome Stickstoffatome darstellen, das wie das vorstehende Aryl substituiert sein kann oder
die gegebenenfalls geschützte Seitenkette einer natürlich vorkommenden α-Aminosäure $R^1$-CH($NH_2$)-COOH bedeutet,
$R^2$ Wasserstoff,
Alkyl mit 1 - 6 C-Atomen,
Alkenyl mit 2 - 6 C-Atomen,
Di-($C_1$-$C_4$)-alkylamino-($C_1$-$C_4$)-alkyl,
($C_1$-$C_5$)-Alkanoyloxy-($C_1$-$C_4$)-alkyl,
($C_1$-$C_6$)-Alkoxy-carbonyloxy-($C_1$-$C_4$)-alkyl,
($C_7$-$C_{13}$)-Aroyloxy-($C_1$-$C_4$)-alkyl,

$(C_6-C_{12})$-Aryloxycarbonyloxy$(C_1-C_4)$-alkyl,
Aryl mit 6 - 12 C-Atomen,
$(C_6-C_{12})$-Aryl-$(C_1-C_4)$-alkyl,
$(C_3-C_9)$-Cycloalkyl oder
$(C_3-C_9)$-Cycloalkyl-$(C_1-C_4)$-alkyl
bedeutet,
$R^3$ einen Rest der Formel

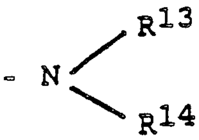

bedeutet,
worin $R^{13}$ und $R^{14}$ gleich oder verschieden unabhängig voneinander
Wasserstoff,
Alkyl mit 1 - 16 C-Atomen,
Alkenyl mit 2 - 16 C-Atomen,
Alkinyl mit 2 - 16 C-Atomen,
Cycloalkyl mit 3 - 9 C-Atomen,
$(C_3-C_9)$-Cycloalkyl-$(C_1-C_4)$-alkyl,
Amino-$(C_1-C_{16})$-alkyl,
Mono-$(C_1-C_4)$-alkylamino-$(C_1-C_{12})$alkyl,
Di-$(C_1-C_4)$-alkylamino-$(C_1-C_8)$-alkyl,
$(C_1-C_5)$-Alkanoyloxy-$(C_1-C_{11})$-alkyl,
$(C_1-C_6)$-Alkoxycarbonyloxy-$(C_1-C_{10})$-alkyl,
$(C_7-C_{13})$-Aroyloxy-$(C_1-C_6)$-alkyl,
$(C_6-C_{12})$-Aryloxycarbonyloxy$(C_1-C_6)$-alkyl,
Aryl mit 6 - 12 C-Atomen oder $(C_6-C_{12})$-Aryl-$(C_1-C_6)$-alkyl bedeuten
oder zusammen mit dem sie tragenden Stickstoffatom, gegebenenfalls durch Amino, $(C_1-C_4)$-Alkylamino-, Di-$(C_1-C_4)$-alkylamino, $(C_1-C_5)$-Alkanoyloxy-, $(C_1-C_6)$-Alkoxycarbonyloxy-, $(C_7-C_{13})$-Aroyloxy- oder $(C_6-C_{12})$-Aryloxycarbonyloxy substituiertes
Pyrrolidinyl,
Piperidinyl,
Piperazinyl,
Homopiperazinyl,
Morpholinyl,
Thiomorpholinyl,
Imidazolyl,
Pyridyl,
Pyrimidinyl,
sym.-Triazinyl bedeuten
und
f) $R^4$ und $R^5$ zusammen mit den sie tragenden Atomen ein mono-, bi- oder tricyclisches heterocyclisches Ringsystem mit 3 bis 15 Ring-C-Atomen bilden
oder deren physiologisch verträgliche Salze mit Säuren oder Basen.

3. Verbindung der Formel II gemäß Anspruch 1 oder 2, in welcher
n = 1 oder 2 ist,
R $(C_1-C_6)$-Alkyl, $(C_2-C_6)$-Alkenyl, $(C_3-C_9)$-Cycloalkyl, Amino-$(C_1-C_4)$-alkyl, $(C_2-C_5)$-Acylamino-$(C_1-C_4)$-alkyl, $(C_7-C_{13})$-Aroylamino-$(C_1-C_4)$-alkyl, $(C_1-C_4)$-Alkoxycarbonylamino-$(C_1-C_4)$-alkyl, $(C_6-C_{12})$-Aryl-$(C_1-C_4)$-alkoxycarbonylamino-$(C_1-C_4)$-alkyl, $(C_6-C_{12})$-Aryl, das durch $(C_1-C_4)$-Alkyl, $(C_1-C_4)$-Alkoxy, Hydroxy, Halogen, Nitro, Amino, $(C_1-C_4)$-Alkylamino, Di-$(C_1-C_4)$-alkylamino oder Methylendioxy monosubstituiert sein kann, oder 3-Indolyl, insbesondere Methyl, Ethyl, Cyclohexyl, tert. Butoxycarbonylamino-$(C_1-C_4)$-alkyl, Benzoyloxycarbonylamino-$(C_1-C_4)$-alkyl oder Phenyl, das durch Phenyl, $(C_1-C_2)$-Alkyl, ($C_1$ oder $C_2$)-Alkoxy, Hydroxy, Fluor, Chlor, Brom, Amino, $(C_1-C_4)$-Alkylamino, Di-$(C_1-C_4)$alkylamino oder Nitro monosubstituiert sein kann, bedeutet,
$R^1$ Wasserstoff oder $(C_1-C_6)$-Alkyl, das gegebenenfalls durch Amino, $(C_1-C_6)$-Acylamino oder Benzoylamino

monosubstituiert sein kann, ($C_2$-$C_6$)-Alkenyl, ($C_3$-$C_9$)-Cycloalkyl, ($C_5$-$C_9$)-Cycloalkenyl, ($C_3$-$C_7$)-Cycloalkyl-($C_1$-$C_4$)-alkyl, ($C_6$-$C_{12}$)-Aryl oder teilhydriertes Aryl, das jeweils durch ($C_1$-$C_4$)-Alkyl, ($C_1$ oder $C_2$)-Alkoxy oder Halogen substituiert sein kann, ($C_6$-$C_{12}$)-Aryl-($C_1$ bis $C_4$)-alkyl oder ($C_7$-$C_{13}$)-Aroyl-($C_1$-$C_2$)-alkyl, die beide wie vorstehend definiert im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder 1 bis 4 Ringatome Stickstoffatome darstellen, oder eine Seitenkette einer natürlich vorkommenden, gegebenenfalls geschützten α-Aminosäure, insbesondere aber Wasserstoff, ($C_1$-$C_3$)-Alkyl, ($C_2$ oder $C_3$)-Alkenyl, die gegebenenfalls geschützte Seitenkette von Lysin, Benzyl, 4-Methoxybenzyl, 4-Ethoxybenzyl, Phenethyl, 4-Amino-butyl oder Benzoylmethyl bedeutet,

$R^2$ Wasserstoff,
($C_1$-$C_6$)-Alkyl, ($C_2$-$C_6$)-Alkenyl oder ($C_6$-$C_{12}$)-Aryl-($C_1$-$C_4$)-alkyl, insbesondere aber Wasserstoff, ($C_1$-$C_4$)-Alkyl oder Benzyl bedeutet,

$R^3$ einen Rest der Formel

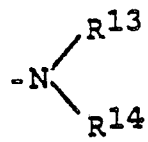

bedeutet,
worin $R^{13}$ und $R^{14}$ gleich oder verschieden unabhängig voneinander Wasserstoff, ($C_4$-$C_{12}$)-Alkyl, ($C_4$-$C_{12}$)-Alkenyl, ($C_5$-$C_7$)-Cycloalkyl, ($C_5$-$C_7$)-Cycloalkyl-($C_1$-$C_4$)-alkyl, Amino-($C_4$-$C_{12}$)-alkyl, ($C_1$-$C_4$)-Alkylamino-($C_4$-$C_{12}$)-alkyl, Di-($C_1$-$C_4$)-Alkylamino-($C_1$-$C_8$)-alkyl, ($C_6$-$C_{12}$)-Aryl-($C_1$-$C_6$)-alkyl, bedeuten oder zusammen mit dem sie tragenden Stickstoffatom gegebenenfalls durch Amino, ($C_1$-$C_4$)-Alkylamino oder Di-($C_1$-$C_4$)-alkylamino substituiertes Pyrrolidinyl, Piperidinyl, Piperazinyl, Homopiperazinyl, Morpholinyl oder Thiomorpholinyl bedeuten,

und

$R^4$ und $R^5$ zusammen mit den sie tragenden Atomen ein mono-, bi- oder tricylisches Ringsystem mit 3 bis 15 Ring-C-Atomen bilden
oder deren physiologisch verträgliche Salze mit Säuren und Basen.

4. Verbindung der Formel II gemäß einem der Ansprüche 1 bis 3, in welcher $R^4$ und $R^5$ zusammen mit den diese tragenden Atomen ein mono-, bi- oder tricyclisches heterocyclisches Ringsystem mit 3 bis 15 Ring-C-Atomen, bis zu 2 Ring-S-Atomen und bis zu 2 Ring-N-Atomen vorzugsweise aus der Reihe Pyrrolidin, Thiazolidin, Tetrahydroisochinolin, Decahydroisochinolin, Octahydroindol, Indolin, Octahydrocyclopenta[b]pyrrol, 2-Azaspiro[4.5]decan, 2-Azaspiro[4.4]-nonan, Spiro[(bicyclo[2.2.1]heptan)-2,3′-pyrrolidin], Spiro[(bicyclo[2.2.2]octan)-2,3′-pyrrolidin], 2-Azatricyclo[4.3.0.$1^{6,9}$]decan, Decahydrocyclohepta[b]pyrrol, Octahydroisoindol, Octahydrocyclopenta[c]pyrrol, 2,3,3a,4,5,7a-Hexahydroindol und 1,2,3,3a,4,6a-Hexahydrocylopenta[b]pyrrol bilden, oder deren physiologisch verträglichen Salze.

5. Verfahren zur Herstellung einer Verbindung der Formel II gemäß einem der Ansprüche 1 bis 4, dadurch gekennzeichnet, daß man ihre Fragmente in einem geeigneten Lösungsmittel gegebenenfalls in Gegenwart einer Base und/oder eines Kupplungshilfsmittels miteinander umsetzt, gegebenenfalls intermediär entstehende ungesättigte Verbindungen, wie Schiffsche Basen, reduziert, zum Schutz reaktiver Gruppen temporär eingeführte Schutzgruppen abspaltet, Verbindungen der Formel II mit freier(en) Carboxylgruppe(n) gegebenenfalls verestert oder in Amide überführt und die erhaltenen Verbindungen gegebenenfalls in ihre physiologisch verträglichen Salze überführt.

6. Verfahren gemäß Anspruch 5, dadurch gekennzeichnet, daß man
a) eine Verbindung der Formel III

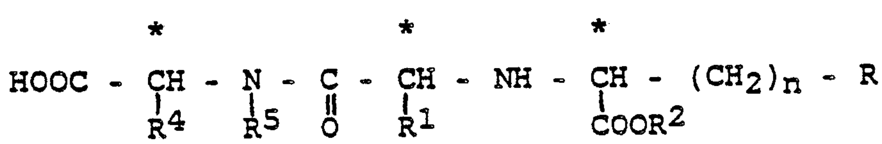

(III)

worin R, $R^1$, $R^2$, $R^4$, $R^5$ und n die gleiche Bedeutung wie in Formel II haben, mit einem Amin der Formel IV

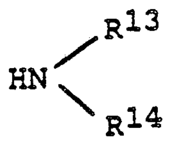

(IV)

worin $R^{13}$ und $R^{14}$ die gleiche Bedeutung wie in Formel II haben, umsetzt, oder daß man

b) eine Verbindung der Formel V

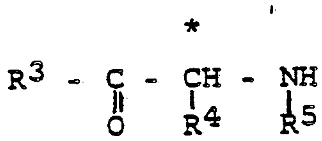

(V)

worin $R^3$, $R^4$ und $R^5$ die gleiche Bedeutung wie in Formel II haben, mit einer Verbindung der Formel VI

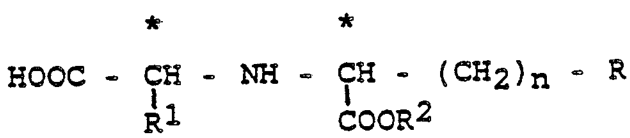

(VI)

worin R, $R^1$, $R^2$ und n die gleiche Bedeutung wie in Formel II haben, umsetzt, oder daß man

c) eine Verbindung der Formel VII

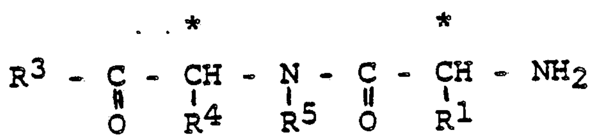

(VII)

worin $R^1$, $R^3$, $R^4$ und $R^5$ die gleiche Bedeutung wie in Formel II haben, mit einer Verbindung der Formel VIII

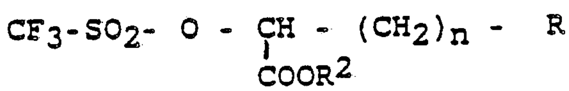

(VIII)

worin R, $R^2$ und n die gleiche Bedeutung wie in Formel II haben, umsetzt, oder daß man

d) eine Verbindung der Formel IX,

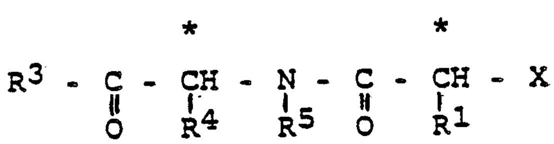

(IX)

worin $R^1$, $R^3$, $R^4$ und $R^5$ die gleiche Bedeutung wie in Formel II haben und in der X eine Abgangsgruppe, die nucleophil verdrängt werden kann, insbesondere ein Cl, Br, J-Atom oder einen Sulfonsäurerest, bedeutet, mit einer Verbindung der Formel X

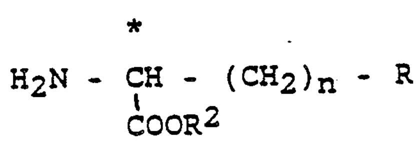

(X)

in der R, $R^2$ und n die gleiche Bedeutung wie in Formel II haben, unter Bedingungen einer nucleophilen Substitution, umsetzt,

oder daß man

e) eine Verbindung der Formel XI

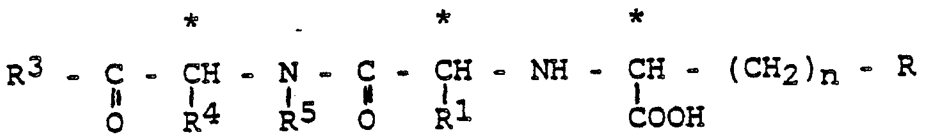 (XI)

in der R, $R^1$, $R^3$, $R^4$, $R^5$ und n die gleiche Bedeutung wie in Formel II haben, unter Anwendung einer der gebräuchlichen Veresterungsmethoden in Verbindungen der Formel II überführt, beispielsweise durch Umsetzung mit einem Alkohol $R^2$-OH unter saurer Katalyse oder nach Aktivierung der Carbonsäurefunktion von XI oder nach Aktivierung der Hydroxyfunktion von $R^2OH$, z.B. unter den Bedingungen einer Mitsunobu-Reaktion,

oder durch Umsetzung mit einer Verbindung der Formel $R^2$ X, worin $R^2$ die gleiche Bedeutung wie in Formel II und X die gleiche Bedeutung wie in Formel IX hat unter den Bedingungen einer nucleophilen Substitution, wie sie unter der Verfahrensvariante d) beschrieben sind,

oder durch Umsetzung mit einem Diazoalkan in einem inerten organischen Lösungsmittel,

oder daß man

f) eine Verbindung der Formel XII

$R^2OOC\text{-}CH=CH\text{-}CO\text{-}R$ (XII),

in welcher R und $R^2$ die gleiche Bedeutung wie in Formel II haben, mit einer Verbindung der Formel VII umsetzt und die Carbonylgruppe hydriert,

oder daß man

g) eine Verbindung der Formel VII mit einer Verbindung der Formel XIII

$R\text{-}(CH_2)_n\text{-}\underset{\overset{\|}{O}}{C}\text{-}COOR^2$ (XIII)

worin R, $R^2$ und n die gleiche Bedeutung wie in Formel II haben, umsetzt und die erhaltenen Schiff-Basen reduziert,

und die so erhaltenen Verbindungen der Formel II gegebenenfalls in ihre physiologisch verträglichen Salze überführt.

7. Verbindung der Formel V

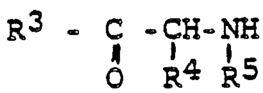 (V),

worin

$R^3$ einen Rest der Formel

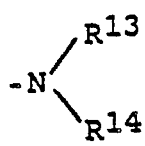

bedeutet,

worin $R^{13}$ und $R^{14}$ gleich oder verschieden unabhängig voneinander

Wasserstoff

Alkyl mit 7 - 16 C-Atomen,

Alkenyl mit 7 - 16 C-Atomen,

Alkinyl mit 7 - 16 C-Atomen,

Cycloalkyl mit 3 - 9 C-Atomen,

$(C_3\text{-}C_9)$-Cycloalkyl-$(C_1\text{-}C_4)$-alkyl,

Amino-$(C_1\text{-}C_{16})$-alkyl,

Mono-$(C_1\text{-}C_4)$-alkylamino-$(C_1\text{-}C_{12})$alkyl,

Di-($C_1$-$C_4$)-alkylamino-($C_1$-$C_8$)alkyl,
($C_1$-$C_5$)-Alkanoyloxy-($C_1$-$C_{11}$)-alkyl,
($C_1$-$C_6$)-Alkoxycarbonyloxy-($C_1$-$C_{10}$)-alkyl,
($C_7$-$C_{13}$)-Aroyloxy-($C_1$-$C_6$)-alkyl,
($C_6$-$C_{12}$)-Aryloxycarbonyloxy-($C_1$-$C_6$)-alkyl,
Aryl mit 6 - 12 C-Atomen oder
($C_6$-$C_{12}$)-Aryl-($C_5$-$C_6$)-alkyl bedeuten
oder zusammen mit dem sie tragenden Stickstoffatom, gegebenenfalls durch Amino, ($C_1$-$C_4$)-Alkylamino, Di-($C_1$-$C_4$)-alkylamino, ($C_1$-$C_5$)-Alkanoyloxy,
($C_1$-$C_6$)-Alkoxycarbonyloxy, ($C_7$-$C_{13}$)-Aroyloxy oder ($C_6$-$C_{12}$)-Aryloxycarbonyloxy substituiertes
Pyrrolidinyl,
Piperidinyl,
Piperazinyl,
Homopiperazinyl,
Morpholinyl,
Thiomorpholinyl,
Imidazolyl,
Pyridyl,
Pyrimidinyl,
sym.-Triazinyl bedeuten
und
$R^4$ und $R^5$ zusammen mit den sie tragenden Atomen ein mono-, bi- oder tricyclisches heterocyclisches Ringsystem mit 3 bis 15 Ring-C-Atomen bilden.

8. Verfahren zur Herstellung einer Verbindung der Formel V gemäß Anspruch 7, dadurch gekennzeichnet, daß man in Verbindungen der Formel XIV,

$$\mathrm{HOOC - \underset{R^4}{\underset{|}{CH}} - \underset{R^5}{\underset{|}{NH}}} \qquad (XIV),$$

worin $R^4$ und $R^5$ die gleiche Bedeutung wie in Formel V haben, die Aminogruppe schützt, dann mit einem Amin der Formel IV
$R^{13}$ - NH - $R^{14}$ (IV),
worin $R^{13}$ und $R^{14}$ die gleiche Bedeutung wie in Formel V haben, umsetzt und die Amino-Schutzgruppe wieder abspaltet.

9. Verbindung gemäß einem oder mehrerer der Ansprüche 1 bis 4 zur Anwendung als Heilmittel.

10. Verbindung gemäß einem oder mehrerer der Ansprüche 1 bis 4 zur Anwendung als Heilmittel bei der Behandlung des Bluthochdrucks.

11. Verbindung gemäß einem oder mehrerer der Ansprüche 1 bis 4 zur Anwendung als Heilmittel bei der Behandlung von Störungen des zentralen Nervensystems.

12. Verbindung gemäß einem oder mehrerer der Ansprüche 1 bis 4 zur Anwendung als Heilmittel bei der Behandlung cognitiver Dysfunktionen.

13. Pharmazeutisches Mittel enthaltend eine Verbindung gemäß einem oder mehrerer der Ansprüche 1 bis 4.

14. Verfahren zur Herstellung eines Mittels gemäß Anspruch 13, dadurch gekennzeichnet, daß man eine Verbindung der Formel II oder deren physiologisch verträgliches Salz und einen physiologisch annehmbaren Träger gegebenenfalls zusammen mit weiteren Zusatz- oder Hilfsstoffen in eine geeignete Darreichungsform bringt.

15. Verwendung einer Verbindung der Formel I,
$X^1$ - $X^2$ (I)
in welcher

$$X^1 \quad \mathrm{R^3\text{-}\underset{O}{\underset{\|}{C}} - \overset{*}{\underset{R^4}{\underset{|}{CH}}} - \underset{R^5}{\underset{|}{N}} - \overset{*}{\underset{O}{\underset{\|}{C}}} - (CHR^1)_m -},$$

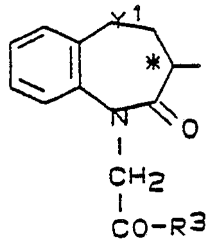 oder 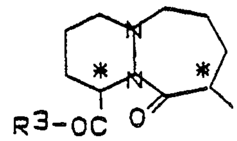 bedeutet,

$X^2$ $-CH_2SH$, 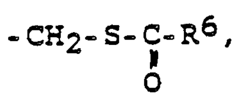, 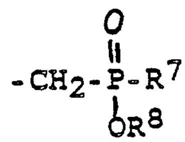 oder

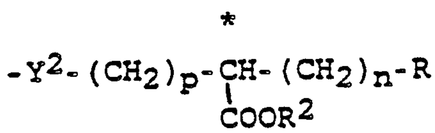 bedeutet,

$Y^1$ für -S- oder $-CH_2$-steht,
$Y^2$ für $-NR^9$- oder $-CH_2$- steht,
m = 0 oder 1 ist,
n = 0, 1 oder 2 ist,
p = 0 oder 1 ist,
R = Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten alicyclischaliphatischen Rest mit 4 - 20 C-Atomen,
einen gegebenenfalls substituierten heteroaromatischen oder heteroaromatisch-($C_1$-$C_8$)-aliphatischen Rest mit 5 - 12 Ringatomen, oder einen Rest $OR^a$ oder $SR^a$ bedeutet, worin
für einen gegebenenfalls substituierten aliphatischen Rest mit 1 - 4 C-Atomen, für einen gegebenenfalls substituierten aromatischen Rest mit 6 - 12 C-Atomen
oder einen gegebenenfalls substituierten heteroaromatischen Rest mit 5 - 12 Ringatomen steht,
$R^1$ Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten alicyclischaliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-($C_1$-$C_8$)-aliphatischen Rest mit 5 - 12 Ringatomen oder, falls nicht von vorstehenden Definitionen schon umfaßt, die erforderlichenfalls geschützte Seitenkette einer natürlich vorkommenden α-Aminosäure bedeutet,
$R^2$ Wasserstoff, einen gegebenenfalls substituierten aliphatischen Rest mit 1 - 21 C-Atomen, einen gegebenenfalls substituierten alicyclischen Rest mit 3 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen Rest der Formel

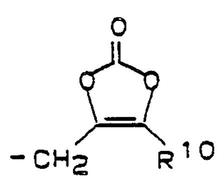

worin $R^{10}$ Wasserstoff, ein aliphatischer Rest mit 1 - 6 C-Atomen oder ein gegebenenfalls substituierter aromatischer Rest mit 6 - 12 C-Atomen ist,
einen Rest der Formel

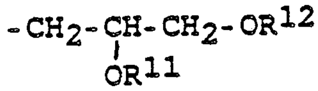

oder

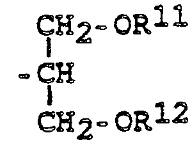

worin $R^{11}$ und $R^{12}$ gleich oder verschieden unabhängig voneinander Wasserstoff, einen gegebenenfalls substituierten Alkylrest mit 1 - 23 C-Atomen oder einen gegebenenfalls substituierten Acylrest mit 1 - 23 C-Atomen bedeuten,
bedeutet,
$R^3$ einen Rest der Formel

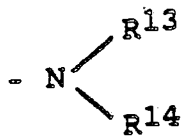

worin $R^{13}$ und $R^{14}$ gleich oder verschieden unabhängig voneinander Wasserstoff, einen gegebenenfalls substituierten aliphatischen Rest mit 1 - 21 C-Atomen, einen gegebenenfalls substituierten alicyclischen Rest mit 3 - 20 C-Atomen, einen gegebenenfalls substituierten aromatischen Rest mit 6 - 12 C-Atomen oder einen gegebenenfalls substituierten araliphatischen Rest mit 7 - 32 C-Atomen bedeuten, oder zusammen mit dem sie tragenden Stickstoffatom einen gegebenenfalls substituierten, gegebenenfalls zugleich teilweise oder vollständig hydrierten, heteroaromatischen Rest mit 2 - 24 C-Atomen bilden, bedeutet,
$R^4$ für Wasserstoff oder $(C_1-C_6)$Alkyl und
$R^5$ für $(C_1-C_6)$Alkyl, $(C_3-C_6)$-Cycloalkyl oder

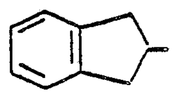

stehen oder
$R^4$ und $R^5$ zusammen mit den sie tragenden Atomen ein mono-, bi-, oder tricyclisches heterocyclisches Ringsystem mit 3 bis 15 Ring-C-Atomen bilden,
$R^6$ Wasserstoff, Amino, $(C_1-C_6)$-Alkyl, $(C_6-C_{12})$-Aryl oder
$(C_7-C_{13})$-Aralkyl,
$R^7$ $(C_1-C_6)$-Alkyl oder $(C_7-C_{13})$-Aralkyl, vorzugsweise $-(CH_2)_4-C_6H_5$,
$R^8$ $(C_1-C_6)$-Alkyl, das gegebenenfalls durch $(C_1-C_6)$-Alkanoyloxy monosubstituiert ist, vorzugsweise 2-Methyl-1-propionyloxy-propyl, und
$R^9$ Wasserstoff oder $(C_1-C_6)$-Alkyl bedeuten;
oder deren physiologisch verträglichen Salzes zur Herstellung eins Arzneimittels mit psychotroper Wirkung.

16. Verwendung einer Verbindung der Formel II,

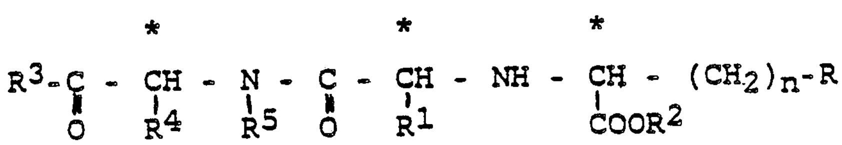

(II)

in welcher
n = 1 oder 2 ist,
R = Wasserstoff
einen gegebenenfalls substituierten aliphatischen Rest mit 1 - 21 C-Atomen,

einen gegebenenfalls substituierten alicyclischen Rest mit 3 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten alicyclischaliphatischen Rest mit 7 - 14 C-Atomen,
einen gegebenenfalls substituierten heteroaromatischen oder heteroaromatisch-($C_1$-$C_8$)-aliphatischen Rest mit 5 - 12 Ringatomen, oder einen Rest $OR^a$ oder $SR^a$ bedeutet, worin
$R^a$ für einen gegebenenfalls substituierten aliphatischen Rest mit 1 - 4 C-Atomen, für einen gegebenenfalls substituierten aromatischen Rest mit 6 - 12 C-Atomen oder einen gegebenenfalls substituierten heteroaromatischen Rest mit 5 - 12 Ringatomen steht,
$R^1$ Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten alicyclischaliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-($C_1$-$C_8$)-aliphatischen Rest mit 5 - 12 Ringatomen oder, falls nicht von vorstehenden Definitionen schon umfaßt, die erforderlichenfalls geschützte Seitenkette einer natürlich vorkommenden α-Aminosäure bedeutet,
$R^2$ Wasserstoff,
einen gegebenenfalls substituierten aliphatischen Rest mit 1 - 21 C-Atomen, einen gegebenenfalls substituierten alicyclischen Rest mit 3 - 20 C-Atomen,
einen gegebenenfalls substituierten aromatischen Rest mit 6- 12 C-Atomen,
einen gegebenenfalls substituierten araliphatischen Rest mit 7 - 32 C-Atomen,
einen Rest der Formel

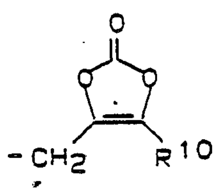

worin $R^{10}$ Wasserstoff, ein aliphatischer Rest mit 1 - 6 C-Atomen oder ein gegebenenfalls substituierter aromatischer Rest mit 6-12 C-Atomen ist,
einen Rest der Formel

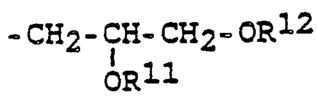 oder 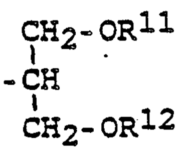

worin $R^{11}$ und $R^{12}$ gleich oder verschieden unabhängig voneinander Wasserstoff, einen gegebenenfalls substituierten Alkylrest mit 1 - 23 C-Atomen oder einen gegebenenfalls substituierten Acylrest mit 1 - 23 C-Atomen bedeuten,
bedeutet,
$R^3$ einen Rest der Formel

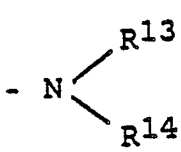

worin $R^{13}$ und $R^{14}$ gleich oder verschieden unabhängig voneinander Wasserstoff, einen gegebenenfalls substituierten aliphatischen Rest mit 1 - 21 C-Atomen, einen gegebenenfalls substituierten alicyclischen Rest mit 3 - 20 C-Atomen, einen gegebenenfalls substituierten aromatischen Rest mit 6 - 12 C-Atomen,

einen gegebenenfalls substituierten araliphatischen Rest mit 7 -32 C-Atomen, oder zusammen mit dem sie tragenden Stickstoffatom einen gegebenenfalls substituierten, gegebenenfalls zugleich teilweise oder vollständig hydrierten, heteroaromatischen Rest mit 2 - 24 C-Atomen bildet, bedeutet, und

$R^4$ und $R^5$ zusammen mit den sie tragenden Atomen ein mono-, bi-, oder tricyclisches heterocyclisches Ringsystem mit 3 bis 15 Ring-C-Atomen bilden,

sowie deren physiologisch verträgliche Salze zur Herstellung eines Arzneimittels mit psychotroper Wirkung.

17. Verwendung gemäß Anspruch 16, dadurch gekennzeichnet, daß Verbindungen der Formel II verwendet werden, in welcher n, R, $R^1$, $R^2$, $R^3$, $R^4$ und $R^5$ wie im Anspruch 1 unter I. a) - e) definiert sind.

18. Verwendung gemäß einem der Ansprüche 16 oder 17, dadurch gekennzeichnet, daß Verbindungen der Formel II verwendet werden, worin $R^4$ und $R^5$ zusammen mit den sie tragenden Atomen ein mono-, bi- oder tricyclisches Ringsystem mit 3 bis 15 Ring-C-Atomen, bis zu 2 Ring-S-Atomen und bis zu 2 Ring-N-Atomen vorzugsweise aus der Reihe Pyrrolidin, Tetrahydroisochinolin, Decahydroisochinolin, Octahydroindol, Indolin, Octahydrocyclopenta[b]pyrrol, 2-Azaspiro[4.5]decan, 2-Azaspiro[4.4]nonan, Spiro[(bicyclo-[2.2.1]-heptan)-2,3′-pyrrolidin], Spiro[(bicyclo-[2.2.2]octan)-2,3′-pyrrolidin], 2-Azatricyclo[4.3.0.1$^{6,9}$]decan, Decahydrocyclohepta[b]pyrrol, Octahydroisoindol, Octahydrocyclopenta[c]pyrrol, 2,3,3a,4,5,7a-Hexahydroindol, 1,2,3,3a,4,6a-Hexahydrocyclopenta[b] pyrrol und 2-Azabicyclo[3.1.0]hexan bilden.

19. Verwendung einer Verbindung der Formel I gemäß Anspruch 15 zur Herstellung eines Arzneimittels zur Behandlung cognitiver Dysfunktionen.

20. Verwendung einer Verbindung der Formel II gemäß Anspruch 16 zur Herstellung eines Arzneimittels zur Behandlung cognitiver Dysfunktionen.

Patentansprüche für folgenden Vertragsstaat: ES

1. Verfahren zur Herstellung einer Verbindung der Formel II

$$R^3-\underset{\underset{O}{\|}}{C}-\underset{\underset{R^4}{|}}{\overset{*}{CH}}-\underset{\underset{R^5}{|}}{N}-\underset{\underset{O}{\|}}{C}-\underset{\underset{R^1}{|}}{\overset{*}{CH}}-NH-\underset{\underset{COOR^2}{|}}{\overset{*}{CH}}-(CH_2)_n-R \qquad (II)$$

in welcher

I. a) n = 1 oder 2 ist

b) R 1. Wasserstoff bedeutet,

2. Alkyl mit 1 - 18 C-Atomen bedeutet,

3. einen aliphatischen acyclischen Rest der Formel $C_aH_{(2a-b+1)}$ bedeutet, worin Doppelbindungen, falls ihre Zahl 1 übersteigt, nicht kumuliert sind, a für eine ganze Zahl 2 bis 18 und b für eine gerade Zahl 2 bis a stehen;

4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (C-2) stehen;

5. Aryl mit 6 - 12 C-Atomen bedeutet, das durch ($C_1$-$C_8$)-Alkyl, ($C_1$-$C_4$)-Alkoxy, Hydroxy, Halogen, Nitro, Amino, Aminomethyl, ($C_1$-$C_4$)-Alkylamino, Di-($C_1$-$C_4$)-alkylamino, ($C_1$-$C_4$)-Alkanoylamino, Methylendioxy, Carboxy, Cyano oder Sulfamoyl, monosubstituiert sein kann;

6. falls n = 2 ist, ($C_6$-$C_{12}$)-Aryl-($C_1$-$C_8$)-alkyl oder Di-:è12)-aryl-($C_1$-$C_8$)-alkyl bedeutet, die im Arylteil jeweils wie unter I. b) 5. beschrieben mono-, di- oder trisubstituiert sein können; oder

7. Alkoxy mit 1 - 4 C-Atomen;

8. Aryloxy mit 6 - 12 C-Atomen,

das wie unter I. b) 6. beschrieben substituiert sein kann;

9. mono- bzw. bicyclisches Heteroaryloxy mit 5 - 7 bzw. 8 - 10 Ringatomen, wovon bis zu 9 Ringatome Kohlenstoff und 1 bis 2 Ringatome Schwefel odeer Sauerstoff und/oder 1 bis 4 Ringatome Stickstoff darstellen, die im Heteroaryl wie unter I. b) 6. beschrieben substituiert sein können.

10. Amino-($C_1$-$C_8$)-alkyl;

11. ($C_1$-$C_4$)-Alkanoylamino-($C_1$-$C_8$)-alkyl;

12. ($C_7$-$C_{13}$)-Aroylamino-($C_1$-$C_8$)-alkyl, das im Arylteil wie unter I. b) 5. monosubstituiert sein kann;

13. ($C_1$-$C_4$)-Alkoxy-carbonylamino-($C_1$-$C_8$)-alkyl;

14. ($C_6$-$C_{12}$)-Aryl-($C_1$-$C_4$)-alkoxycarbonylamino-($C_1$-$C_8$)-alkyl;

15. $(C_6-C_{12})$-Aryl-$(C_1-C_4)$-alkylamino-$(C_1-C_8)$-alkyl;
16. $(C_1-C_4)$-Alkylamino-$(C_1-C_8)$-alkyl;
17. Di-$(C_1-C_4)$-alkylamino-$(C_1-C_8)$-alkyl;
18. Guanidino-$(C_1-C_8)$-alkyl;
19. Imidazolyl;
20. Indolyl;
21. $(C_1-C_4)$-Alkylthio;
22. falls n = 2 ist, $(C_1-C_4)$-Alkylthio-$(C_1-C_8)$-alkyl;
23. $(C_6-C_{12})$-Arylthio-$(C_1-C_8)$-alkyl;
das im Arylteil wie unter I. b) 6. beschrieben, substituiert sein kann,
24. $(C_6-C_{12})$-Aryl-$(C_1-C_8)$-alkylthio, das im Arylteil wie unter I. b) 6. beschrieben substituiert sein kann;
25. falls n = 2 ist, Carboxy-$(C_1-C_8)$-alkyl;
26. Carboxy;
27. Carbamoyl;
28. falls n = 2 ist, Carbamoyl-$(C_1-C_8)$-alkyl;
29. $(C_1-C_4)$-Alkoxy-carbonyl-$(C_1-C_8)$-alkyl;
30. falls n = 2 ist, $(C_6-C_{12})$-Aryloxy-$(C_1-C_8)$-alkyl, das im Arylteil unter I. b) 6. beschrieben substituiert sein kann; oder
31. $(C_6-C_{12})$-Aryl-$(C_1-C_8)$-alkoxy,
das im Arylteil wie unter I. b) 6. beschrieben substituiert sein kann, bedeutet;
c) $R^1$ 1. Wasserstoff bedeutet,
2. Alkyl mit 1 - 18 C-Atomen bedeutet;
3. einen aliphatischen acyclischen Rest der Formel $C_aH_{(2a-b+1)}$ bedeutet, worin Doppelbindungen, falls ihre Zahl 1 übersteigt, nicht kumuliert sind, a für eine ganze Zahl 2 bis 18 und b für eine gerade Zahl 2 bis a stehen;
4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (c-2) stehen; oder
5. Aryl mit 6 - 12 C-Atomen,
das wie unter I. b) 6. beschrieben substituiert sein kann;
6. $(C_6-C_{12})$-Aryl-$(C_1-C_8)$-alkyl oder $(C_7-C_{13})$-Aroyl-$(C_1-C_8)$-alkyl,
die beide im Arylteil wie unter I. b) 6. beschrieben substituiert sein können;
7. mono- bzw. bicyclisches, gegebenenfalls teilhydriertes Heteroaryl oder Heteroaryl-$(C_1-C_8)$-alkyl mit 5 - 7 bzw. 8 - 10 Ringatomen, wovon bis zu 9 Ringatome Kohlenstoff und 1 bis 2 Ringatome Schwefel-oder Sauerstoffatome und/oder 1 bis 4 Ringatome Stickstoffatome darstellen, die im Heteroaryl wie bei Aryl unter I. b) 6. beschrieben substituiert sein können;
8. falls von c) 1. -7. noch nicht umfaßt, die gegebenenfalls geschützte Seitenkette einer natürlich vorkommenden α-Aminosäure der Formel $R^1$-CH($NH_2$)-COOH; oder
9. falls von c) 1. -8. noch nicht umfaßt, die gegebenenfalls geschützte Seitenkette von Cyclohexylalanin, 2-Thienylalanin, 3-Thienylalanin, O-$(C_3-C_5)$-Alkyl-Tyrosin oder C-Phenylglycin bedeutet.
d) $R^2$
1. Wasserstoff bedeutet;
2. Alkyl mit 1 - 18 C-Atomen bedeutet;
3. einen aliphatischen acyclischen Rest der Formel $C_aH_{(2a-b+1)}$ bedeutet, worin Doppelbindungen, falls ihre Zahl 1 übersteigt, nicht kumuliert sind, a für eine ganze Zahl 2 bis 18 und b für eine gerade Zahl 2 bis a stehen;
4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (c-2) stehen; oder
5. Di-$(C_1-C_4)$-alkylamino-$(C_1-C_8)$-alkyl;
6. $(C_1-C_5)$-Alkanoyloxy-$(C_1-C_8)$-alkyl;
7. $(C_1-C_6)$-Alkoxy-carbonyloxy-$(C_1-C_8)$-alkyl;
8. $(C_7-C_{13})$-Aroyloxy-$(C_1-C_8)$-alkyl;
9. $(C_6-C_{12})$-Aryloxycarbonyloxy$(C_1-C_8)$-alkyl;
10. Aryl mit 6 - 12 C-Atomen;
11. $(C_7-C_{20})$-Aralkyl;
12. Phthalidyl;
13. einen Rest der Formel

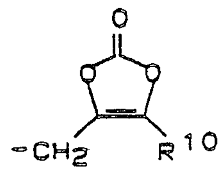

worin $R^{10}$ Wasserstoff, $(C_1$-$C_6)$-Alkyl oder Aryl mit 6 - 12 C-Atomen ist; oder
14. einen Rest der Formel

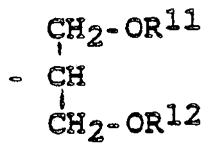

bedeutet,
worin $R^{11}$ und $R^{12}$ gleich oder verschieden unabhängig voneinander Wasserstoff, einen gegebenenfalls substituierten Alkylrest mit 1 - 23 C-Atomen oder einen gegebenenfalls substituierten Acylrest mit 1 - 23 C-Atomen bedeuten
wobei die unter d) 8., 9., 10., 11. und 12. genannten Reste wie unter I. b) 6. beschrieben im Arylteil substituiert sein können;
e) $R^3$ einen Rest der Formel

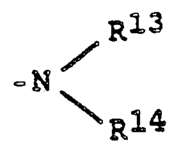

bedeutet,
worin $R^{13}$ und $R^{14}$ gleich oder verschieden unabhängig voneinander
1. Wasserstoff bedeuten;
2. Alkyl mit 1 - 21 C-Atomen bedeuten;
3. einen aliphatischen acyclischen Rest der Formel $C_aH_{(2a-b+1)}$ bedeuten, worin a für eine ganze Zahl 2 bis 21 und b für eine gerade Zahl 2 bis a stehen;
4. einen mono-, di-, tri-, tetra- oder pentacyclischen, nicht aromatischen Kohlenwasserstoffrest der Formel $C_cH_{(2c-d-1)}$ bedeuten, der gegebenenfalls verzweigt ist, worin c für eine ganze Zahl 3 - 20 und d für eine gerade Zahl 0 bis (c-2) stehen; oder
5. Amino-$(C_1$-$C_{16})$-alkyl;
6. Mono-$(C_1$-$C_4)$-alkylamino-$(C_1$-$C_{16})$alkyl;
7. Di-$(C_1$-$C_4)$-alkylamino-$(C_1$-$C_{13})$alkyl;
8. $(C_1$-$C_5)$-Alkanoyloxy-$(C_1$-$C_{16})$-alkyl;
9. $(C_1$-$C_6)$-Alkoxy-carbonyloxy-$(C_1$-$C_{15})$-alkyl;
10. $(C_7$-$C_{13})$-Aroyloxy-$(C_1$-$C_8)$-alkyl;
11. $(C_6$-$C_{12})$-Aryloxy-carbonyloxy-$(C_1$-$C_9)$alkyl;
12. Aryl mit 6 - 12 C-Atomen, oder
13. $(C_7$-$C_{20})$-Aralkyl bedeuten
oder zusammen mit dem sie tragenden Stickstoffatom einen gegebenenfalls substituierten, gegebenenfalls teilweise oder vollständig hydrierten heteroaromatischen Rest mit 2 - 24 C-Atomen bilden und
f) $R^4$ und $R^5$ zusammen mit den sie tragenden Atomen ein mono-, bi- oder tricyclisches heterocyclisches Ringsystem mit 3 bis 15 Ring C-Atomen bilden
oder deren physiologisch verträgliche Salze mit Säuren oder Basen,
II. <u>ausgenommen</u> Verbindungen der Formel II und deren Salze, worin
a) n = 1 oder 2 ist;
b) R 1. Wasserstoff;
2. Alkyl mit 1 - 8 C-Atomen;

3. Alkenyl mit 2 - 8 C-Atomen;
4. Cycloalkyl mit 3 - 9 C-Atomen;
5. Aryl mit 6 - 12 C-Atomen,
das durch ($C_1$-$C_4$)-Alkyl, ($C_1$-$C_4$)-Alkoxy, Hydroxy, Halogen, Nitro, Amino, Aminomethyl, ($C_1$-$C_4$)Alkylamino, Di-($C_1$-$C_4$)-alkylamino, ($C_1$-$C_4$)Alkanoylamino, Methylendioxy, Carboxy, Cyano und/oder Sulfamoyl mono-, di- oder trisubstituiert sein kann;
6. Alkoxy mit 1 - 4 C-Atomen;
7. Aryloxy mit 6 - 12 C-Atomen,
das wie unter II. b) 5. beschrieben substituiert sein kann;
8. mono- bzw. bicyclisches Heteroaryloxy mit 5-7 bzw. 8 - 10 Ringatomen, wovon 1 bis 2 Ringatome Schwefel- oder Sauerstoffatome und/oder 1 bis 4 Ringatome Stickstoff darstellen, das wie unter II. b) 5. beschrieben substituiert sein kann;
9. Amino-($C_1$-$C_4$)-alkyl;
10. ($C_1$-$C_4$)-Alkanoylamino-($C_1$-$C_4$)-alkyl;
11. ($C_7$-$C_{13}$)-Aroylamino-($C_1$-$C_4$)-alkyl;
12. ($C_1$-$C_4$)-Alkoxy-carbonylamino-($C_1$-$C_4$)-alkyl;
13. ($C_6$-$C_{12}$)-Aryl-($C_1$-$C_4$)-alkoxycarbonylamino-($C_1$-$C_4$)-alkyl;
14. ($C_6$-$C_{12}$)-Aryl-($C_1$-$C_4$)-alkylamino-($C_1$-$C_4$)-alkyl;
15. ($C_1$-$C_4$)-Alkylamino-($C_1$-$C_4$)-alkyl;
16. Di-($C_1$-$C_4$)-alkylamino-($C_1$-$C_4$)-alkyl;
17. Guanidino-($C_1$-$C_4$)-alkyl;
18. Imidazolyl;
19. Indolyl;
20. ($C_1$-$C_4$)-Alkylthio;
21. ($C_1$-$C_4$)-Alkylthio-($C_1$-$C_4$)-alkyl;
22. ($C_6$-$C_{12}$)-Arylthio-($C_1$-$C_4$)-alkyl,
das im Arylteil wie unter II. b) 5. beschrieben substituiert sein kann;
23. ($C_6$-$C_{12}$)-Aryl-($C_1$-$C_4$)-alkylthio,
das im Arylteil wie unter II. b) 5. beschrieben substituiert sein kann;
24. Carboxy-($C_1$-$C_4$)-alkyl;
25. Carboxy;
26. Carbamoyl;
27. Carbamoyl-($C_1$-$C_4$)-alkyl;
28. ($C_1$-$C_4$)-Alkoxy-carbonyl-($C_1$-$C_4$)-alkyl;
29. ($C_6$-$C_{12}$)-Aryloxy-($C_1$-$C_4$)-alkyl,
das im Arylteil wie unter II. b) 5. beschrieben substituiert sein kann; oder
30. ($C_6$-$C_{12}$)-Aryl-($C_1$-$C_4$)-alkoxy,
das im Arylteil wie unter II. b) 5. beschrieben substituiert sein kann, bedeutet;
c) $R^1$ 1. Wasserstoff;
2. Alkyl mit 1 - 6 C-Atomen;
3. Alkenyl mit 2 - 6 C-Atomen;
4. Alkinyl mit 2 - 6 C-Atomen;
5. Cycloalkyl mit 3 - 9 C-Atomen;
6. Cycloalkenyl mit 5 - 9 C-Atomen;
7. ($C_3$-$C_9$)-Cycloalkyl-($C_1$-$C_4$)-alkyl;
8. ($C_5$-$C_9$)-Cycloalkenyl-($C_1$-$C_4$)-alkyl;
9. gegebenenfalls teilhydriertes Aryl mit 6 - 12 C-Atomen, das wie II. b) 5. beschrieben substituiert sein kann;
10. ($C_6$-$C_{12}$)-Aryl-($C_1$-$C_4$)-alkyl oder ($C_7$-$C_{13}$)-Aroyl-($C_1$ oder $C_2$)-alkyl,
die beide wie im Arylteil wie unter II. b) 5. beschrieben substituiert sein können;
11. mono- bzw. bicyclisches, gegebenenfalls teilhydriertes Heteroaryl mit 5 - 7 bzw. 8 - 10 Ringatomen, wovon 1 bis 2 Ringatome Schwefel- oder Sauerstoffatome und/oder 1 bis 4 Ringatome Stickstoffatome darstellen, das wie unter II. b) 5. beschrieben substituiert sein kann; oder
12. falls von den vorstehenden Definitionen nicht umfaßt, die gegebenenfalls geschützte Seitenkette einer natürlich vorkommenden α-Aminosäure der Formel $R^1$-$CH(NH_2)$-COOH bedeutet;
d) $R^2$
1. Wasserstoff;
2. Alkyl mit 1 - 6 C-Atomen;

3. Alkenyl mit 2 - 6 C-Atomen;
4. Di-($C_1$-$C_4$)-alkylamino-($C_1$-$C_4$)-alkyl;
5. ($C_1$-$C_5$)-Alkanoyloxy-($C_1$-$C_4$)-alkyl;
6. ($C_1$-$C_6$)-Alkoxy-carbonyloxy-($C_1$-$C_4$)-alkyl;
7. ($C_7$-$C_{13}$)-Aroyloxy-($C_1$-$C_4$)-alkyl;
8. ($C_6$-$C_{12}$)-Aryloxycarbonyloxy($C_1$-$C_4$)-alkyl;
9. Aryl mit 6 - 12 C-Atomen;
10. ($C_6$-$C_{12}$)-Aryl-($C_1$-$C_4$)-alkyl;
11. ($C_3$-$C_9$)-Cycloalkyl; oder
12. ($C_3$-$C_9$)-Cycloalkyl-($C_1$-$C_4$)-alkyl bedeutet ,

e) $R^3$ einen Rest der Formel

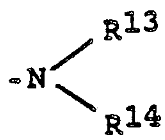

bedeutet,
worin $R^{13}$ und $R^{14}$ gleich oder verschieden unabhängig voneinander
1. Wasserstoff;
2. Alkyl mit 1 - 6 C-Atomen; oder
3. ($C_6$-$C_{12}$)-Aryl-($C_1$-$C_4$)-alkyl

bedeuten und

f) $R^4$ und $R^5$ zusammen mit den sie tragenden Atomen ein mono-, bi- oder tricyclisches heterocyclisches Ringsystem mit 3 bis 15 Ring-C-Atomen bilden, dadurch gekennzeichnet, daß man ihre Fragmente in einem geeigneten Lösungsmittel gegebenenfalls in Gegenwart einer Base und/oder eines Kupplungshilfsmittels miteinander umsetzt, gegebenenfalls intermediär entstehende ungesättigte Verbindungen, wie Schiffsche Basen, reduziert, zum Schutz reaktiver Gruppen temporär eingeführte Schutzgruppen abspaltet, Verbindungen der Formel II mit freier(en) Carboxylgruppe(n) gegebenenfalls verestert oder in Amide überführt und die erhaltenen Verbindungen gegebenenfalls in ihre physiologisch verträglichen Salze überführt.

2. Verfahren gemäß Anspruch 1, dadurch gekennzeichnet, daß man eine Verbindung der Formel II herstellt, in welcher
n = 1 oder 2 ist
R = Wasserstoff,
Alkyl mit 1 - 8 C-Atomen,
Alkenyl mit 2 -6 C-Atomen,
Cycloalkyl mit 3 - 9 C-Atomen,
Aryl mit 6 - 12 C-Atomen,
das durch ($C_1$-$C_4$)-Alkyl, ($C_1$-$C_4$)-Alkoxy, Hydroxy, Halogen, Nitro, Amino, Aminomethyl, ($C_1$-$C_4$)-Alkylamino, Di-($C_1$-$C_4$)-alkylamino, ($C_1$-$C_4$)-Alkanoylamino, Methylendioxy, Carboxy, Cyano oder Sulfamoyl, monosubstituiert sein kann,
Alkoxy mit 1 - 4 C-Atomen,
Aryloxy mit 6 - 12 C-Atomen,
das wie oben bei Aryl beschrieben mono-, di- oder trisubstituiert sein kann,
mono- bzw. bicyclisches Heteroaryloxy mit 5 - 7 bzw. 8 - 10 Ringatomen, wovon 1 bis 2 Ringatome Schwefel-oder Sauerstoffatome und/oder 1 bis 4 Ringatome Stickstoff darstellen, das wie oben bei Aryloxy beschrieben substituiert sein kann,
Amino-($C_1$-$C_4$)-alkyl,
($C_1$-$C_4$)-Alkanoylamino-($C_1$-$C_4$)alkyl,
($C_7$-$C_{13}$)-Aroylamino-($C_1$-$C_4$)-alkyl, das wie oben bei Aryl beschrieben substituiert sein kann,
($C_1$-$C_4$)-Alkoxy-carbonylamino-($C_1$-$C_4$)-alkyl,
($C_6$-$C_{12}$)-Aryl-($C_1$-$C_4$)-alkoxycarbonylamino-($C_1$-$C_4$)-alkyl,
($C_6$-$C_{12}$)-Aryl-($C_1$-$C_4$)-alkylamino-($C_1$-$C_4$)-alkyl,
($C_1$-$C_4$)-Alkylamino-($C_1$-$C_4$)-alkyl,
Di-($C_1$-$C_4$)-alkylamino-($C_1$-$C_4$)-alkyl,
Guanidino-($C_1$-$C_4$)-alkyl,

Imidazolyl, Indolyl,
$(C_1-C_4)$-Alkylthio,
$(C_1-C_4)$-Alkylthio-$(C_1-C_4)$-alkyl,
$(C_6-C_{12})$-Arylthio-$(C_1-C_4$-alkyl,
das im Arylteil wie oben bei Aryloxy beschrieben, substituiert sein kann,
$(C_6-C_{12})$-Aryl-$(C_1-C_4)$-alkylthio,
das im Arylteil wie oben bei Aryloxy beschrieben substituiert sein kann,
Carboxy-$(C_1-C_4)$-alkyl,
Carboxy, Carbamoyl,
Carbamoyl-$(C_1-C_4)$-alkyl,
$(C_1-C_4)$-Alkoxy-carbonyl-$(C_1-C_4)$-alkyl,
$(C_6-C_{12})$-Aryloxy-$(C_1-C_4)$-alkyl,
das im Arylteil wie oben bei Aryloxy beschrieben substituiert sein kann oder
$(C_6-C_{12})$-Aryl-$(C_1-C_4)$-alkoxy,
das im Arylteil wie oben bei Aryloxy beschrieben substituiert sein kann,
$R^1$ Wasserstoff,
Alkyl mit 1 - 6 C-Atomen,
Alkenyl mit 2 - 6 C-Atomen;
Alkinyl mit 2 - 6 C-Atomen,
Cycloalkyl mit 3 - 9 C-Atomen,
Cycloalkenyl mit 5 - 9 C-Atomen,
$(C_3-C_9)$-Cycloalkyl-$(C_1-C_4)$-alkyl,
$(C_5-C_9)$-Cycloalkenyl-$(C_1-C_4)$-alkyl,
gegebenenfalls teilhydriertes Aryl mit 6 -12 C-Atomen, das wie oben bei R beschrieben mono-, bi-oder trisubstituiert sein kann, $(C_6-C_{12})$-Aryl-$(C_1-C_4)$-alkyl oder $(C_7-C_{13})$-Aroyl-$(C_1$ oder $C_2)$-alkyl
die beide wie das vorstehende Aryl substituiert sein können,
mono- bzw. bicyclisches, gegebenenfalls teilhydriertes Heteroaryl mit 5 - 7 bzw. 8 - 10 Ringatomen, wovon 1 bis 2 Ringatome Schwefel- oder Sauerstoffatome und/oder 1 bis 4 Ringatome Stickstoffatome darstellen, das wie das vorstehende Aryl substituiert sein kann oder
die gegebenenfalls geschützte Seitenkette einer natürlich vorkommenden $\alpha$-Aminosäure $R^1-CH(NH_2)-COOH$ bedeutet,
$R^2$ Wasserstoff,
Alkyl mit 1 - 6 C-Atomen,
Alkenyl mit 2 - 6 C-Atomen,
Di-$(C_1-C_4)$-alkylamino-$(C_1-C_4)$-alkyl,
$(C_1-C_5)$-Alkanoyloxy-$(C_1-C_4)$-alkyl,
$(C_1-C_6)$-Alkoxy-carbonyloxy-$(C_1-C_4)$-alkyl,
$(C_7-C_{13})$-Aroyloxy-$(C_1-C_4)$-alkyl,
$(C_6-C_{12})$-Aryloxycarbonyloxy$(C_1-C_4)$-alkyl,
Aryl mit 6 - 12 C-Atomen,
$(C_6-C_{12})$-Aryl-$(C_1-C_4)$-alkyl,
$(C_3-C_9)$-Cycloalkyl oder
$(C_3-C_9)$-Cycloalkyl-$(C_1-C_4)$-alkyl
bedeutet,
$R^3$ einen Rest der Formel

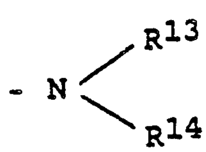

bedeutet,
worin $R^{13}$ und $R^{14}$ gleich oder verschieden unabhängig voneinander
Wasserstoff,
Alkyl mit 1 - 16 C-Atomen,
Alkenyl mit 2 - 16 C-Atomen,
Alkinyl mit 2 - 16 C-Atomen,

Cycloalkyl mit 3 - 9 C-Atomen,
$(C_3-C_9)$-Cycloalkyl-$(C_1-C_4)$-alkyl,
Amino-$(C_1-C_{16})$-alkyl,
Mono-$(C_1-C_4)$-alkylamino-$(C_1-C_{12})$alkyl,
Di-$(C_1-C_4)$-alkylamino-$(C_1-C_8)$-alkyl,
$(C_1-C_5)$-Alkanoyloxy-$(C_1-C_{11})$-alkyl,
$(C_1-C_6)$-Alkoxycarbonyloxy-$(C_1-C_{10})$-alkyl,
$(C_7-C_{13})$-Aroyloxy-$(C_1-C_6)$-alkyl,
$(C_6-C_{12})$-Aryloxycarbonyloxy$(C_1-C_6)$-alkyl,
Aryl mit 6 - 12 C-Atomen oder
$(C_6-C_{12})$-Aryl-$(C_1-C_6)$-alkyl bedeuten
oder zusammen mit dem sie tragenden Stickstoffatom, gegebenenfalls durch Amino, $(C_1-C_4)$-Alkylamino-, Di-$(C_1-C_4)$-alkylamino, $(C_1-C_5)$-Alkanoyloxy-, $(C_1-C_6)$-Alkoxycarbonyloxy-, $(C_7-C_{13})$-Aroyloxy- oder $(C_6-C_{12})$-Aryloxycarbonyloxy substituiertes
Pyrrolidinyl,
Piperidinyl,
Piperazinyl,
Homopiperazinyl,
Morpholinyl,
Thiomorpholinyl,
Imidazolyl,
Pyridyl,
Pyrimidinyl,
sym.-Triazinyl bedeuten
und
f) $R^4$ und $R^5$ zusammen mit den sie tragenden Atomen ein mono-, bi- oder tricyclisches heterocyclisches Ringsystem mit 3 bis 15 Ring-C-Atomen bilden oder deren physiologisch verträgliche Salze mit Säuren oder Basen.

3. Verfahren gemäß Anspruch 1 oder 2, dadurch gekennzeichnet, daß man eine Verbindung der Formel II herstellt, in welcher
n = 1 oder 2 ist,
R $(C_1-C_6)$-Alkyl, $(C_2-C_6)$-Alkenyl, $(C_3-C_9)$-Cycloalkyl, Amino-$(C_1-C_4)$-alkyl, $(C_2-C_5)$-Acylamino-$(C_1-C_4)$-alkyl, $(C_7-C_{13})$-Aroylamino-$(C_1-C_4)$-alkyl, $(C_1-C_4)$-Alkoxycarbonylamino-$(C_1-C_4)$-alkyl, $(C_6-C_{12})$-Aryl-$(C_1-C_4)$-alkoxycarbonylamino-$(C_1-C_4)$-alkyl, $(C_6-C_{12})$-Aryl, das durch $(C_1-C_4)$-Alkyl, $(C_1-C_4)$-Alkoxy, Hydroxy, Halogen, Nitro, Amino, $(C_1-C_4)$-Alkylamino, Di-$(C_1-C_4)$-alkylamino oder Methylendioxy monosubstituiert sein kann, oder 3-Indolyl, insbesondere Methyl, Ethyl, Cyclohexyl, tert. Butoxycarbonylamino-$(C_1-C_4)$-alkyl, Benzoyloxycarbonylamino-$(C_1-C_4)$-alkyl oder Phenyl, das durch Phenyl, $(C_1-C_2)$-Alkyl, ($C_1$ oder $C_2$)-Alkoxy, Hydroxy, Fluor, Chlor, Brom, Amino, $(C_1-C_4)$-Alkylamino, Di-$(C_1-C_4)$alkylamino oder Nitro monosubstituiert sein kann, bedeutet,
$R^1$ Wasserstoff oder $(C_1-C_6)$-Alkyl, das gegebenenfalls durch Amino, $(C_1-C_6)$-Acylamino oder Benzoylamino monosubstituiert sein kann, $(C_2-C_6)$-Alkenyl, $(C_3-C_9)$-Cycloalkyl, $(C_5-C_9)$-Cycloalkenyl, $(C_3-C_7)$-Cycloalkyl-$(C_1-C_4)$-alkyl, $(C_6-C_{12})$-Aryl oder teilhydriertes Aryl, das jeweils durch $(C_1-C_4)$-Alkyl, ($C_1$ oder $C_2$)-Alkoxy oder Halogen substituiert sein kann, $(C_6-C_{12})$-Aryl-($C_1$ bis $C_4$)-alkyl oder $(C_7-C_{13})$-Aroyl-$(C_1-C_2)$-alkyl, die beide wie vorstehend definiert im Arylrest substituiert sein können, ein mono- bzw. bicyclischer Heterocyclen- Rest mit 5 bis 7 bzw. 8 bis 10 Ringatomen, wovon 1 bis 2 Ringatome Schwefel- oder Sauerstoffatome und/oder 1 bis 4 Ringatome Stickstoffatome darstellen, oder eine Seitenkette einer natürlich vorkommenden, gegebenenfalls geschützten α-Aminosäure, insbesondere aber Wasserstoff, $(C_1-C_3)$-Alkyl, ($C_2$ oder $C_3$)-Alkenyl, die gegebenenfalls geschützte Seitenkette von Lysin, Benzyl, 4-Methoxybenzyl, 4-Ethoxybenzyl, Phenethyl, 4-Amino-butyl oder Benzoylmethyl bedeutet,
$R^2$ Wasserstoff,
$(C_1-C_6)$-Alkyl, $(C_2-C_6)$-Alkenyl oder $(C_6-C_{12})$-Aryl-$(C_1-C_4)$-alkyl, insbesondere aber Wasserstoff, $(C_1-C_4)$-Alkyl oder Benzyl bedeutet,
$R^3$ einen Rest der Formel

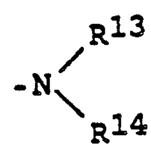

bedeutet,
worin $R^{13}$ und $R^{14}$ gleich oder verschieden unabhängig voneinander Wasserstoff, $(C_4\text{-}C_{12})$-Alkyl, $(C_4\text{-}C_{12})$-Alkenyl, $(C_5\text{-}C_7)$-Cycloalkyl, $(C_5\text{-}C_7)$-Cycloalkyl-$(C_1\text{-}C_4)$-alkyl, Amino-$(C_4\text{-}C_{12})$-alkyl, $(C_1\text{-}C_4)$-Alkylamino-$(C_4\text{-}C_{12})$-alkyl, Di-$(C_1\text{-}C_4)$-Alkylamino-$(C_1\text{-}C_8)$-alkyl, $(C_6\text{-}C_{12})$-Aryl-$(C_1\text{-}C_6)$-alkyl, bedeuten oder zusammen mit dem sie tragenden Stickstoffatom gegebenenfalls durch Amino, $(C_1\text{-}C_4)$-Alkylamino oder Di-$(C_1\text{-}C_4)$-alkylamino substituiertes Pyrrolidinyl, Piperidinyl, Piperazinyl, Homopiperazinyl, Morpholinyl oder Thiomorpholinyl bedeuten,
und
$R^4$ und $R^5$ zusammen mit den sie tragenden Atomen ein mono-, bi- oder tricyclisches Ringsystem mit 3 bis 15 Ring-C-Atomen bilden
oder deren physiologisch verträgliche Salze mit Säuren und Basen.

4. Verfahren gemäß einem der Ansprüche 1 bis 3, dadurch gekennzeichnet, daß man eine Verbindung der Formel II herstellt, in welcher $R^4$ und $R^5$ zusammen mit den diese tragenden Atomen ein mono-, bi- oder tricyclisches heterocyclisches Ringsystem mit 3 bis 15 Ring-C-Atomen, bis zu 2 Ring-S-Atomen und bis zu 2 Ring-N-Atomen vorzugsweise aus der Reihe Pyrrolidin, Thiazolidin, Tetrahydroisochinolin, Decahydroisochinolin, Octahydroindol, Indolin, Octahydrocyclopenta[b]pyrrol, 2-Azaspiro[4.5]decan, 2-Azaspiro-[4.4]-nonan, Spiro[(bicyclo[2.2.1]heptan)-2,3′-pyrrolidin], Spiro[(bicyclo[2.2.2]octan)-2,3′-pyrrolidin],2-Azatricyclo[$4.3.0.1^{6,9}$]decan, Decahydrocyclohepta[b]pyrrol, Octahydroisoindol, Octahydrocyclopenta[c]-pyrrol, 2,3,3a,4,5,7a-Hexahydroindol und 1,2,3,3a,4,6a-Hexahydrocyclopenta[b]pyrrol bilden, oder deren physiologisch verträglichen Salze.

5. Verfahren gemäß Anspruch 1, dadurch gekennzeichnet, daß man
a) eine Verbindung der Formel III

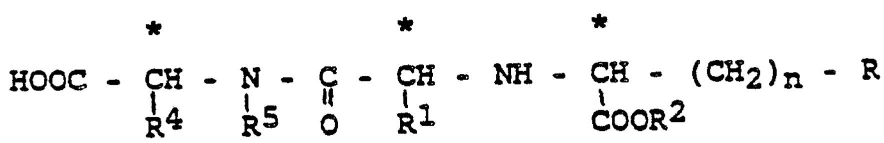
(III)

worin R, $R^1$, $R^2$, $R^4$, $R^5$ und n die gleiche Bedeutung wie in Formel II haben, mit einem Amin der Formel IV

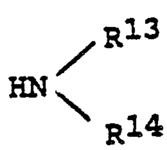
(IV)

worin $R^{13}$ und $R^{14}$ die gleiche Bedeutung wie in Formel II haben, umsetzt,
oder daß man
b) eine Verbindung der Formel V

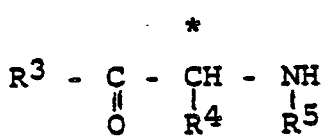
(V)

worin $R^3$, $R^4$ und $R^5$ die gleiche Bedeutung wie in Formel II haben, mit einer Verbindung der Formel VI

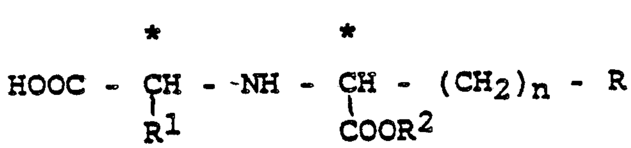
(VI)

worin R, $R^1$, $R^2$ und n die gleiche Bedeutung wie in Formel II haben, umsetzt,
oder daß man
c) eine Verbindung der Formel VII

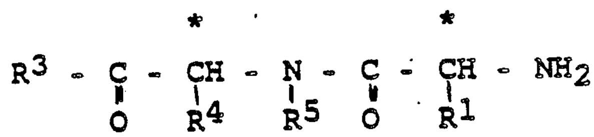

(VII)

worin $R^1$, $R^3$, $R^4$ und $R^5$ die gleiche Bedeutung wie in Formel II haben, mit einer Verbindung der Formel VIII

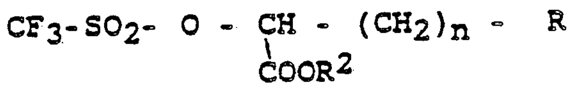

(VIII)

worin R, $R^2$ und n die gleiche Bedeutung wie in Formel II haben, umsetzt,
oder daß man
d) eine Verbindung der Formel IX,

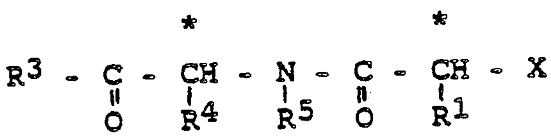

(IX)

worin $R^1$, $R^3$, $R^4$ und $R^5$ die gleiche Bedeutung wie in Formel II haben und
in der X eine Abgangsgruppe, die nucleophil verdrängt werden kann, insbesondere ein Cl, Br, J-Atom oder einen Sulfonsäurerest, bedeutet, mit einer Verbindung der Formel X

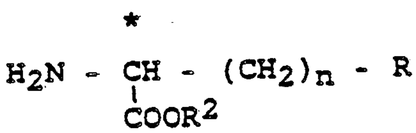

(X)

in der R, $R^2$ und n die gleiche Bedeutung wie in Formel II haben, unter Bedingungen einer nucleophilen Substitution, umsetzt,
oder daß man
e) eine Verbindung der Formel XI

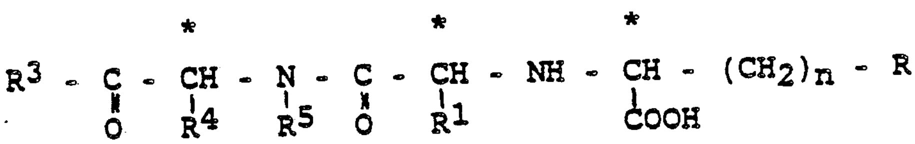

(XI)

in der R, $R^1$, $R^3$, $R^4$, $R^5$ und n die gleiche Bedeutung wie in Formel II haben, unter Anwendung einer der gebräuchlichen Veresterungsmethoden in Verbindungen der Formel II überführt, beispielsweise durch Umsetzung mit einem Alkohol $R^2$-OH unter saurer Katalyse oder nach Aktivierung der Carbonsäurefunktion von XI oder nach Aktivierung der Hydroxyfunktion von $R^2OH$, z.B. unter den Bedingungen einer Mitsunobu-Reaktion,
oder durch Umsetzung mit einer Verbindung der Formel $R^2$ X, worin $R^2$ die gleiche Bedeutung wie in Formel II und X die gleiche Bedeutung wie in Formel IX hat unter den Bedingungen einer nucleophilen Substitution, wie sie unter der Verfahrensvariante d) beschreiben sind,
oder durch Umsetzung mit einem Diazoalkan in einem inerten organischen Lösungsmittel,
oder daß man
f) eine Verbindung der Formel XII
$R^2OOC-CH=CH-CO-R$ (XII),
in welcher R und $R^2$ die gleiche Bedeutung wie in Formel II haben, mit einer Verbindung der Formel VII umsetzt und die Carbonylgruppe hydriert,
oder daß man

g) eine Verbindung der Formel VII mit einer Verbindung der Formel XIII

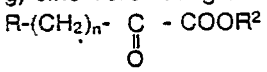 (XIII)

worin R, $R^2$ und n die gleiche Bedeutung wie in Formel II haben, umsetzt und die erhaltenen Schiff-Basen reduziert,

und die so erhaltenen Verbindungen der Formel II gegebenenfalls in ihre physiologisch verträglichen Salze überführt.

6. Verfahren zur Herstellung einer Verbindung der Formel V

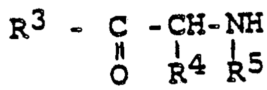 (V),

worin

$R^3$ einen Rest der Formel

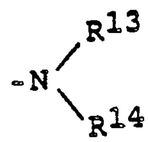

bedeutet,

worin $R^{13}$ und $R^{14}$ gleich oder verschieden unabhängig voneinander

Wasserstoff

Alkyl mit 7 - 16 C-Atomen,

Alkenyl mit 7 - 16 C-Atomen,

Alkinyl mit 7 - 16 C-Atomen

Cycloalkyl mit 3 - 9 C-Atomen,

$(C_3-C_9)$-Cycloalkyl-$(C_1-C_4)$-alkyl,

Amino-$(C_1-C_{16})$-alkyl,

Mono-$(C_1-C_4)$-alkylamino-$(C_1-C_{12})$alkyl,

Di-$(C_1-C_4)$-alkylamino-$(C_1-C_8)$alkyl,

$(C_1-C_5)$-Alkanoyloxy-$(C_1-C_{11})$-alkyl,

$(C_1-C_6)$-Alkoxycarbonyloxy-$(C_1-C_{10})$-alkyl,

$(C_7-C_{13})$-Aroyloxy-$(C_1-C_6)$-alkyl,

$(C_6-C_{12})$-Aryloxycarbonyloxy-$(C_1-C_6)$-alkyl,

Aryl mit 6 - 12 C-Atomen oder

$(C_6-C_{12})$-Aryl-$(C_5-C_6)$-alkyl bedeuten

oder zusammen mit dem sie tragenden Stickstoffatom, gegebenenfalls durch Amino, $(C_1-C_4)$-Alkylamino, Di-$(C_1-C_4)$-alkylamino, $(C_1-C_5)$-Alkanoyloxy, $(C_1-C_6)$-Alkoxycarbonyloxy, $(C_7-C_{13})$-Aroyloxy oder $(C_6-C_{12})$-Aryloxycarbonyloxy substituiertes

Pyrrolidinyl,

Piperidinyl,

Piperazinyl,

Homopiperazinyl,

Morpholinyl,

Thiomorpholinyl,

Imidazolyl,

Pyridyl,

Pyrimidinyl,

sym.-Triazinyl bedeuten

und

$R^4$ und $R^5$ zusammen mit den sie tragenden Atomen ein mono-, bi- oder tricyclisches heterocyclisches Ringsystem mit 3 bis 15 Ring-C-Atomen bilden, dadurch gekennzeichnet, daß man in Verbindungen der Formel XIV,

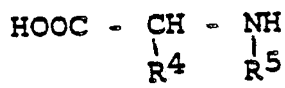 (XIV),

worin $R^4$ und $R^5$ die gleiche Bedeutung wie in Formel V haben, die Aminogruppe schützt, dann mit einem Amin der Formel IV

$R^{13}$ - NH - $R^{14}$ (IV),

worin $R^{13}$ und $R^{14}$ die gleiche Bedeutung wie in Formel V haben, umsetzt und die Amino-Schutzgruppe wieder abspaltet.

7. Verfahren zur Hestellung eines pharmazeutischen Mittels enthaltend eine Verbindung gemäß einem oder mehreren der Ansprüche 1 bis 4, dadurch gekennzeichnet, daß man eine Verbindung der Formel II oder deren physiologisch verträgliches Salz und einen physiologisch annehmbaren Träger gegebenenfalls zusammen mit weiteren Zusatz-oder Hilfsstoffen in eine geeignete Darreichungsform bringt.

8. Verwendung einer Verbindung der Formel I,

$X^1 - X^2$ (I)

in welcher

$X^1$ 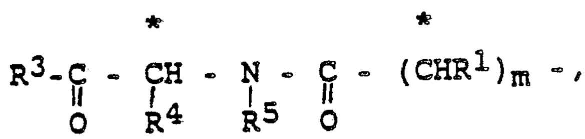

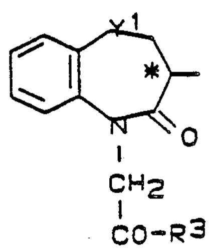 oder 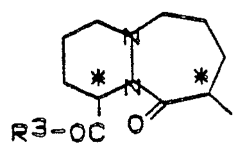 bedeutet,

$X^2$ $-CH_2SH$, 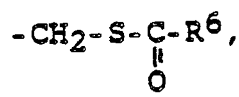 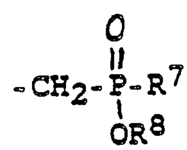 oder

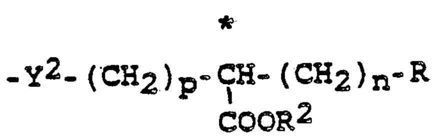 bedeutet,

$Y^1$ für -S- oder $-CH_2-$ steht,

$Y^2$ für $-NR^9-$ oder $-CH_2-$ steht,

m = 0 oder 1 ist,

n = 0, 1 oder 2 ist,

p = 0 oder 1 ist,

R = Wasserstoff,

einen gegebenenfalls substituierten aliphatischen Rest mit 1 - 21 C-Atomen,

einen gegebenenfalls substituierten alicyclischen Rest mit 3 - 20 C-Atomen,

einen gegebenenfalls substituierten aromatischen Rest mit 6 - 12 C-Atomen,

einen gegebenenfalls substituierten araliphatischen Rest mit 7 - 32 C-Atomen,

einen gegebenenfalls substituierten alicyclischaliphatischen Rest mit 4 - 20 C-Atomen,

einen gegebenenfalls substituierten heteroaromatischen oder heteroaromatisch-($C_1$-$C_8$)-aliphatischen Rest mit 5 - 12 Ringatomen, oder einen Rest $OR^a$ oder $SR^a$ bedeutet, worin

$R^a$ für einen gegebenenfalls substituierten aliphatischen Rest mit 1 - 4 C-Atomen, für einen gegebenenfalls substituierten aromatischen Rest mit 6 - 12 C-Atomen

oder einen gegebenenfalls substituierten heteroaromatischen Rest mit 5 - 12 Ringatomen steht,
$R^1$ Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten alicyclischaliphatischen Rest mit 4- 20 C-Atomen,
einen gegebenenfalls substituierten aromatischen Rest mit 6 -12 C-Atomen,
einen gegebenenfalls substituierten araliphatischen Rest mit 7 - 32 C-Atomen,
einen gegebenenfalls substituierten heteroaromatischen oder heteroaromatisch-($C_1$-$C_8$)-aliphatischen Rest mit 5 - 12 Ringatomen oder, falls nicht von vorstehenden Definitionen schon umfaßt, die erforderlichenfalls geschützte Seitenkette einer natürlich vorkommenden α-Aminosäure bedeutet,
$R^2$ Wasserstoff, einen gegebenenfalls substituierten aliphatischen Rest mit 1 - 21 C-Atomen, einen gegebenenfalls substituierten alicyclischen Rest mit 3 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen Rest der Formel

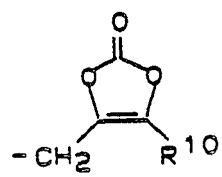

worin $R^{10}$ Wasserstoff, ein aliphatischer Rest mit 1 - 6 C-Atomen oder ein gegebenenfalls substituierter aromatischer Rest mit 6 - 12 C-Atomen ist,
einen Rest der Formel

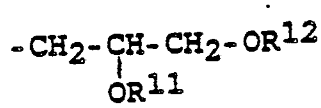 oder 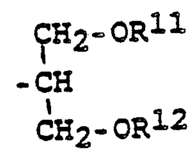

worin $R^{11}$ und $R^{12}$ gleich oder verschieden unabhängig voneinander Wasserstoff, einen gegebenenfalls substituierten Alkylrest mit 1 - 23 C-Atomen oder einen gegebenenfalls substituierten Acylrest mit 1 - 23 C-Atomen bedeuten,
bedeutet,
$R^3$ einen Rest der Formel

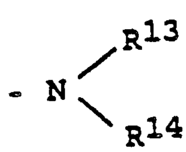

worin $R^{13}$ und $R^{14}$ gleich oder verschieden unabhängig voneinander Wasserstoff, einen gegebenenfalls substituierten aliphatischen Rest mit 1 - 21 C-Atomen, einen gegebenenfalls substituierten alicyclischen Rest mit 3 - 20 C-Atomen, einen gegebenenfalls substituierten aromatischen Rest mit 6 - 12 C-Atomen oder einen gegebenenfalls substituierten araliphatischen Rest mit 7 - 32 C-Atomenbedeuten, oder zusammen mit dem sie tragenden Stickstoffatom einen gegebenenfalls substituierten, gegebenenfalls zugleich teilweise oder vollständig hydrierten, heteroaromatischen Rest mit 2 - 24 C-Atomen bilden, bedeutet
$R^4$ für Wasserstoff oder ($C_1$-$C_6$)Alkyl und
$R^5$ für ($C_1$-$C_6$)Alkyl, ($C_3$-$C_6$)-Cycloalkyl oder

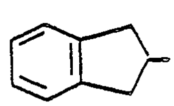

stehen oder
$R^4$ und $R^5$ zusammen mit den sie tragenden Atomen ein mono-, bi-, oder tricyclisches heterocyclisches Ringsystem mit 3 bis 15 Ring-C-Atomen bilden,
$R^6$ Wasserstoff, Amino, $(C_1-C_6)$-Alkyl, $(C_6-C_{12})$-Aryl oder $(C_7-C_{13})$-Aralkyl,
$R^7$ $(C_1-C_6)$-Alkyl oder $(C_7-C_{13})$-Aralkyl, vorzugsweise $-(CH_2)_4-C_6H_5$,
$R^8$ $(C_1-C_6)$-Alkyl, das gegebenenfalls durch $(C_1-C_6)$-Alkanoyloxy monosubstituiert ist, vorzugsweise 2-Methyl-1-propionyloxy-propyl, und
$R^9$ Wasserstoff oder $(C_1-C_6)$-Alkyl bedeuten;
oder deren physiologisch verträglichen Salzes zur Herstellung eines Arzneimittels mit psychotroper Wirkung.

9. Verwendung einer Verbindung der Formel II,

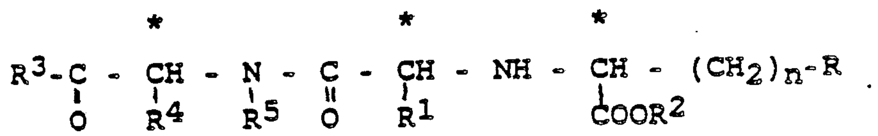 (II)

in welcher
n = 1 oder 2 ist,
R = Wasserstoff
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten alicyclischaliphatischen Rest mit 7 - 14 C-Atomen,
einen gegebenenfalls substituierten heteroaromatischen oder heteroaromatisch-$(C_1-C_8)$-aliphatischen Rest mit 5 - 12 Ringatomen, oder einen Rest $OR^a$ oder $SR^a$ bedeutet, worin
$R^a$ für einen gegebenenfalls substituierten aliphatischen Rest mit 1 - 4 C-Atomen, für einen gegebenenfalls substituierten aromatischen Rest mit 6 - 12 C-Atomen oder einen gegebenenfalls substituierten heteroaromatischen Rest mit 5 - 12 Ringatomen steht,
$R^1$ Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten alicyclischaliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-$(C_1-C_8)$-aliphatischen Rest mit 5 - 12 Ringatomen oder, falls nicht von vorstehenden Definitionen schon umfaßt, die erforderlichenfalls geschützte Seitenkette einer natürlich vorkommenden α-Aminosäure bedeutet,
$R^2$ Wasserstoff,
einen gegebenenfalls substituierten aliphatischen Rest mit 1 - 21 C-Atomen, einen gegebenenfalls substituierten alicyclischen Rest mit 3 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen Rest der Formel

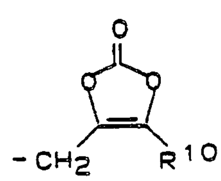

worin $R^{10}$ Wasserstoff, ein aliphatischer Rest mit 1 - 6 C-Atomen oder ein gegebenenfalls substituierter aromatischer Rest mit 6-12 C-Atomen ist,
einen Rest der Formel

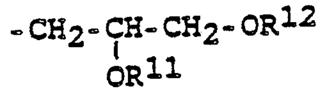

oder

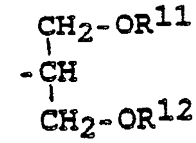

worin $R^{11}$ und $R^{12}$ gleich oder verschieden unabhängig voneinander Wasserstoff, einen gegebenenfalls substituierten Alkylrest mit 1 - 23 C-Atomen oder einen gegebenenfalls substituierten Acylrest mit 1 - 23 C-Atomen bedeuten,
bedeutet,
$R^3$ einen Rest der Formel

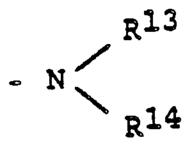

worin $R^{13}$ und $R^{14}$ gleich oder verschieden unabhängig voneinander Wasserstoff, einen gegebenenfalls substituierten aliphatischen Rest mit 1 - 21 C-Atomen, einen gegebenenfalls substituierten alicyclischen Rest mit 3 - 20 C-Atomen, einen gegebenenfalls substituierten aromatischen Rest mit 6 - 12 C-Atomen, einen gegebenenfalls substituierten araliphatischen Rest mit 7 - 32 C-Atomen, oder zusammen mit dem sie tragenden Stickstoffatom einen gegebenenfalls substituierten, gegebenenfalls zugleich teilweise oder vollständig hydrierten, heteroaromatischen Rest mit 2 - 24 C-Atomen bildet, bedeutet, und
$R^4$ und $R^5$ zusammen mit den sie tragenden Atomen ein mono-, bi-, oder tricyclisches heterocyclisches Ringsystem mit 3 bis 15 Ring-C-Atomen bilden,
sowie deren physiologisch verträgliche Salze zur Herstellung eines Arzneimittels mit psychotroper Wirkung.

10. Verwendung gemäß Anspruch 9, dadurch gekennzeichnet, daß Verbindungen der Formel II verwendet werden, in welcher n, R, $R^1$, $R^2$, $R^3$, $R^4$ und $R^5$ wie im Anspruch 1 unter I. a) - e) definiert sind.

11. Verwendung gemäß einem der Ansprüche 9 oder 10, dadurch gekennzeichnet, daß Verbindungen der Formel II verwendet werden, worin $R^4$ und $R^5$ zusammen mit den sie tragenden Atomen ein mono-, bi- oder tricyclisches Ringsystem mit 3 bis 15 Ring-C-Atomen, bis zu 2 Ring-S-Atomen und bis zu 2 Ring-N-Atomen vorzugsweise aus der Reihe Pyrrolidin, Tetrahydroisochinolin, Decahydroisochinolin, Octahydroindol, Indolin, Octahydrocyclopenta[b]pyrrol, 2-Azaspiro[4.5]decan, 2-Azaspiro[4.4]nonan, Spiro[(bicyclo-[2.2.1]-heptan)-2,3′-pyrrolidin], Spiro[(bicyclo-[2.2.2]octan)-2,3′-pyrrolidin], 2-Azatricyclo[$4.3.0.1^{6,9}$]decan, Decahydrocyclohepta[b]pyrrol, Octahydroisoindol, Octahydrocyclopenta[c]pyrrol, 2,3,3a,4,5,7a-Hexahydroindol, 1,2,3,3a,4,6a-Hexahydrocyclopenta[b]pyrrol und 2-Azabicyclo[3.1.0]hexan bilden.

12. Verwendung einer Verbindung der Formel I gemäß Anspruch 9 zur Herstellung eines Arzneimittels zur Behandlung cognitiver Dysfunktionen.

13. Verwendung einer Verbindung der Formel II gemäß Anspruch 10 zur Herstellung eines Arzneimittels zur Behandlung cognitiver Dysfunktion.

Patentansprüche für folgenden Vertragsstaat: GR

1. Verfahren zur Herstellung einer Verbindung der Formel II

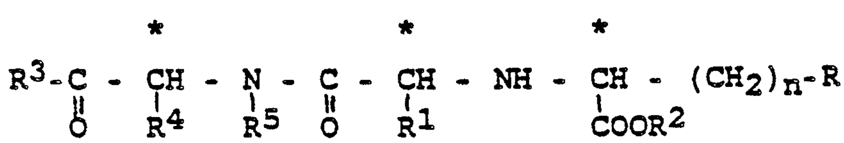

(II)

in welcher

I. a) n = 1 oder 2 ist

b) R 1. Wasserstoff bedeutet,

2. Alkyl mit 1 - 18 C-Atomen bedeutet,

3. einen aliphatischen acyclischen Rest der Formel $C_aH_{(2a-b+1)}$ bedeutet, worin Doppelbindungen, falls ihre

Zahl 1 übersteigt, nicht kumuliert sind, a für eine ganze Zahl 2 bis 18 und b für eine gerade Zahl 2 bis a stehen;

4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (C-2) stehen;

5. Aryl mit 6 - 12 C-Atomen bedeutet, das durch ($C_1$-$C_8$)-Alkyl, ($C_1$-$C_4$)-Alkoxy, Hydroxy, Halogen, Nitro, Amino, Aminomethyl, ($C_1$-$C_4$)-Alkylamino, Di-($C_1$-$C_4$)-alkylamino, ($C_1$-$C_4$)-Alkanoylamino, Methylendioxy, Carboxy, Cyano oder Sulfamoyl, monosubstituiert sein kann;

6. falls n = 2 ist, ($C_6$-$C_{12}$)-Aryl-($C_1$-$C_8$)-alkyl oder Di-($C_6$-$C_{12}$)-aryl-($C_1$-$C_8$)-alkyl bedeutet, die im Arylteil jeweils wie unter I. b) 5. beschrieben mono-, di- oder trisubstituiert sein können; oder

7. Alkoxy mit 1 - 4 C-Atomen;

8. Aryloxy mit 6 - 12 C-Atomen,
das wie unter I. b) 6. beschrieben substituiert sein kann;

9. mono- bzw. bicyclisches Heteroaryloxy mit 5 - 7 bzw. 8 - 10 Ringatomen, wovon bis zu 9 Ringatome Kohlenstoff und 1 bis 2 Ringatome Schwefel oder Sauerstoff und/oder 1 bis 4 Ringatome Stickstoff darstellen, die im Heteroaryl wie unter I. b) 6. beschrieben substituiert sein können.

10. Amino-($C_1$-$C_8$)-alkyl;

11. ($C_1$-$C_4$)-Alkanoylamino-($C_1$-$C_8$)-alkyl;

12. ($C_7$-$C_{13}$)-Aroylamino-($C_1$-$C_8$)-alkyl, das im Arylteil wie unter I. b) 5. monosubstituiert sein kann;

13. ($C_1$-$C_4$)-Alkoxy-carbonylamino-($C_1$-$C_8$)-alkyl;

14. ($C_6$-$C_{12}$)-Aryl-($C_1$-$C_4$)-alkoxycarbonylamino-($C_1$-$C_8$)-alkyl;

15. ($C_6$-$C_{12}$)-Aryl-($C_1$-$C_4$)-alkylamino-($C_1$-$C_8$)-alkyl;

16. ($C_1$-$C_4$)-Alkylamino-($C_1$-$C_8$)-alkyl;

17. Di-($C_1$-$C_4$)-alkylamino-($C_1$-$C_8$)-alkyl;

18. Guanidino-($C_1$-$C_8$)-alkyl;

19. Imidazolyl;

20. Indolyl;

21. ($C_1$-$C_4$)-Alkylthio;

22. falls n = 2 ist, ($C_1$-$C_4$)-Alkylthio-($C_1$-$C_8$)-alkyl;

23. ($C_6$-$C_{12}$)-Arylthio-($C_1$-$C_8$)-alkyl;
das im Arylteil wie unter I. b) 6. beschrieben, substituiert sein kann,

24. ($C_6$-$C_{12}$)-Aryl-($C_1$-$C_8$)-alkylthio,
das im Arylteil wie unter I. b) 6. beschrieben substituiert sein kann;

25. falls n = 2 ist, Carboxy-($C_1$-$C_8$)-alkyl;

26. Carboxy;

27. Carbamoyl;

28. falls n = 2 ist, Carbamoyl-($C_1$-$C_8$)-alkyl;

29. ($C_1$-$C_4$)-Alkoxy-carbonyl-($C_1$-$C_8$)-alkyl;

30. falls n = 2 ist, ($C_6$-$C_{12}$)-Aryloxy-($C_1$-$C_8$)-alkyl, das im Arylteil unter I. b) 6. beschrieben substituiert sein kann; oder

31. ($C_6$-$C_{12}$)-Aryl-($C_1$-$C_8$)-alkoxy,
das im Arylteil wie unter I. b) 6. beschrieben substituiert sein kann, bedeutet;

c) $R^1$ 1. Wasserstoff bedeutet;

2. Alkyl mit 1 - 18 C-Atomen bedeutet;

3. einen aliphatischen acyclischen Rest der Formel $C_aH_{(2a-b+1)}$ bedeutet, worin Doppelbindungen, falls ihre Zahl 1 übersteigt, nicht kumuliert sind, a für eine ganze Zahl 2 bis 18 und b für eine gerade Zahl 2 bis a stehen;

4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (c-2) stehen; oder

5. Aryl mit 6 - 12 C-Atomen,
das wie unter I. b) 6. beschrieben substituiert sein kann;

6. ($C_6$-$C_{12}$)-Aryl-($C_1$-$C_8$)-alkyl oder ($C_7$-$C_{13}$)-Aroyl-($C_1$-$C_8$)-alkyl,
die beide im Arylteil wie unter I. b) 6. beschrieben substituiert sein können;

7. mono- bzw. bicyclisches, gegebenenfalls teilhydriertes Heteroaryl oder Heteroaryl-($C_1$-$C_8$)-alkyl mit 5 - 7 bzw. 8 - 10 Ringatomen, wovon bis zu 9 Ringatome Kohlenstoff und 1 bis 2 Ringatome Schwefel-oder Sauerstoffatome und/oder 1 bis 4 Ringatome Stickstoffatome darstellen, die im Heteroaryl wie bei Aryl unter I. b) 6. beschrieben substituiert sein können;

8. falls von c) 1. -7. noch nicht umfaßt, die gegebenenfalls geschützte Seitenkette einer natürlich vorkommenden α-Aminosäure der Formel $R^1$-CH($NH_2$)-COOH; oder
9. falls von c) 1.-8. noch nicht umfaßt, die gegebenenfalls geschützte Seitenkette von Cyclohexylalanin, 2-Thienylalanin, 3-Thienylalanin, O-($C_3$-$C_5$)-Alkyl-Tyrosin oder C-Phenylglycin bedeutet.

d) $R^2$
1. Wasserstoff bedeutet;
2. Alkyl mit 1 - 18 C-Atomen bedeutet;
3. einen aliphatischen acyclischen Rest der Formel $C_aH_{(2a-b+1)}$ bedeutet, worin Doppelbindungen, falls ihre Zahl 1 übersteigt, nicht kumuliert sind, a für eine ganze Zahl 2 bis 18 und b für eine gerade Zahl 2 bis a stehen;
4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (c-2) stehen; oder
5. Di-($C_1$-$C_4$)-alkylamino-($C_1$-$C_8$)-alkyl;
6. ($C_1$-$C_5$)-Alkanoyloxy-($C_1$-$C_8$)-alkyl;
7. ($C_1$-$C_6$)-Alkoxy-carbonyloxy-($C_1$-$C_8$)-alkyl;
8. ($C_7$-$C_{13}$)-Aroyloxy-($C_1$-$C_8$)-alkyl;
9. ($C_6$-$C_{12}$)-Aryloxycarbonyloxy($C_1$-$C_8$)-alkyl;
10. Aryl mit 6 - 12 C-Atomen;
11. ($C_7$-$C_{20}$)-Aralkyl;
12. Phthalidyl;
13. einen Rest der Formel

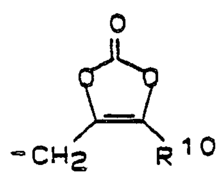

worin $R^{10}$ Wasserstoff, ($C_1$-$C_6$)-Alkyl oder Aryl mit 6 - 12 C-Atomen ist; oder
14. einen Rest der Formel

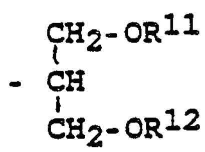

bedeutet,
worin $R^{11}$ und $R^{12}$ gleich oder verschieden unabhängig voneinander Wasserstoff, einen gegebenenfalls substituierten Alkylrest mit 1 - 23 C-Atomen oder einen gegebenenfalls substituierten Acylrest mit 1 - 23 C-Atomen bedeuten
wobei die unter d) 8., 9., 10., 11. und 12. genannten Reste wie unter I. b) 6. beschrieben im Arylteil substituiert sein können;
e) $R^3$ einen Rest der Formel

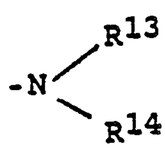

bedeutet,
worin $R^{13}$ und $R^{14}$ gleich oder verschieden unabhängig voneinander
1. Wasserstoff bedeuten;
2. Alkyl mit 1 - 21 C-Atomen bedeuten;
3. einen aliphatischen acyclischen Rest der Formel $C_aH_{(2a-b+1)}$ bedeuten, worin a für eine ganze Zahl 2 bis

21 und b für eine gerade Zahl 2 bis a stehen;

4. einen mono-, di-, tri-, tetra- oder pentacyclischen, nicht aromatischen Kohlenwasserstoffrest der Formel $C_cH_{(2c-d-1)}$ bedeuten, der gegebenenfalls verzweigt ist, worin c für eine ganze Zahl 3 - 20 und d für eine gerade Zahl 0 bis (c-2) stehen; oder

5. Amino-($C_1$-$C_{16}$)-alkyl;

6. Mono-($C_1$-$C_4$)-alkylamino-($C_1$-$C_{16}$)alkyl;

7. Di-($C_1$-$C_4$)-alkylamino-($C_1$-$C_{13}$)alkyl;

8. ($C_1$-$C_5$)-Alkanoyloxy-($C_1$-$C_{16}$)-alkyl;

9. ($C_1$-$C_6$)-Alkoxy-carbonyloxy-($C_1$-$C_{15}$)-alkyl;

10. ($C_7$-$C_{13}$)-Aroyloxy-($C_1$-$C_8$)-alkyl;

11. ($C_6$-$C_{12}$)-Aryloxy-carbonyloxy-($C_1$-$C_9$)alkyl;

12. Aryl mit 6 - 12 C-Atomen, oder

13. ($C_7$-$C_{20}$)-Aralkyl bedeuten

oder zusammen mit dem sie tragenden Stickstoffatom einen gegebenenfalls substituierten, gegebenenfalls teilweise oder vollständig hydrierten heteroaromatischen Rest mit 2 - 24 C-Atomen bilden und

f) $R^4$ und $R^5$ zusammen mit den sie tragenden Atomen ein mono-, bi- oder tricyclisches heterocyclisches Ringsystem mit 3 bis 15 Ring C-Atomen bilden

oder deren physiologisch verträgliche Salze mit Säuren oder Basen,

II. ausgenommen Verbindungen der Formel II und deren Salze, worin

a) n = 1 oder 2 ist;

b) R 1. Wasserstoff;

2. Alkyl mit 1 - 8 C-Atomen;

3. Alkenyl mit 2 - 8 C-Atomen;

4. Cycloalkyl mit 3 - 9 C-Atomen;

5. Aryl mit 6 - 12 C-Atomen, das durch ($C_1$-$C_4$)-Alkyl, ($C_1$-$C_4$)-Alkoxy, Hydroxy, Halogen, Nitro, Amino, Aminomethyl, ($C_1$-$C_4$)Alkylamino, Di-($C_1$-$C_4$)-alkylamino, ($C_1$-$C_4$)Alkanoylamino, Methylendioxy, Carboxy, Cyano und/oder Sulfamoyl mono-, di- oder trisubstituiert sein kann;

6. Alkoxy mit 1 - 4 C-Atomen;

7. Aryloxy mit 6 - 12 C-Atomen, das wie unter II. b) 5. beschrieben substituiert sein kann;

8. mono- bzw. bicyclisches Heteroaryloxy mit 5-7 bzw. 8 - 10 Ringatomen, wovon 1 bis 2 Ringatome Schwefel- oder Sauerstoffatome und/oder 1 bis 4 Ringatome Stickstoff darstellen, das wie unter II. b) 5. beschrieben substituiert sein kann;

9. Amino-($C_1$-$C_4$)-alkyl;

10. ($C_1$-$C_4$)-Alkanoylamino-($C_1$-$C_4$)-alkyl;

11. ($C_7$-$C_{13}$)-Aroylamino-($C_1$-$C_4$)-alkyl;

12. ($C_1$-$C_4$)-Alkoxy-carbonylamino-($C_1$-$C_4$)-alkyl;

13. ($C_6$-$C_{12}$)-Aryl-($C_1$-$C_4$)-alkoxycarbonylamino-($C_1$-$C_4$)-alkyl;

14. ($C_6$-$C_{12}$)-Aryl-($C_1$-$C_4$)-alkylamino-($C_1$-$C_4$)-alkyl;

15. ($C_1$-$C_4$)-Alkylamino-($C_1$-$C_4$)-alkyl;

16. Di-($C_1$-$C_4$)-alkylamino-($C_1$-$C_4$)-alkyl;

17. Guanidino-($C_1$-$C_4$)-alkyl;

18. Imidazolyl;

19. Indolyl;

20. ($C_1$-$C_4$)-Alkylthio;

21. ($C_1$-$C_4$)-Alkylthio-($C_1$-$C_4$)-alkyl;

22. ($C_6$-$C_{12}$)-Arylthio-($C_1$-$C_4$)-alkyl, das im Arylteil wie unter II. b) 5. beschrieben substituiert sein kann;

23. ($C_6$-$C_{12}$)-Aryl-($C_1$-$C_4$)-alkylthio, das im Arylteil wie unter II. b) 5. beschrieben substituiert sein kann;

24. Carboxy-($C_1$-$C_4$)-alkyl;

25. Carboxy;

26. Carbamoyl;

27. Carbamoyl-($C_1$-$C_4$)-alkyl;

28. ($C_1$-$C_4$)-Alkoxy-carbonyl-($C_1$-$C_4$)-alkyl;

29. ($C_6$-$C_{12}$)-Aryloxy-($C_1$-$C_4$)-alkyl, das im Arylteil wie unter II. b) 5. beschrieben substituiert sein kann; oder

30. ($C_6$-$C_{12}$)-Aryl-($C_1$-$C_4$)-alkoxy,

das im Arylteil wie unter II. b) 5. beschrieben substituiert sein kann, bedeutet;

c) $R^1$ 1. Wasserstoff;

2. Alkyl mit 1 - 6 C-Atomen;

3. Alkenyl mit 2 - 6 C-Atomen;

4. Alkinyl mit 2 - 6 C-Atomen;
5. Cycloalkyl mit 3 - 9 C-Atomen;
6. Cycloalkenyl mit 5 - 9 C-Atomen;
7. ($C_3$-$C_9$)-Cycloalkyl-($C_1$-$C_4$)-alkyl;
8. ($C_5$-$C_9$)-Cycloalkenyl-($C_1$-$C_4$)-alkyl;
9. gegebenenfalls teilhydriertes Aryl mit 6 - 12 C-Atomen, das wie II. b) 5. beschrieben substituiert sein kann;
10. ($C_6$-$C_{12}$)-Aryl-($C_1$-$C_4$)-alkyl oder ($C_7$-$C_{13}$)-Aroyl-($C_1$ oder $C_2$)-alkyl,
die beide wie im Arylteil wie unter II. b) 5. beschrieben substituiert sein können;
11. mono- bzw. bicyclisches, gegebenenfalls teilhydriertes Heteroaryl mit 5 - 7 bzw. 8 - 10 Ringatomen, wovon 1 bis 2 Ringatome Schwefel- oder Sauerstoffatome und/oder 1 bis 4 Ringatome Stickstoffatome darstellen, das wie unter II. b) 5. beschrieben substituiert sein kann; oder
12. falls von den vorstehenden Definitionen nicht umfaßt, die gegebenenfalls geschützte Seitenkette einer natürlich vorkommenden α-Aminosäure der Formel $R^1$-CH($NH_2$)-COOH bedeutet;
d) $R^2$
1. Wasserstoff;
2. Alkyl mit 1 - 6 C-Atomen;
3. Alkenyl mit 2 - 6 C-Atomen;
4. Di-($C_1$-$C_4$)-alkylamino-($C_1$-$C_4$)-alkyl;
5. ($C_1$-$C_5$)-Alkanoyloxy-($C_1$-$C_4$)-alkyl;
6. ($C_1$-$C_6$)-Alkoxy-carbonyloxy-($C_1$-$C_4$)-alkyl;
7. ($C_7$-$C_{13}$)-Aroyloxy-($C_1$-$C_4$)-alkyl;
8. ($C_6$-$C_{12}$)-Aryloxycarbonyloxy($C_1$-$C_4$)-alkyl;
9. Aryl mit 6 - 12 C-Atomen;
10. ($C_6$-$C_{12}$)-Aryl-($C_1$-$C_4$)-alkyl;
11. ($C_3$-$C_9$)-Cycloalkyl; oder
12. ($C_3$-$C_9$)-Cycloalkyl-($C_1$-$C_4$)-alkyl bedeutet ,
e) $R^3$ einen Rest der Formel

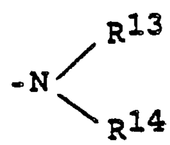

bedeutet,
worin $R^{13}$ und $R^{14}$ gleich oder verschieden unabhängig voneinander
1. Wasserstoff;
2. Alkyl mit 1 - 6 C-Atomen; oder
3. ($C_6$-$C_{12}$)-Aryl-($C_1$-$C_4$)-alkyl
bedeuten und
f) $R^4$ und $R^5$ zusammen mit den sie tragenden Atomen ein mono-, bi- oder tricyclisches heterocyclisches Ringsystem mit 3 bis 15 Ring-C-Atomen bilden, dadurch gekennzeichnet, daß man ihre Fragmente in einem geeigneten Lösungsmittel gegebenenfalls in Gegenwart einer Base und/oder eines Kupplungshilfsmittels miteinander umsetzt, gegebenenfalls intermediär entstehende ungesättigte Verbindungen, wie Schiffsche Basen, reduziert, zum Schutz reaktiver Gruppen temporär eingeführte Schutzgruppen abspaltet, Verbindungen der Formel II mit freier(en) Carboxylgruppe(n) gegebenenfalls verestert oder in Amide überführt und die erhaltenen Verbindungen gegebenenfalls in ihre physiologisch verträglichen Salze überführt.

2. Verfahren gemäß Anspruch 1, dadurch gekennzeichnet, daß man eine Verbindung der Formel II herstellt, in welcher
n = 1 oder 2 ist
R = Wasserstoff,
Alkyl mit 1 - 8 C-Atomen,
Alkenyl mit 2 - 6 C-Atomen,
Cycloalkyl mit 3 - 9 C-Atomen,
Aryl mit 6 - 12 C-Atomen,
das durch ($C_1$-$C_4$)-Alkyl, ($C_1$-$C_4$)-Alkoxy, Hydroxy, Halogen, Nitro, Amino, Aminomethyl, ($C_1$-$C_4$)-Alkylamino,

Di-($C_1$-$C_4$)-alkylamino, ($C_1$-$C_4$)-Alkanoylamino, Methylendioxy, Carboxy, Cyano oder Sulfamoyl, monosubstituiert sein kann,
Alkoxy mit 1 - 4 C-Atomen,
Aryloxy mit 6 - 12 C-Atomen,
das wie oben bei Aryl beschrieben mono-, di- oder trisubstituiert sein kann,
mono- bzw. bicyclisches Heteroaryloxy mit 5 - 7 bzw. 8 - 10 Ringatomen, wovon 1 bis 2 Ringatome Schwefel-oder Sauerstoffatome und/oder 1 bis 4 Ringatome Stickstoff darstellen, das wie oben bei Aryloxy beschrieben substituiert sein kann,
Amino-($C_1$-$C_4$)-alkyl,
($C_1$-$C_4$)-Alkanoylamino-($C_1$-$C_4$)alkyl,
($C_7$-$C_{13}$)-Aroylamino-($C_1$-$C_4$)-alkyl, das wie oben bei Aryl beschrieben substituiert sein kann,
($C_1$-$C_4$)-Alkoxy-carbonylamino-($C_1$-$C_4$)-alkyl,
($C_6$-$C_{12}$)-Aryl-($C_1$-$C_4$)-alkoxycarbonylamino-($C_1$-$C_4$)-alkyl,
($C_6$-$C_{12}$)-Aryl-($C_1$-$C_4$)-alkylamino-($C_1$-$C_4$)-alkyl,
($C_1$-$C_4$)-Alkylamino-($C_1$-$C_4$)-alkyl,
Di-($C_1$-$C_4$)-alkylamino-($C_1$-$C_4$)-alkyl,
Guanidino-($C_1$-$C_4$)-alkyl,
Imidazolyl, Indolyl,
($C_1$-$C_4$)-Alkylthio,
($C_1$-$C_4$)-Alkylthio-($C_1$-$C_4$)-alkyl,
($C_6$-$C_{12}$)-Arylthio-($C_1$-$C_4$-alkyl,
das im Arylteil wie oben bei Aryloxy beschrieben, substituiert sein kann,
($C_6$-$C_{12}$)-Aryl-($C_1$-$C_4$)-alkylthio,
das im Arylteil wie oben bei Aryloxy beschrieben substituiert sein kann,
Carboxy-($C_1$-$C_4$)-alkyl,
Carboxy, Carbamoyl,
Carbamoyl-($C_1$-$C_4$)-alkyl,
($C_1$-$C_4$)-Alkoxy-carbonyl-($C_1$-$C_4$)-alkyl,
($C_6$-$C_{12}$)-Aryloxy-($C_1$-$C_4$)-alkyl,
das im Arylteil wie oben bei Aryloxy beschrieben substituiert sein kann oder
($C_6$-$C_{12}$)-Aryl-($C_1$-$C_4$)-alkoxy,
das im Arylteil wie oben bei Aryloxy beschrieben substituiert sein kann,
$R^1$ Wasserstoff,
Alkyl mit 1 - 6 C-Atomen,
Alkenyl mit 2 - 6 C-Atomen,
Alkinyl mit 2 - 6 C-Atomen,
Cycloalkyl mit 3 - 9 C-Atomen,
Cycloalkenyl mit 5 - 9 C-Atomen,
($C_3$-$C_9$)-Cycloalkyl-($C_1$-$C_4$)-alkyl,
($C_5$-$C_9$)-Cycloalkenyl-($C_1$-$C_4$)-alkyl,
gegebenenfalls teilhydriertes Aryl mit 6 - 12 C-Atomen, das wie oben bei R beschrieben mono-, bi-oder trisubstituiert sein kann, ($C_6$-$C_{12}$)-Aryl-($C_1$-$C_4$)-alkyl oder ($C_7$-$C_{13}$)-Aroyl-($C_1$ oder $C_2$)-alkyl
die beide wie das vorstehende Aryl substituiert sein können,
mono- bzw. bicyclisches, gegebenenfalls teilhydriertes Heteroaryl mit 5 - 7 bzw. 8 - 10 Ringatomen, wovon 1 bis 2 Ringatome Schwefel- oder Sauerstoffatome und/oder 1 bis 4 Ringatome Stickstoffatome darstellen, das wie das vorstehende Aryl substituiert sein kann oder
die gegebenenfalls geschützte Seitenkette einer natürlich vorkommenden $\alpha$-Aminosäure
$R^1$-CH($NH_2$)-COOH bedeutet,
$R^2$ Wasserstoff,
Alkyl mit 1 - 6 C-Atomen,
Alkenyl mit 2 - 6 C-Atomen,
Di-($C_1$-$C_4$)-alkylamino-($C_1$-$C_4$)-alkyl,
($C_1$-$C_5$)-Alkanoyloxy-($C_1$-$C_4$)-alkyl,
($C_1$-$C_6$)-Alkoxy-carbonyloxy-($C_1$-$C_4$)-alkyl,
($C_7$-$C_{13}$)-Aroyloxy-($C_1$-$C_4$)-alkyl,
($C_6$-$C_{12}$)-Aryloxycarbonyloxy($C_1$-$C_4$)-alkyl,
Aryl mit 6 - 12 C-Atomen,
($C_6$-$C_{12}$)-Aryl-($C_1$-$C_4$)-alkyl,

($C_3$-$C_9$)-Cycloalkyl oder
($C_3$-$C_9$)-Cycloalkyl-($C_1$-$C_4$)-alkyl
bedeutet,
$R^3$ einen Rest der Formel

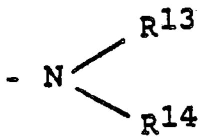

bedeutet,
worin $R^{13}$ und $R^{14}$ gleich oder verschieden unabhängig voneinander
Wasserstoff,
Alkyl mit 1 - 16 C-Atomen,
Alkenyl mit 2 - 16 C-Atomen,
Alkinyl mit 2 - 16 C-Atomen,
Cycloalkyl mit 3 - 9 C-Atomen,
($C_3$-$C_9$)-Cycloalkyl-($C_1$-$C_4$)-alkyl,
Amino-($C_1$-$C_{16}$)-alkyl,
Mono-($C_1$-$C_4$)-alkylamino-($C_1$-$C_{12}$)alkyl,
Di-($C_1$-$C_4$)-alkylamino-($C_1$-$C_8$)-alkyl,
($C_1$-$C_5$)-Alkanoyloxy-($C_1$-$C_{11}$)-alkyl,
($C_1$-$C_6$)-Alkoxycarbonyloxy-($C_1$-$C_{10}$)-alkyl,
($C_7$-$C_{13}$)-Aroyloxy-($C_1$-$C_6$)-alkyl,
($C_6$-$C_{12}$)-Aryloxycarbonyloxy($C_1$-$C_6$)-alkyl,
Aryl mit 6 - 12 C-Atomen oder
($C_6$-$C_{12}$)-Aryl-($C_1$-$C_6$)-alkyl bedeuten
oder zusammen mit dem sie tragenden Stickstoffatom, gegebenenfalls durch Amino, ($C_1$-$C_4$)-Alkylamino-, Di-($C_1$-$C_4$)-alkylamino, ($C_1$-$C_5$)-Alkanoyloxy-, ($C_1$-$C_6$)-Alkoxycarbonyloxy-, ($C_7$-$C_{13}$)-Aroyloxy- oder ($C_6$-$C_{12}$)-Aryloxycarbonyloxy substituiertes
Pyrrolidinyl,
Piperidinyl,
Piperazinyl,
Homopiperazinyl,
Morpholinyl,
Thiomorpholinyl,
Imidazolyl,
Pyridyl,
Pyrimidinyl,
sym.-Triazinyl bedeuten
und
f) $R^4$ und $R^5$ zusammen mit den sie tragenden Atomen ein mono-, bi- oder tricyclisches heterocyclisches Ringsystem mit 3 bis 15 Ring-C-Atomen bilden oder deren physiologisch verträgliche Salze mit Säuren oder Basen.

3. Verfahren gemäß Anspruch 1 oder 2, dadurch gekennzeichnet, daß man eine Verbindung der Formel II herstellt, in welcher
n = 1 oder 2 ist,
R ($C_1$-$C_6$)-Alkyl, ($C_2$-$C_6$)-Alkenyl, ($C_3$-$C_9$)-Cycloalkyl, Amino-($C_1$-$C_4$)-alkyl, ($C_2$-$C_5$)-Acylamino-($C_1$-$C_4$)-alkyl, ($C_7$-$C_{13}$)-Aroylamino-($C_1$-$C_4$)-alkyl, ($C_1$-$C_4$)-Alkoxycarbonylamino-($C_1$-$C_4$)-alkyl, ($C_6$-$C_{12}$)-Aryl-($C_1$-$C_4$)-alkoxycarbonylamino-($C_1$-$C_4$)-alkyl, ($C_6$-$C_{12}$)-Aryl, das durch ($C_1$-$C_4$)-Alkyl, ($C_1$-$C_4$)-Alkoxy, Hydroxy, Halogen, Nitro, Amino, ($C_1$-$C_4$)-Alkylamino, Di-($C_1$-$C_4$)-alkylamino oder Methylendioxy monosubstituiert sein kann, oder 3-Indolyl, insbesondere Methyl, Ethyl, Cyclohexyl, tert. Butoxycarbonylamino-($C_1$-$C_4$)-alkyl, Benzoyloxycarbonylamino-($C_1$-$C_4$)-alkyl oder Phenyl, das durch Phenyl, ($C_1$-$C_2$)-Alkyl, ($C_1$ oder $C_2$)-Alkoxy, Hydroxy, Fluor, Chlor, Brom, Amino, ($C_1$-$C_4$)-Alkylamino, Di-($C_1$-$C_4$)alkylamino oder Nitro monosubstituiert sein kann, bedeutet,
$R^1$ Wasserstoff oder ($C_1$-$C_6$)-Alkyl, das gegebenenfalls durch Amino, ($C_1$-$C_6$)-Acylamino oder Benzoylamino monosubstituiert sein kann, ($C_2$-$C_6$)-Alkenyl, ($C_3$-$C_9$)-Cycloalkyl, ($C_5$-$C_9$)-Cycloalkenyl, ($C_3$-$C_7$)-Cycloalkyl-

($C_1$-$C_4$)-alkyl, ($C_6$-$C_{12}$)-Aryl oder teilhydriertes Aryl, das jeweils durch ($C_1$-$C_4$)-Alkyl, ($C_1$ oder $C_2$)-Alkoxy oder Halogen substituiert sein kann, ($C_6$-$C_{12}$)-Aryl-($C_1$ oder $C_4$)-alkyl oder ($C_7$-$C_{13}$)-Aroyl-($C_1$-$C_2$)-alkyl, die beide wie vorstehend definiert im Arylrest substituiert sein können, ein mono- bzw. bicyclischer Heterocyclen- Rest mit 5 bis 7 bzw. 8 bis 10 Ringatomen, wovon 1 bis 2 Ringatome Schwefel- oder Sauerstoffatome und/oder 1 bis 4 Ringatome Stickstoffatome darstellen, oder eine Seitenkette einer natürlich vorkommenden, gegebenenfalls geschützten α-Aminosäure, insbesondere aber Wasserstoff, ($C_1$-$C_3$)-Alkyl, ($C_2$ oder $C_3$)-Alkenyl, die gegebenenfalls geschützte Seitenkette von Lysin, Benzyl, 4-Methoxybenzyl, 4-Ethoxybenzyl, Phenethyl, 4-Amino-butyl oder Benzoylmethyl bedeutet,

$R^2$ Wasserstoff,

($C_1$-$C_6$)-Alkyl, ($C_2$-$C_6$)-Alkenyl oder ($C_6$-$C_{12}$)-Aryl-($C_1$-$C_4$)-alkyl, insbesondere aber Wasserstoff, ($C_1$-$C_4$)-Alkyl oder Benzyl bedeutet,

$R^3$ einen Rest der Formel

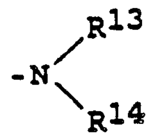

bedeutet,

worin $R^{13}$ und $R^{14}$ gleich oder verschieden unabhängig voneinander Wasserstoff, ($C_4$-$C_{12}$)-Alkyl, ($C_4$-$C_{12}$)-Alkenyl, ($C_5$-$C_7$)-Cycloalkyl, ($C_5$-$C_7$)-Cycloalkyl-($C_1$-$C_4$)-alkyl, Amino-($C_4$-$C_{12}$)-alkyl, ($C_1$-$C_4$)-Alkylamino-($C_4$-$C_{12}$)-alkyl, Di-($C_1$-$C_4$)-Alkylamino-($C_1$-$C_8$)-alkyl, ($C_6$-$C_{12}$)-Aryl-($C_1$-$C_6$)-alkyl, bedeuten oder zusammen mit dem sie tragenden Stickstoffatom gegebenenfalls durch Amino, ($C_1$-$C_4$)-Alkylamino oder Di-($C_1$-$C_4$)-alkylamino substituiertes Pyrrolidinyl, Piperidinyl, Piperazinyl, Homopiperazinyl, Morpholinyl oder Thiomorpholinyl bedeuten,

und

$R^4$ und $R^5$ zusammen mit den sie tragenden Atomen ein mono-, bi- oder tricylisches Ringsystem mit 3 bis 15 Ring-C-Atomen bilden

oder deren physiologisch verträgliche Salze mit Säuren und Basen.

4. Verfahren gemäß einem der Ansprüche 1 bis 3, dadurch gekennzeichnet, daß man eine Verbindung der Formel II herstellt, in welcher $R^4$ und $R^5$ zusammen mit den diese tragenden Atomen ein mono-, bi- oder tricyclisches heterocyclisches Ringsystem mit 3 bis 15 Ring-C-Atomen, bis zu 2 Ring-S-Atomen und bis zu 2 Ring-N-Atomen vorzugsweise aus der Reihe Pyrrolidin, Thiazolidin, Tetrahydroisochinolin, Decahydroisochinolin, Octahydroindol, Indolin, Octahydrocyclopenta[b]pyrrol, 2-Azaspiro[4.5]decan, 2-Azaspiro-[4.4]-nonan, Spiro[(bicyclo[2.2.1]heptan)-2,3′-pyrrolidin], Spiro[(bicyclo[2.2.2]octan)-2,3′-pyrrolidin], 2-Azatricyclo[$4.3.0.1^{6,9}$]decan, Decahydrocyclohepta[b]pyrrol, Octahydroisoindol, Octahydrocyclopenta[c]-pyrrol, 2,3,3a,4,5,7a-Hexahydroindol und 1,2,3,3a,4,6a-Hexahydrocylopenta[b]pyrrol bilden, oder deren physiologisch verträglichen Salze.

5. Verfahren gemäß Anspruch 1, dadurch gekennzeichnet, daß man

a) eine Verbindung der Formel III

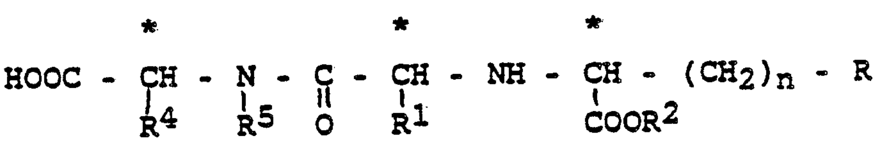

(III)

worin R, $R^1$, $R^2$, $R^4$, $R^5$ und n die gleiche Bedeutung wie in Formel II haben, mit einem Amin der Formel IV

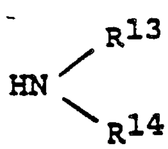

(IV)

worin $R^{13}$ und $R^{14}$ die gleiche Bedeutung wie in Formel II haben, umsetzt,

oder daß man

b) eine Verbindung der Formel V

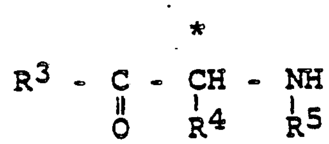

(V)

worin $R^3$, $R^4$ und $R^5$ die gleiche Bedeutung wie in Formel II haben, mit einer Verbindung der Formel VI

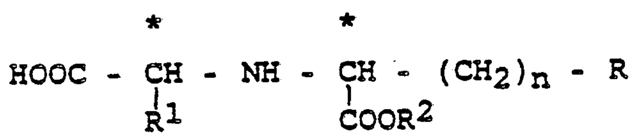

(VI)

worin R, $R^1$, $R^2$ und n die gleiche Bedeutung wie in Formel II haben, umsetzt,
oder daß man
c) eine Verbindung der Formel VII

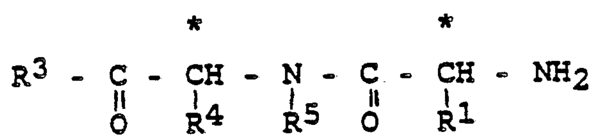

(VII)

worin $R^1$, $R^3$, $R^4$ und $R^5$ die gleiche Bedeutung wie in Formel II haben, mit einer Verbindung der Formel VIII

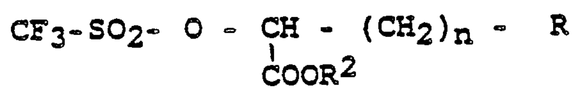

(VIII)

worin R, $R^2$ und n die gleiche Bedeutung wie in Formel II haben, umsetzt,
oder daß man
d) eine Verbindung der Formel IX,

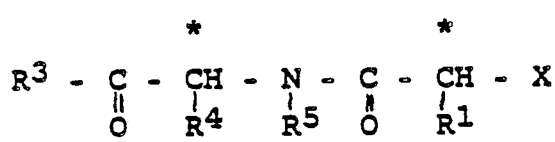

(IX)

worin $R^1$, $R^3$, $R^4$ und $R^5$ die gleiche Bedeutung wie in Formel II haben und
in der X eine Abgangsgruppe, die nucleophil verdrängt werden kann, insbesondere ein Cl, Br, J-Atom oder einen Sulfonsäurerest, bedeutet, mit einer Verbindung der Formel X

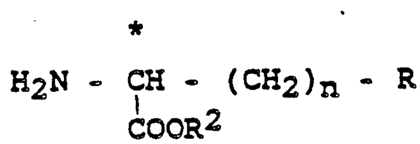

(X)

in der R, $R^2$ und n die gleiche Bedeutung wie in Formel II haben, unter Bedingungen einer nucleophilen Substitution, umsetzt,
oder daß man
e) eine Verbindung der Formel XI

$$R^3-\underset{\underset{O}{\parallel}}{C}-\overset{*}{\underset{\underset{R^4}{|}}{CH}}-\underset{\underset{R^5}{|}}{N}-\underset{\underset{O}{\parallel}}{C}-\overset{*}{\underset{\underset{R^1}{|}}{CH}}-NH-\overset{*}{\underset{\underset{COOH}{|}}{CH}}-(CH_2)_n-R \quad (XI)$$

in der R, $R^1$, $R^3$, $R^4$, $R^5$ und n die gleiche Bedeutung wie in Formel II haben, unter Anwendung einer der gebräuchlichen Veresterungsmethoden in Verbindungen der Formel II überführt, beispielsweise durch Umsetzung mit einem Alkohol $R^2$-OH unter saurer Katalyse oder nach Aktivierung der Carbonsäurefunktion von XI oder nach Aktivierung der Hydroxyfunktion von $R^2OH$, z.B. unter den Bedingungen einer Mitsunobu-Reaktion,
oder durch Umsetzung mit einer Verbindung der Formel $R^2$ X, worin $R^2$ die gleiche Bedeutung wie in Formel II und X die gleiche Bedeutung wie in Formel IX hat unter den Bedingungen einer nucleophilen Substitution, wie sie unter der Verfahrensvariante d) beschrieben sind,
oder durch Umsetzung mit einem Diazoalkan in einem inerten organischen Lösungsmittel,
oder daß man
f) eine Verbindung der Formel XII
$R^2OOC-CH=CH-CO-R$ (XII),
in welcher R und $R^2$ die gleiche Bedeutung wie in Formel II haben, mit einer Verbindung der Formel VII umsetzt und die Carbonylgruppe hydriert,
oder daß man
g) eine Verbindung der Formel VII mit einer Verbindung der Formel XIII

$$R-(CH_2)_n-\underset{\underset{O}{\parallel}}{C}-COOR^2 \quad (XIII)$$

worin R, $R^2$ und n die gleiche Bedeutung wie in Formel II haben, umsetzt und die erhaltenen Schiff-Basen reduziert,
und die so erhaltenen Verbindungen der Formel II gegebenenfalls in ihre physiologisch verträglichen Salze überführt.

6. Verbindung der Formel V

$$R^3-\underset{\underset{O}{\parallel}}{C}-\underset{\underset{R^4}{|}}{CH}-\underset{\underset{R^5}{\backslash}}{NH} \quad (V),$$

worin
$R^3$ einen Rest der Formel

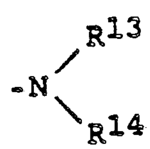

bedeutet,
worin $R^{13}$ und $R^{14}$ gleich oder verschieden unabhängig voneinander
Wasserstoff
Alkyl mit 7 - 16 C-Atomen,
Alkenyl mit 7 - 16 C-Atomen,
Alkinyl mit 7 - 16 C-Atomen
Cycloalkyl mit 3 - 9 C-Atomen,
$(C_3-C_9)$-Cycloalkyl-$(C_1-C_4)$-alkyl,
Amino-$(C_1-C_{16})$-alkyl,
Mono-$(C_1-C_4)$-alkylamino-$(C_1-C_{12})$alkyl,
Di-$(C_1-C_4)$-alkylamino-$(C_1-C_8)$alkyl,
$(C_1-C_5)$-Alkanoyloxy-$(C_1-C_{11})$-alkyl,
$(C_1-C_6)$-Alkoxycarbonyloxy-$(C_1-C_{10})$-alkyl,
$(C_7-C_{13})$-Aroyloxy-$(C_1-C_6)$-alkyl,
$(C_6-C_{12})$-Aryloxycarbonyloxy-$(C_1-C_6)$-alkyl,

Aryl mit 6 - 12 C-Atomen oder
$(C_6-C_{12})$-Aryl-$(C_5-C_6)$-alkyl bedeuten
oder zusammen mit dem sie tragenden Stickstoffatom, gegebenenfalls durch Amino, $(C_1-C_4)$-Alkylamino, Di-$(C_1-C_4)$-alkylamino, $(C_1-C_5)$-Alkanoyloxy, $(C_1-C_6)$-Alkoxycarbonyloxy, $(C_7-C_{13})$-Aroyloxy oder $(C_6-C_{12})$-Aryloxycarbonyloxy substituiertes
Pyrrolidinyl,
Piperidinyl,
Piperazinyl,
Homopiperazinyl,
Morpholinyl,
Thiomorpholinyl,
Imidazolyl,
Pyridyl,
Pyrimidinyl,
sym.-Triazinyl bedeuten
und
$R^4$ und $R^5$ zusammen mit den sie tragenden Atomen ein mono-, bi- oder tricyclisches heterocyclisches Ringsystem mit 3 bis 15 Ring-C-Atomen bilden.

7. Verfahren zur Herstellung einer Verbindung der Formel V gemäß Anspruch 6, dadurch gekennzeichnet, daß man in Verbindungen der Formel XIV,

$$HOOC - \underset{R^4}{\underset{|}{CH}} - \underset{R^5}{\underset{|}{NH}} \qquad (XIV),$$

worin $R^4$ und $R^5$ die gleiche Bedeutung wie in Formel V haben, die Aminogruppe schützt, dann mit einem Amin der Formel IV
$R^{13} - NH - R^{14}$ (IV),
worin $R^{13}$ und $R^{14}$ die gleiche Bedeutung wie in Formel V haben, umsetzt und die Amino-Schutzgruppe wieder abspaltet.

8. Verfahren zur Herstellung eines pharmazeutischen Mittels enthaltend eine Verbindung gemäß einem oder mehreren der Ansprüche 1 bis 4, dadurch gekennzeichnet, daß man eine Verbindung der Formel II oder deren physiologisch verträgliches Salz und einen physiologisch annehmbaren Träger gegebenenfalls zusammen mit weiteren Zusatz-oder Hilfsstoffen in eine geeignete Darreichungsform bringt.

9. Verwendung einer Verbindung der Formel I,
$X^1 - X^2$ (I)
in welcher

$X^1$ 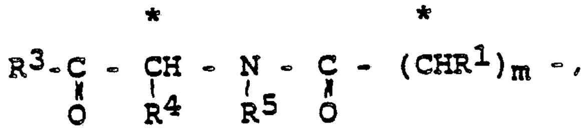

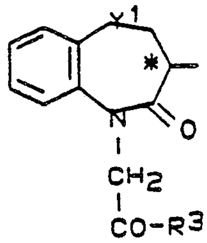 oder 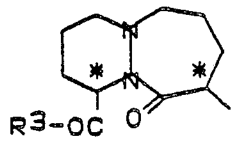 bedeutet,

$X^2$ $-CH_2SH$, 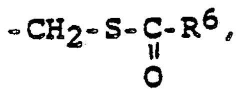, 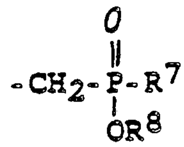 oder

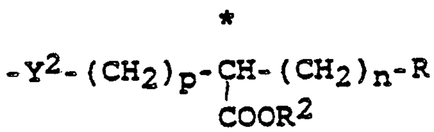 bedeutet,

$Y^1$ für -S- oder $-CH_2-$ steht,
$Y^2$ für $-NR^9-$ oder $-CH_2-$ steht,
m = 0 oder 1 ist,
n = 0, 1 oder 2 ist,
p = 0 oder 1 ist,
R = Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten alicyclisch-aliphatischen Rest mit 4 - 20 C-Atomen,
einen gegebenenfalls substituierten heteroaromatischen oder heteroaromatisch-($C_1$-$C_8$)-aliphatischen Rest mit 5 - 12 Ringatomen, oder einen Rest $OR^a$ oder $SR^a$ bedeutet, worin
$R^a$ für einen gegebenenfalls substituierten aliphatischen Rest mit 1 - 4 C-Atomen, für einen gegebenenfalls substituierten aromatischen Rest mit 6 - 12 C-Atomen
oder einen gegebenenfalls substituierten heteroaromatischen Rest mit 5 - 12 Ringatomen steht,
$R^1$ Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten alicyclisch-aliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-($C_1$-$C_8$)-aliphatischen Rest mit 5 - 12 Ringatomen oder, falls nicht von vorstehenden Definitionen schon umfaßt, die erforderlichenfalls geschützte Seitenkette einer natürlich vorkommenden α-Aminosäure bedeutet,
$R^2$ Wasserstoff, einen gegebenenfalls substituierten aliphatischen Rest mit 1 - 21 C-Atomen, eine gegebenenfalls substituierten alicyclischen Rest mit 3 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen Rest der Formel

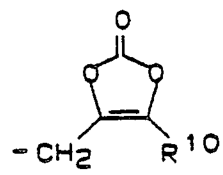

worin $R^{10}$ Wasserstoff, ein aliphatischer Rest mit 1 - 6 C-Atomen oder ein gegebenenfalls substituierter aromatischer Rest mit 6 - 12 C-Atomen ist, einen Rest der Formel

$$-CH_2-CH(OR^{11})-CH_2-OR^{12} \quad \text{oder} \quad -CH(CH_2-OR^{11})(CH_2-OR^{12})$$

worin $R^{11}$ und $R^{12}$ gleich oder verschieden unabhängig voneinander Wasserstoff, einen gegebenenfalls substituierten Alkylrest mit 1 - 23 C-Atomen oder einen gegebenenfalls substituierten Acylrest mit 1 - 23 C-Atomen bedeuten,
bedeutet,
$R^3$ einen Rest der Formel

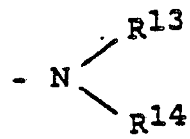

worin $R^{13}$ und $R^{14}$ gleich oder verschieden unabhängig voneinander Wasserstoff, einen gegebenenfalls substituierten aliphatischen Rest mit 1 - 21 C-Atomen, einen gegebenenfalls substituierten alicyclischen Rest mit 3 - 20 C-Atomen, einen gegebenenfalls substituierten aromatischen Rest mit 6 - 12 C-Atomen oder einen gegebenenfalls substituierten araliphatischen Rest mit 7 - 32 C-Atomenbedeuten, oder zusammen mit dem sie tragenden Stickstoffatom einen gegebenenfalls substituierten, gegebenenfalls zugleich teilweise oder vollständig hydrierten, heteroaromatischen Rest mit 2 - 24 C-Atomen bilden, bedeutet
$R^4$ für Wasserstoff oder $(C_1-C_6)$Alkyl und
$R^5$ für $(C_1-C_6)$Alkyl, $(C_3-C_6)$-Cycloalkyl oder

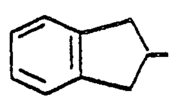

stehen oder
$R^4$ und $R^5$ zusammen mit den sie tragenden Atomen ein mono-, bi-, oder tricyclisches heterocyclisches Ringsystem mit 3 bis 15 Ring-C-Atomen bilden,
$R^6$ Wasserstoff, Amino, $(C_1-C_6)$-Alkyl, $(C_6-C_{12})$-Aryl oder
$(C_7-C_{13})$-Aralkyl,
$R^7$ $(C_1-C_6)$-Alkyl oder $(C_7-C_{13})$-Aralkyl, vorzugsweise $-(CH_2)_4-C_6H_5$,
$R^8$ $(C_1-C_6)$-Alkyl, das gegebenenfalls durch $(C_1-C_6)$-Alkanoyloxy monosubstituiert ist, vorzugsweise 2-Methyl-1-propionyloxy-propyl, und
$R^9$ Wasserstoff oder $(C_1-C_6)$-Alkyl bedeuten;
oder deren physiologisch verträglichen Salzes zur Herstellung eines Arzneimittels mit psychotroper Wirkung.

10. Verwendung einer Verbindung der Formel II,

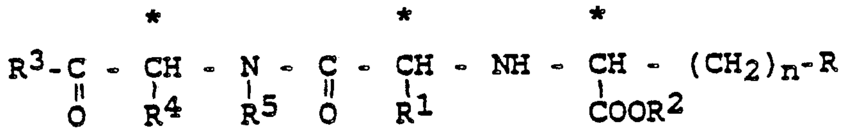 (II)

in welcher
n = 1 oder 2 ist,
R = Wasserstoff
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten alicyclisch-aliphatischen Rest mit 7 - 14 C-Atomen,
einen gegebenenfalls substituierten heteroaromatischen oder heteroaromatisch-($C_1$-$C_8$)-aliphatischen Rest mit 5 - 12 Ringatomen, oder einen Rest $OR^a$ oder $SR^a$ bedeutet, worin
$R^a$ für einen gegebenenfalls substituierten aliphatischen Rest mit 1 - 4 C-Atomen, für einen gegebenenfalls substituierten aromatischen Rest mit 6 - 12 C-Atomen oder einen gegebenenfalls substituierten heteroaromatischen Rest mit 5 - 12 Ringatomen steht,
$R^1$ Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten alicyclisch-aliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-($C_1$-$C_8$)-aliphatischen Rest mit 5 - 12 Ringatomen oder, falls nicht von vorstehenden Definitionen schon umfaßt, die erforderlichenfalls geschützte Seitenkette einer natürlich vorkommenden α-Aminosäure bedeutet,
$R^2$ Wasserstoff,
einen gegebenenfalls substituierten aliphatischen Rest mit 1 - 21 C-Atomen, einen gegebenenfalls substituierten alicyclischen Rest mit 3 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen Rest der Formel

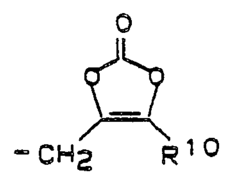

worin $R^{10}$ Wasserstoff, ein aliphatischer Rest mit 1 - 6 C-Atomen oder ein gegebenenfalls substituierter aromatischer Rest mit 6-12 C-Atomen ist,
einen Rest der Formel

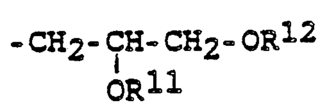 oder 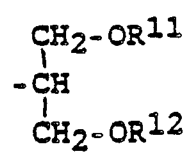

worin $R^{11}$ und $R^{12}$ gleich oder verschieden unabhängig voneinander Wasserstoff, einen gegebenenfalls substituierten Alkylrest mit 1 - 23 C-Atomen oder einen gegebenenfalls substituierten Acylrest mit 1 - 23 C-Atomen bedeuten, bedeutet,
$R^3$ einen Rest der Formel

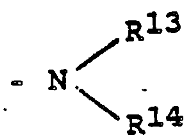

worin $R^{13}$ und $R^{14}$ gleich oder verschieden unabhängig voneinander Wasserstoff, einen gegebenenfalls substituierten aliphatischen Rest mit 1 - 21 C-Atomen, einen gegebenenfalls substituierten alicyclischen Rest mit 3 - 20 C-Atomen, einen gegebenenfalls substituierten aromatischen Rest mit 6 - 12 C-Atomen, einen gegebenenfalls substituierten araliphatischen Rest mit 7 - 32 C-Atomen, oder zusammen mit dem sie tragenden Stickstoffatom einen gegebenenfalls substituierten, gegebenenfalls zugleich teilweise oder vollständig hydrierten, heteroaromatischen Rest mit 2 - 24 C-Atomen bildet, bedeutet, und

$R^4$ und $R^5$ zusammen mit den sie tragenden Atomen ein mono-, bi-, oder tricyclisches heterocyclisches Ringsystem mit 3 bis 15 Ring-C-Atomen bilden,

sowie deren physiologisch verträgliche Salze zur Herstellung eines Arzneimittels mit psychotroper Wirkung.

11. Verwendung gemäß Anspruch 10, dadurch gekennzeichnet, daß Verbindungen der Formel II verwendet werden, in welcher n, R, $R^1$, $R^2$, $R^3$, $R^4$ und $R^5$ wie im Anspruch 1 unter I. a) - e) definiert sind.

12. Verwendung gemäß einem der Ansprüche 10 oder 11, dadurch gekennzeichnet, daß Verbindungen der Formel II verwendet werden, worin $R^4$ und $R^5$ zusammen mit den sie tragenden Atomen ein mono-, bi- oder tricyclisches Ringsystem mit 3 bis 15 Ring-C-Atomen, bis zu 2 Ring-S-Atomen und bis zu 2 Ring-N-Atomen vorzugsweise aus der Reihe Pyrrolidin, Tetrahydroisochinolin, Decahydroisochinolin, Octahydroindol, Indolin, Octahydrocyclopenta[b]pyrrol, 2-Azaspiro[4.5]decan, 2-Azaspiro[4.4]nonan, Spiro[(bicyclo-[2.2.1]-heptan)-2,3′-pyrrolidin], Spiro[(bicyclo-[2.2.2]octan)-2,3′-pyrrolidin], 2-Azatricyclo[4.3.0.$1^{6,9}$]decan, Decahydrocyclohepta[b]pyrrol, Octahydroisoindol, Octahydrocyclopenta[c]pyrrol, 2,3,3a,4,5,7a-Hexahydroindol, 1,2,3,3a,4,6a-Hexahydrocyclopenta[b]pyrrol und 2-Azabicyclo[3.1.0]hexan bilden.

13. Verwendung einer Verbindung der Formel I gemäß Anspruch 9 zur Herstellung eines Arzneimittels zur Behandlung cognitiver Dysfunktionen.

14. Verwendung einer Verbindung der Formel II gemäß Anspruch 10 zur Herstellung eines Arzneimittels zur Behandlung cognitiver Dysfunktion.

Europäisches Patentamt

# EUROPÄISCHER RECHERCHENBERICHT

Nummer der Anmeldung

EP 89101493.8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl.4) |
|---|---|---|---|
| D,X | EP - A1 - 0 012 401 (MERCK & CO. INC.) * Ansprüche 1,2,7,9; Seiten 8-10 * | 1-7,9, 10,13, 14 | C 07 K 5/06<br>A 61 K 37/02 |
| D,Y | * Ansprüche 1,2,7,9 * | 11,12, 15-20 | |
| | -- | | |
| D,X | EP - A1 - 0 050 800 (SCHERING CORPORATION) * Ansprüche 1,10,12; Seite 20 * | 1-7,9, 10,13, 14 | |
| D,Y | * Ansprüche 1,10,12 * | 11,12, 15-20 | |
| | -- | | |
| X | EP - A1 - 0 079 521 (MERCK & CO. INC.) * Anspruch 1; Seite 1 * | 1-4,9, 10,13, 14 | |
| | -- | | **RECHERCHIERTE SACHGEBIETE (Int. Cl.4)** |
| Y | EP - A2 - 0 243 645 (HOECHST AKTIENGESELLSCHAFT) * Ansprüche 1,15; Seiten 2,22 * | 11,12, 15-20 | C 07 K 5/00<br>A 61 K 37/00 |
| A | * Ansprüche 1,8; Seite 22 * | 1-5,9, 10,13, 14 | |
| | -- | | |
| A | EP - A1 - 0 196 841 (SYNTEX (USA)INC) * Ansprüche 1,24; Spalte 1 * | 1-7,9, 10,13, 14 | |
| | -- | | |
| A | EP - A1 - 0 080 822 (BEECHAM GROUP PLC) * Ansprüche 1,12; Seite 8 * | 1-6,9, 10,13, 14 | |
| | -- | | |

Der vorliegende Recherchenbericht wurde für alle Patentansprüche erstellt

| Recherchenort | Abschlußdatum der Recherche | Prüfer |
|---|---|---|
| WIEN | 12-05-1989 | PETROUSEK |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

Europäisches Patentamt

# EUROPÄISCHER RECHERCHENBERICHT

Nummer der Anmeldung

-2-

EP 89101493.8

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| A | EP - A1 - 0 115 091 (ENI-ENTE NAZIONALE IDROCARBURI)<br>* Anspruch; Seite 1 *<br>-- | 1-4,9, 10,13, 14 | |
| A | EP - A1 - 0 052 991 (E.R.SQUIBB & SONS, INC.)<br>* Ansprüche 1,34; Seite 8 *<br>-- | 1-6,9, 10,13, 14 | |
| A | EP - A2/A3 - 0 048 159 (UNIVERSITY OF MIAMI)<br>* Anspruch 1; Seiten 28,31 *<br>---- | 1-6,9, 10,13, 14 | RECHERCHIERTE SACHGEBIETE (Int. Cl.4) |

Der vorliegende Recherchenbericht wurde für alle Patentansprüche erstellt.

| Recherchenort | Abschlußdatum der Recherche | Prüfer |
|---|---|---|
| WIEN | 12-05-1989 | PETROUSEK |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82